# EUROPEAN PATENT APPLICATION

(11) **EP 1 544 291 A1**
(43) Date of publication of application: **22.06.2005**
(21) Application number: 03765329.2
(22) Date of filing: 18.07.2003
(51) Int. Cl.: C12N 9/00, C12N 15/52, C12N 1/21, C12N 1/19, C12N 5/10, C12N 1/15, A61K 38/43

(54) **NOVEL PROCESS FOR PRODUCING ANTIBODY ENZYME, NOVEL ANTIBODY ENZYME AND UTILIZATION THEREOF**

(30) Priority: 19.07.2002 JP 2002211756; 19.07.2002 JP 2002211768; 27.02.2003 JP 2003051943; 17.07.2003 JP 2003198270; 17.07.2003 JP 2003198281; 17.07.2003 JP 2003198292
(71) Applicant: Japan Science and Technology Agency, Kawaguchi-shi, Saitama 332-0012 (JP)
(72) Inventor: UDA, Taizo, Miyoshi-shi, Hiroshima 728-0013 (JP); HIFUMI, Emi, Shobara-shi, Hiroshima 727-0021 (JP)
(74) Representative: HOFFMANN EITLE
(86) International application number: PCT/JP2003/009147
(87) International publication number: WO 2004/009805

(57) **Abstract**

A process for producing an antibody enzyme which involves an antibody structure analysis step of confirming the presence of a catalyst triplet residue structure wherein a serine residue, an aspartate residue and a histidine residue or a glutamate residue are located stereostructurally close to each other in the stereostructure of an antibldy anticipated based on its amino acid sequence. Since the above-described catalyst triplet residue structure is a structure specific to an antibody enzyme, an antibody enzyme can be efficiently screened by using the same. Examples of the antibody enzyme as described above include an antibody enzyme against Helicobacter pyloriurease and an antibody enzyme against chemokine receptor CCR-5.

## Description

### Field of the invention

The present invention relates to a novel process for efficiently producing an antibody enzyme that has high molecule recognition capability of an antibody and the enzymatic activity, an antibody enzyme produced according to the producing process, and an antibody enzyme having a stereostructurally specific structure that can be applied in the producing process.

Furthermore, the present invention relates to, among the antibody enzymes in particular, an antibody against Helicobacter pylori urease and an antibody enzyme against chemokine receptor CCR-5 that is a coreceptor of AIDS-causing virus as well as an application method of the antibody enzymes.

### Background of the invention

Recently, there are various reports on an antibody having enzyme-like activity, that is, an antibody enzyme. For instance, S. Paul et al. report that an autoantibody isolated from an autoimmune disease patient followed by purifying has an activity of decomposing VIP (Vasoactive Intestinal Peptide) (Reference literature 1: Paul, S., et al., Science, 244: 1158, 1989). Furthermore, Gabibov et al. report that an autoantibody isolated from a patient suffered from autoimmune disease such as SLE (Systemic lupus erythematosus) or immune cell proliferation has an antibody that enzymatically decomposes a DNA (Reference literature 2: Shuster, A.M., et al., Science, 256: 665, 1992, and Gabibov, A.G., et al., Appl. Biochem. Biotech., 47: 293, 1994).

Still furthermore, the present inventors obtained a monoclonal antibody of which antigen is a stable region of an envelope protein gp41 of AIDS virus (HIV) that causes AIDS (acquired immune deficiency syndrome) and analyzed a function of the monoclonal antibody in detail. As a result, the inventors report that a light chain region of the monoclonal antibody has a very high activity in specifically decomposing the envelope protein gp41 of the HIV (Reference literature 3 : Super Catalytic Antibody [ I ] : Decomposition of targeted protein by its antibody light chain. Hifumi, E. , Okamoto, Y., Uda, T., J. Biosci. Bioeng., 88(3), 323-327 (1999)).

Thus, the antibody enzyme, having high molecule recognizing capability of antibody and the enzymatic activity, is expected to apply in many fields such as the healthcare, chemical industry, and food industry.

So far, as a process of preparing and obtaining such antibody enzymes, only a method in which peptides or proteins in a ground state are immunized in an animal and so on, and, of all obtained antibodies, the enzymatic activity was measured to select and obtain only antibodies having the targeted enzymatic activity is known.

However, there are problems in the abovementioned existing process for preparing and obtaining antibody enzymes in that it necessitates much labor and long time, and even when the enzymatic activity is measured of all antibodies the probability of finding the antibody enzymes is only less than 10%, that is, the efficiency is very low.

Thus, a process of efficiently preparing or obtaining antibody enzymes is not yet found. Accordingly, it is largely expected to develop an antibody enzyme having excellent function and a process of efficiently obtaining and preparing antibody enzymes and to further apply to researches and industries.

The present invention was carried out in view of the abovementioned situations and intends to provide a novel process for efficiently producing antibody enzymes that have high molecule recognition capability of an antibody and the enzymatic activity and antibody enzymes produced according to the producing process, as well as antibody enzymes having a stereostructurally specific structure that can be applied to the producing process.

The Helicobacter Pylori is a slender S-shaped gram-negative bacterium and can be detected with high frequency from biological specimens of stomachs of gastric ulcer suffering patients and peptic ulcer suffering patients. From this, the Helicobacter Pylori is considered to cause gastric ulcer/gastric catarrh and suggested to be finally relevant to occurrence of gastric cancer. This is reported also from WHO (World Health Organization).

A rate of Japanese who are infected with the Helicobacter Pylori (hereinafter, referred to as HP) is said amounts to 80% in adults of 50 years old or more; accordingly, researches and developments of bacteria elimination and rapid diagnosis of the HP are socially in demand. In order to eliminate the HP, several antibiotics have been developed, and, in recent years, by administering three kinds of antibiotics in combination, recognizable healing effect is recognized.

As mentioned above, as antibacterial agents or antiviral drugs for eliminating bacteria and viruses, the antibiotic drugs have worked as a major player. However, recently, the occurrence speed of drug resistant bacteria against an antibiotic drug becomes very fast. In spite of substantially 10 years being spent for developing an antibiotic drug, according to recent reports, after the administration of the antibiotic drug, in faster cases, within only several months, drug resistant bacteria appear and the antibiotic drug becomes ineffective. This is a very problematic situation.

There are reports according to which similar problems are occurring against the Helicobacter Pylori and in spite of only a short time after the start of administration of an antibiotic drug, drug resistant bacteria against the HP have appeared. Furthermore, it is also found that, in some cases, after the bacteria elimination by use of an antibiotic drug, gastric ulcer reoccurs. From these backgrounds, a development of novel drugs against the HP different from existing ones is strongly demanded.

Since an interior of a human stomach is strongly acidic owing to secretion of gastric-acid, normally externally intruded bacteria cannot exist; however, the Helicobacter Pylori, owing to an action of urease that is expressed in its own cell envelope, can exist even in a strongly acidic stomach. Accordingly, when the activity of the Helicobacter Pylori urease can be suppressed, the bacteria cannot exist in a stomach, resulting in inhibiting infection from occurring.

The present invention pays attention to the point and provides an antibody enzyme that, by exhibiting a decomposition action against, in particular among the above antibody enzymes, the Helicobacter Pylori, is effective in eliminating the Helicobacter Pylori and can avoid the appearance of drug-resistant bacteria. Furthermore, the invention provides genes that code the antibody enzymes, transformants in which the genes are introduced, and a remedy/infection inhibitor that makes use of these and is used for patients infected by the Helicobacter Pylori.

The AIDS (acquired immunodeficiency syndrome) is a disease state where owing to the infection of human immunodeficiency virus I type (HIV-1), immune function is deteriorated, resultantly AIDS-associated opportunistic infection, malignant tumor, neurosis, dementia and so on are caused together. When infected with the HIV-1, after an acute period, a symptomless period, and AIDS-associated syndrome, the AIDS is developed. Owing to AIDS-related researches and advances of remedy, the AIDS is becoming from fatal disease to a remediable disease; however, at present, there is no sure anti-HIV drug and a treatment method due to drugs is not yet established.

In researches of an infection mechanism of the HIV, in 1984, as a receptor of the HIV-1, CD4 was identified. It is known that the CD4 is present on a surface of a human cell and, when it combines with gp120 that is an envelope glycoprotein of the HIV-1 to infect the cell.

However, since the CD4 alone does not cause fusion between an envelope of virus and a cell membrane of a host cell, infection of the HIV-1 is not caused; accordingly, the presence of a coreceptor was suggested. When it has passed more than 10 years after the discovery of the CD4, a chemokine receptor that is a coreceptor of the HIV was discovered. Furthermore, it was also reported that when the chemokine receptor CCR-5 was not normal, resistance against infection and crisis of the HIV-1 was exhibited. Accordingly, at present, many researchers and enterprises venture on development of drugs that can inhibit the HIV-1 from intruding with the coreceptor as a target.

Present situations of researches and developments of anti-HIV drugs with such a HIV coreceptor as a target are described in reference literature 4 (T. Murakami and N. Yamamoto "Development of novel anti-HIV drug-drug affecting on HIV coreceptor (second receptor)", Protein, Nucleic acid, Enzyme, Vol.43, 677-685 p (1998)).

As shown in Table 1 in the reference literature 4, all over the world pharmaceutical companies, in collaboration with basic research laboratories such as universities, venture on research and development of anti-HIV drugs with the HIV coreceptor as a target. As a candidate of the anti-HIV drug that can prevent the HIV-1 from infecting with the chemokine receptor CCR-5 as a target, a compound that works as an antagonist thereof is reported.

However, at present, an effective drug that directly attacks the chemokine receptor CCR-5 and deletes its function is not at all found.

In this connection, the present invention further provides, among the abovementioned antibody enzymes, in particular, an antibody enzyme that can decompose the chemokine receptor CCR-5 to delete its function and can be utilized in preventing the AIDS viruses from infecting and remedying the AIDS. Furthermore, the invention provides a gene that codes the antibody enzyme, a transformant in which the gene is introduced, and an anti-HIV drug that makes use of the above.

### Disclosure of the Invention

The present inventors, in view of the abovementioned situations, studied hard and, when a structure and a function were analyzed in detail of an antibody having the enzymatic activity that cleaves and/or decomposes a polypeptide and an antigen protein, found independently that in stereostructures of all antibody enzymes, a catalytic triad residue exists and the catalytic triad residue is relevant with the polypeptide decomposing activity, and came to completion of the invention.

Furthermore, the inventors, in order to overcome the above problems, studied hard to obtain, among antibody enzymes that have, while being an antibody, an enzyme action and can completely decompose a target protein, in particular, an antibody enzyme against the HP urease. As a result, it is found that among antibodies against the HP urease, there are ones that exhibit a function as an antibody enzyme, and thereby the invention came to completion.

Still furthermore, the inventors, in order to overcome the above problems, studied hard to obtain, among antibody enzymes, in particular, an antibody enzyme against chemokine receptor CCR-5. As a result, it is found that among antibodies against the chemokine receptor CCR-5, there are ones that exhibit a function as an antibody enzyme, and thereby the invention came to completion.

A production process of an antibody enzyme involving the invention, in order to overcome the abovementioned problems, includes an antibody structure analysis process that performs a stereostructure estimation step where a stereostructure of an antibody is estimated from amino-acid sequences; and a catalytic triad residue structure confirmation step where, in an estimated stereostructure of the antibody, whether a catalytic triad residue structure where a serine residue, an aspartate residue and a histidine residue or a glutamate residue are present stereostructurally in proximity to each other is present or not is confirmed.

In the catalytic triad structure confirmation step, in order to confirm the catalytic triad structure, any one of indices below can be preferably used.

Index of type ① or ①*: It has either a structure same as a catalytic triad residue structure common in known antibody enzymes (type ①) or a structure where when among the known catalytic triad residue structures one amino-acid residue that is different in position is used, a catalytic triad residue structure can be assumed to constitute (type ①*).
Index of type ②: A serine residue, an aspartate residue and a histidine residue or a glutamate residue are present in the range of 3 to 20 angstrom in the stereostructure.
Index of type ③: When an antibody has a germ-cell gene type derived from a known antibody enzyme, it is used as an index.
Index of type ④: A complimentarity determining region 1 (CDR1) being constituted of 16 amino-acid residues and a histidine residue being located at the 93^{rd} according to Kabat numbering scheme are used as an index.
Index of type ④*: A complimentarity determining region 1 (CDR1) being constituted of 11 amino-acid residues and a histidine residue being located at the 91^{st} or 55^{th} according to Kabat numbering scheme are used as an index.

Furthermore, the antibody enzyme production process preferably includes an antibody production process in which an antibody is produced by use of a hybridoma obtained by fusing a mouse spleen lymph corpuscle immunized with antigen and a mouse myeloma cell. As the antigen, a hapten-conjugated protein obtained by coupling a substance that is made an antigenic determinant to a carrier protein can be used; however, it is not particularly restricted. It goes without saying that a protein itself as well can be used as an immunogen.

The antibody enzyme production process may further include a catalytic triad residue structure introduction process where by use of the stereostructure information obtained after the antibody structure analysis process, a catalytic triad residue structure is introduced into an antibody according to a genetic engineering procedure.

The catalytic triad residue structure is one that the present inventors found newly this time as a feature of an antibody enzyme having an activity of cleaving and/or decomposing a polypeptide or antigen protein. Accordingly, since according to the process, antibodies are screened by use of the catalytic triad residue structure unique to the antibody enzyme, the antibody enzymes can be produced more efficiently than ever.

An antibody enzyme involving the invention has in a stereostructure a catalytic triad residue structure where a serine residue, an aspartate residue, and a histidine residue or glutamate residue are present stereostructurally in proximity. As an example of the antibody enzyme, as shown in examples described later, heavy and light chains of i4SL1-2 and i41-7 antibodies can be cited. The heavy and light chains of the i4SL1-2 and i41-7 antibodies are experimentally confirmed that, while maintaining a nature, as an antibody, of being high in the affinity with a substrate, these exhibit the enzymatic activity.

The "catalytic triad residue structure" means a structure in which it is inferred that three amino-acid residues containing at least serine are contained in an active site and form an active center. A protease having the catalytic triad residue structure contains serine in the active site; accordingly, it is called a serine protease. Accordingly, the antibody enzyme can be said one kind of the serine protease. When a structure that can be estimated to be a catalytic triad residue is contained, high activity can be expected as the protease.

An antibody enzyme involving the invention, further, may be an antibody enzyme of which complimentarity determining region 1 (CDR1) is constituted of 16 amino-acid residues and that has a histidine residue at the 93^{rd} according to Kabat numbering scheme, or an antibody enzyme of which complimentarity determining region 1 (CDR1) is formed of 11 amino-acid residues and that has a histidine residue at the 91^{st} or 55^{th} according to Kabat numbering scheme, or furthermore one of which mouse germ cell gene type is produced from a gene selected from bb1, bl1, bd2, cr1, cs1, bj2, hf24, 12-41, 19-14, 19-17, 19-23, 19-25 and 21-12 according to the classification due to Thieve et al.

As shown in examples described below, as a result of analysis of genes of antibody enzymes that are derived from a mouse and have the activity of cleaving and/or decomposing a polypeptide and antigen protein, it is experimentally confirmed that many of the antibody enzymes are produced from a mouse germ cell gene selected from, according to the classification due to Thieve et al., bb1, bl1, bd2, cr1, cs1, bj2, hf24, 12-41, 19-14, 19-17, 19-23, 19-25, and 21-12. Accordingly, antibody enzymes that are produced from a mouse germ-cell gene selected from the bb1, bl1, bd2, cr1, cs1, bj2, hf24, 12-41, 19-14, 19-17, 19-23, 19-25, and 21-12 are fundamentally antibody enzymes that have the catalytic triad residue structure in a stereostructure and the activity of cleaving and/or decomposing a polypeptide and antigen protein.

An antibody enzyme involving the invention may be a heavy chain or a light chain of an antibody. Furthermore, in the invention, a gene that codes the antibody enzyme is contained as well.

Furthermore, an antibody enzyme involving the invention is, among the abovementioned antibody enzymes, in particular, an antibody of which antigen is Helicobacter Pylori urease or a fragment of the antibody, and that is characterized in that it works as a degrading enzyme of the urease. Here, the "fragment of an antibody " means the respective peptide chains that constitute an antibody of which antigen is HP urease, and furthermore peptide fragments of a portion of region in the respective peptide chains.

An antibody enzyme, while being an antibody, has the enzymatic activity. Among the antibody enzymes described above, in particular, one that exhibits high degradation activity against the antigen protein as a target is called a "super-antibody enzyme". The "super-antibody enzyme" can completely decompose a target protein and furthermore has the activity near to that of natural enzyme (reference literature 3). The abovementioned antibody enzyme is an antibody of which antigen is HP urease and completely decomposes the HP urease. Accordingly, it is contained in the "super-antibody enzyme".

The antibody enzyme, with nature as an antibody of the HP urease strongly remained, has the nature of decomposing the HP urease that is an antigen thereof. Accordingly, the antibody enzyme is high in the specificity and can aim and decompose only the urease that is indispensable for the existence of the Helicobacter Pylori.

Furthermore, when an antibiotic drug is used as an antibacterial agent or antiviral drug, the bacteria or the viruses mutate their proteins to acquire the tolerance against the antibiotic drug, and thereby drug resistant bacterium (cell) appears. However, portions indispensable for the existence of the bacterium or viruses themselves cannot be mutated. The antibody enzyme can aim and attack the indispensable portions and can completely delete the bioactivity thereof; accordingly, reoccurrence of the symptom as well as the appearance of the drug resistant bacterium (cell) can be avoided.

The antibody enzyme preferably contains a variable region of an antibody of the HP urease. The "variable region" means, among H chains and L chains that constitute an antibody, a portion made of substantially 110 amino-acid residues from an N terminal. The "variable region" exhibits diversity in a primary structure depending on the kind of the antibody, and when the antibody has the enzymatic activity, an active center thereof is highly probably contained therein. Accordingly, when the antibody enzyme contains the variable region of the antibody, it can have high activity as enzyme.

As an antibody enzyme involving the invention, specifically, ones that are formed by containing an amino acid sequence shown in sequence No. 14 or an amino acid sequence in which in an amino acid sequence shown in the sequence No.14 at least one amino acid is replaced, deleted, inserted and/or added can be cited.

The amino acid sequence shown in sequence No.14 is a variable region of an L chain of a monoclonal antibody HpU-18 of the HP urease. The variable region of the L chain of the HpU-18, as shown also in an example described later, was recognized to be high in the activity as the degrading enzyme of the HP urease.

Furthermore, the "at least one amino acid being replaced, deleted, inserted and/or added" may be replacement, deletion, insertion and/or addition of amino acids of the number to an extent that, according to a known variant protein production process such as a site-directed mutagenesis by use of, for instance, a genetic engineering procedure, can replace, delete, insert and/or add. Thus, when the genetic engineering procedure is applied, in the amino acid sequence shown in the sequence No.14, an antibody enzyme that is made of an amino acid sequence in which at least one amino acid is replaced, deleted, inserted and/or added, in other words, is a variant of the antibody enzyme made of the amino acid sequence shown in the sequence No. 14.

As another example of an antibody enzyme involving the invention, ones that are formed by containing an amino acid sequence shown in any one of sequence Nos.15, 16, 17, 19 and 21, and furthermore ones that are formed by containing an amino acid sequence in which in an amino acid sequence shown in any one of sequence Nos.15, 16, 17, 19 and 21, at least one amino acid is replaced, deleted, inserted and/or added can be cited.

An amino acid sequence shown in sequence No.15 is a variable region of an L chain of a monoclonal antibody HpU-9 of the HP urease and an amino acid sequence shown in sequence No.16 is a variable region of an H chain of a monoclonal antibody HpU-2 of the HP urease. Furthermore, an amino acid sequence shown in sequence No.17 is a variable region of an L chain of a monoclonal antibody HpU-20 of the HP urease and an amino acid sequence shown in sequence No.19 is a variable region of an H chain of a monoclonal antibody HpU-20 of the HP urease. Still furthermore, an amino acid sequence shown in sequence No.21 is a variable region of an L chain of a monoclonal antibody UA-15 of the HP urease. Of the antibody enzymes having these amino acid sequences as well, as shown in an example described later, as a degrading enzyme of the HP urease, high activity could be recognized.

The abovementioned antibody enzymes have an action of decomposing the HP urease that is indispensable for the existence of the HP urease; accordingly, these can be used for elimination of HP bacteria. Accordingly, a curative drug involving the invention for a patient infected with the HP bacteria is prepared by containing the antibody enzyme. When the curative drug is orally taken in, it can directly attack the HP bacteria existing in a stomach; accordingly, side effects are less and it can work immediately.

The antibody enzyme can be not only used as the curative drug but also taken in orally; accordingly, it can be contained in food to take in on a daily basis, and thereby the infection with the HP bacteria can be prevented. Accordingly, in the invention, an infection preventive agent of the HP bacteria formed by containing the antibody enzyme is also contained.

The invention includes a gene that codes the antibody enzyme and when the gene is introduced in a proper host (such as bacterium and yeast), an antibody enzyme according to the invention can be expressed in the host.

As a gene involving the invention, specifically, ones made of a base sequence shown in any one of sequence Nos.18, 20, 22, 47, 48 and 49 can be cited. A base sequence shown in sequence No.18 is one of base sequences of a gene that codes a variable region of a light chain (L chain) of monoclonal antibody HpU-20 of the HP urease. A base sequence shown in sequence No.20 is a base sequence of a gene that codes a variable region of a heavy chain (H chain) of monoclonal antibody HpU-20 of the HP urease. Furthermore, a base sequence shown in sequence No.22 is a base sequence of a gene that codes a variable region of a light chain (L chain) of monoclonal antibody UA-15 of the HP urease. A base sequence shown in sequence No.47 is one of base sequences of a gene that codes a variable region of a light chain (L chain) of monoclonal antibody HpU-18 of the HP urease. A base sequence shown in sequence No.48 is one of base sequences of a gene that codes a variable region of a light chain (L chain) of monoclonal antibody HpU-9 of the HP urease. A base sequence shown in sequence No.49 is one of base sequences of a gene that codes a variable region of a heavy chain (H chain) of monoclonal antibody HpU-2 of the HP urease.

The abovementioned "gene" contains not only a double-stranded DNA but also respective single-stranded DNAs such as a sense strand and an antisense strand that constitute the double-stranded DNA and RNA. Furthermore, the "gene" may be one that contains, other than sequences that code peptides according to the invention, sequences such as a sequence of an untranslated region (UTR) or a sequence of a vector sequence (including an expression vector sequence).

Furthermore, the invention contains a transformant in which the gene is introduced. The transformant can express the antibody enzyme in its body. Accordingly, when a plant is used as a host of the transformant and the gene is introduced in the plant and expressed, a transformant that contains the antibody enzyme can be obtained. The transformant contains the antibody enzyme provided with a function of preventing the HP infection; accordingly, when it is taken in on a daily basis, the HP can be suppressed from infecting; that is, it can be used as functional food. The antibody enzyme is destroyed in its structure when heated; accordingly, as plants in which the gene can be introduced, vegetables suitable for raw diet are preferable. As such vegetables, specific example includes tomato, cucumber and carrot.

The antibody enzyme involving the invention is characterized in that it is an antibody of human-derived chemokine receptor CCR-5 or an antibody fragment thereof and can decompose the chemokine receptor CCR-5. Here, the "antibody fragment" means the respective peptide chains that constitute an antibody of which antigen is the chemokine receptor CCR-5 and further peptide fragments of a portion of a region in the respective peptide chains.

The antibody enzyme is an antibody of which antigen is the chemokine receptor CCR-5 and completely decompose the CCR-5; accordingly, it is included in the "super antibody enzyme".

The antibody enzyme, with the nature as an antibody of the chemokine receptor CCR-5 strongly remained, has the nature of decomposing the chemokine receptor CCR-5 that is an antigen thereof. Accordingly, the antibody enzyme is high in the specificity and can aim and decompose only the chemokine receptor CCR-5. The chemokine receptor CCR-5 is a coreceptor that plays an important role in the infection of the HIV; accordingly, the antibody enzyme that can delete the function thereof can be effectively utilized as an anti-HIV drug that is used in the prevention of the HIV infection or remedy of AIDS.

The antibody enzyme is preferably formed with a variable region of the chemokine receptor CCR-5 antibody contained therein. The "variable region" means, among H and L chains that constitute an antibody, a portion made of substantially 110 amino-acid residues from an N terminal. The variable region, depending on the kind of the antibody, shows the diversity in its primary structure, and, when the antibody has the activity as the enzyme, the active center thereof is probably contained therein. Accordingly, when the antibody enzyme contains the variable region, it can have high activity as an enzyme.

As an antibody enzyme involving the invention, specifically, one that is formed by containing an amino acid sequence shown in sequence No.26 or one that is formed by containing an amino acid sequence in which in an amino acid sequence shown in sequence No.26 at least one amino acid is replaced, deleted, inserted and/or added can be cited.

The amino acid sequence shown in the sequence No.26 is a variable region of a light chain (L chain) of monoclonal antibody ECL2B-2 of the chemokine receptor CCR-5. The variable region of the L chain of the ECL2B-2, as shown in an example described later, was recognized to be high in the activity as a CCR-5 degrading enzyme.

As another example of an antibody enzyme involving the invention, one that is formed by containing an amino acid sequence shown in the sequence No.30, or an amino acid sequence in which in an amino acid sequence shown in the sequence No.30, at least one amino acid is replaced, deleted, inserted and/or added can be cited.

The amino acid sequence shown in sequence No.30 is a variable region of a light chain (L chain) of monoclonal antibody ECL2B-3 of the chemokine receptor CCR-5. In the variable region of the light chain of the ECL2B-3 as well, as shown in an example described later, the activity as a CCR-5 degrading enzyme is confirmed to be high.

As still another example of an antibody enzyme involving the invention, one that is formed by containing an amino acid sequence shown in the sequence No.34, or an amino acid sequence in which in an amino acid sequence shown in the sequence No.34, at least one amino acid is replaced, deleted, inserted and/or added can be cited.

The amino acid sequence shown in sequence No.34 is a variable region of a light chain (L chain) of monoclonal antibody ECL2B-4 of the chemokine receptor CCR-5. In the variable region of the light chain of the ECL2B-4 as well, as shown in an example described later, the activity as a CCR-5 degrading enzyme is confirmed to be high.

As further still another example of an antibody enzyme involving the invention, one that is formed by containing an amino acid sequence shown in the sequence No.36, or an amino acid sequence in which in an amino acid sequence shown in the sequence No. 36, at least one amino acid is replaced, deleted, inserted and/or added can be cited.

An amino acid sequence shown in sequence No.36 is a variable region of a heavy chain (H chain) of monoclonal antibody ECL2B-4 of the chemokine receptor CCR-5. In the variable region of the L chain of the ECL2B-4 as well, as shown in an example described later, the activity as a CCR-5 degrading enzyme is confirmed to be high.

Furthermore, the "at least one amino acid being replaced, deleted, inserted and/or added" may be replacement, deletion, insertion and/or addition of amino acids of the number to an extent that, according to a known variant protein production process such as a site-directed mutagenesis by use of, for instance, a genetic engineering procedure, can be replaced, deleted, inserted and/or added. Thus, when the genetic engineering procedure is applied, an antibody enzyme that is made of an amino acid sequence in which, in the amino acid sequence shown in any one of the sequence Nos.26, 30, 34 and 36, at least one amino acid is replaced, deleted, inserted and/or added is, in other words, a variant of the antibody enzyme made of the amino acid sequence shown in any one of the sequence Nos.26, 30, 34 and 36.

The antibody enzyme according to the invention decomposes the chemokine receptor CCR-5 that plays an important role in the HIV infection to delete the function thereof; accordingly, it can be used also as an anti-HIV drug that disturbs the HIV infection. That is, the anti-HIV drug according to the invention is characterized by containing the antibody enzyme and by disturbing the infection of the AIDS viruses.

Furthermore, the antibody enzyme according to the invention can delay the development of the AIDS to an HIV infected patient or inhibit disease from progressing. That is, the anti-HIV drug according to the invention, containing the antibody enzyme, may be one that works as a curative drug against the AIDS virus-infected patient.

The anti-HIV drug according to the invention, containing the antibody enzyme alone, can be used by directly administering by means of such as intravenous injection. However, in addition to the antibody enzyme, a carrier that can be physiologically tolerated may be further contained. Production of such an anti-HIV drug can be carried out according to a so far known production process. The anti-HIV drug uniquely aims and decomposes a CCR-5 molecule alone; accordingly, it can be expected that not only the effect is remarkable but also the side effects are less.

The invention includes also a gene that codes the antibody enzyme. When the gene is expressibly introduced in an appropriate host (such as bacterium and yeast), the antibody enzyme according to the invention can be expressed in the host.

The abovementioned "gene" contains not only a double-stranded DNA but also respective single-stranded DNAs such as a sense strand and an antisense strand that constitute the double-stranded DNA and RNA. Furthermore, the "gene" contains, other than sequences that code the antibody enzymes according to the invention, sequences such as a sequence of an untranslated region (UTR) or a sequence of a vector sequence (including an expression vector sequence).

As the gene involving the invention, specifically, one that is formed of a base sequence shown in sequence No.27 can be cited. The base sequence shown in sequence No.27 is a base sequence of a gene that codes a variable region of a light chain (L chain) of monoclonal antibody ECL2B-2 of the chemokine receptor CCR-5.

As another example of the gene involving the invention, one that is formed of a base sequence shown in the sequence No. 31 can be cited. The base sequence shown in the sequence No.31 is a base sequence of a gene that codes a variable region of a light chain (L chain) of monoclonal antibody ECL2B-3 of the chemokine receptor CCR-5.

As still another example of the gene involving the invention, one that is formed of a base sequence shown in the sequence No.35 can be cited. The base sequence shown in the sequence No.35 is a base sequence of a gene that codes a variable region of a light chain (L chain) of monoclonal antibody ECL2B-4 of the chemokine receptor CCR-5.

As further still another example of the gene involving the invention, one that is formed of a base sequence shown in the sequence No.37 can be cited. The base sequence shown in the sequence No.37 is a base sequence of a gene that codes a variable region of a heavy chain (H chain) of monoclonal antibody ECL2B-4 of the chemokine receptor CCR-5.

Furthermore, in the invention, a transformant in which the gene is introduced is also included. The transformant is one in which the gene is expressibly introduced in an appropriate host (such as bacteria and yeast) and can express the antibody enzyme according to the invention in its body. Accordingly, the transformant as well can be utilized as an anti-HIV drug that prevents the infection of the AIDS viruses and an anti-HIV drug for treating an AIDS-infected patient.

Still furthermore, the invention contains a computer program that instructs a computer to carry out an antibody structure analysis process in the antibody enzyme production process; and a machine-readable recording medium in which a computer program that instructs a computer to carry out an antibody structure analysis process is recorded.

### Brief Description of the Drawings

Fig.1A is a flow chart showing an example of an antibody enzyme production process involving an embodiment in the present invention, and Fig.1B is a flow chart showing an example of an antibody enzyme production process involving another embodiment in the present invention.
Fig.2 is a block diagram showing, in the antibody enzyme production process involving an embodiment of the invention, an example of a constitution of a system that performs an antibody structure analysis process.
Fig.3 is a flow chart showing a treatment procedure of the antibody structure analysis process shown in Fig. 2.
Fig.4A is a diagram showing an amino acid sequence (primary structure) of a variable region of a heavy chain of antibody i41SL1-2 and a base sequence that codes the amino acid sequence, and Fig.4B is a diagram showing an amino acid sequence (primary structure) of a variable region of a light chain of the antibody i41SL1-2 and a base sequence that codes the amino acid sequence.
Fig.5A is a diagram showing a three-dimensional stereostructure of a variable region of a light chain of the antibody i41SL1-2 and Fig.5B is a diagram showing a three-dimensional stereostructure of a variable region of a heavy chain of the antibody i41SL1-2.
Fig.6A is a diagram showing results of a polypeptide decomposition reaction of a light chain of the antibody i41SL1-2 and Fig.6B is a diagram showing results of a polypeptide decomposition reaction of a heavy chain of the antibody i41SL1-2.
Fig.7A is a diagram showing results of kinetic analysis of the polypeptide decomposition reaction of a light chain of the antibody i41SL1-2 and Fig.7B is a diagram showing results of kinetic analysis of the polypeptide decomposition reaction of a heavy chain of the antibody i41SL1-2.
Fig.8A is a diagram showing an amino acid sequence (primary structure) of a variable region of a heavy chain of antibody i41-7 and a base sequence that codes the amino acid sequence, and Fig.8B is a diagram showing an amino acid sequence (primary structure) of a variable region of a light chain of the antibody i41-7 and a base sequence that codes the amino acid sequence.
Fig.9A is a diagram showing a three-dimensional stereostructure of a variable region of a light chain of the antibody i41-7, Fig.9B is a diagram that sees from another angle a three-dimensional stereostructure of a variable region of a light chain of the antibody i41-7, and Fig.9C is a diagram showing a three-dimensional stereostructure of a variable region of a heavy chain of the antibody i41-7.
Fig.10A includes diagrams showing results of polypeptide decomposition reactions. In Fig.10A, a right one shows results of the polypeptide decomposition reactions of a light chain of the antibody i41-7 and left one shows results of the polypeptide decomposition reactions of a heavy chain of the antibody i41-7. Fig.10B includes electrophoretograms and a diagram showing results of decomposition reactions of an antigen protein (inactive 41S-2-L) due to a light chain of the antibody i41-7.
Fig.11A is a diagram showing a three-dimensional stereostructure of light and heavy chains of 1DBA clone, and Figs.11B and 11C are diagrams in which portions of the above diagram are enlarged.
Fig.12 is a diagram showing results of the polypeptide decomposition reactions of an i41-7 intact antibody, heavy and light chains of the antibody i41-7 and a MA-2 antibody.
Fig.13 is a diagram showing an amino acid sequence of a variable region of a HpU-18 L chain and a base sequence of a gene that codes the amino acid sequence.
Fig.14 is a diagram showing a stereostructure of a variable region of the HpU-18 L chain.
Fig.15 is a diagram showing an amino acid sequence in a variable region of a HpU-9 L chain and a base sequence of a gene that codes the amino acid sequence.
Fig.16 is a diagram showing a stereostructure of a variable region of the HpU-9 L chain.
Fig.17 is a diagram showing an amino acid sequence of a variable region of a HpU-2 H chain and a base sequence of a gene that codes the amino acid sequence.
Fig.18 is a diagram showing a stereostructure of a variable region of the HpU-2 H chain.
Fig.19 is a diagram showing results of enzymatic activity tests due to an HpU antibody in the present embodiment.
Fig.20 is a diagram showing results of agarose gel electrophoresis of an amplification product containing HpU-2 antibody fragment genes amplified by PCR in the embodiment.
Fig.21 is a diagram showing a stereostructure of a variable region of the HpU-20 L chain.
Fig.22 is a diagram showing a stereostructure of a variable region of the HpU-20 H chain.
Fig.23 is a diagram showing an amino acid sequence in a variable region of an HpU-20 L chain and a base sequence of a gene that codes the amino acid sequence.
Fig.24 is a diagram showing an amino acid sequence of a variable region of an HpU-20 H chain and a base sequence of a gene that codes the amino acid sequence.
Fig.25 is a diagram showing results (Lot 1) of decomposition experiments in which TP41-1 peptide (denoted as TP in the drawing) is decomposed with HpU-20-L and HpU-20-H.
Fig.26 is a diagram showing results (Lot 2) of decomposition experiments in which TP41-1 peptide (denoted as TP in the drawing) is decomposed with HpU-20-L and HpU-20-H.
Fig.27 is a diagram showing results when, in a decomposition experiment where proteins are decomposed with a HpU-20 L chain, SDS-PAGE is carried out of samples at 1 hr after the start of the reaction.
Fig.28 is a diagram showing results when, in an experiment where proteins are decomposed with a HpU-20 L chain, SDS-PAGE is carried out of samples at 1 hr after the start of the reaction.
Fig.29 is a diagram showing results when, in an experiment where proteins are decomposed with a HpU-20 L chain, SDS-PAGE is carried out of samples at 1 hr after the start of the reaction.
Fig.30 is a diagram showing a stereostructure estimated after stereostructure modeling (molecular modeling) of a variable region of UA-15 made of light and heavy chains. In Fig.30, it is shown that only on a light chain, a catalytic triad residue structure appears.
Fig.31 is a diagram showing results (Lot 1) of decomposition experiments in which TP41-1 peptide (denoted as TP in the drawing) is decomposed with UA-15-L (denoted as L in the drawing) and UA-15-H (denoted as H in the drawing).
Fig.32 is a diagram showing results (Lot 2) of decomposition experiments in which TP41-1 peptide (denoted as TP in the drawing) is decomposed with UA-15-L (denoted as L in the drawing) and UA-15-H (denoted as H in the drawing).
Fig.33 is a diagram showing results of decomposition experiments where TP41-1 peptide (denoted as TP in the drawing) is decomposed with UA-15-L (denoted as L in the drawing) at respective temperatures of 15, 25 and 37 degrees centigrade. In the decomposition experiments, after experiments of which results are shown in Fig. 31 are carried out, TP41-1 peptide is once more added and difference of reaction temperatures is checked.
Fig.34 is a diagram showing results of kinetic analysis of the decomposition reaction of TP41-1 peptide with UA-15-L.
Fig.35 is a diagram showing a stereostructure of a variable region of ECL2B-2.
Fig.36 is a diagram showing a stereostructure of a variable region of ECL2B-3.
Fig.37 is a diagram showing, in comparison, amino acid sequences of i41SL1-2-L, VIPase-L and ECL2B-2-L.
Fig.38 is a diagram showing results when an enzyme activity test is carried out of ECL2B-2-L and ECL2B-3-L.
Fig.39 is a diagram showing a stereostructure of a variable region of ECL2B-4-L.
Fig.40 is a diagram showing a stereostructure of a variable region of ECL2B-4-H.
Fig.41 is a diagram showing an amino acid sequence (primary structure) of a variable region of ECL2B-4-L and a base sequence that codes the amino acid sequence.
Fig.42 is a diagram showing an amino acid sequence (primary structure) of a variable region of ECL2B-4-H and a base sequence that codes the amino acid sequence.
Fig.43 is a diagram showing results of decomposition experiments in which ECL2B peptide is decomposed with ECL2B-4-L (denoted as L in the drawing) and ECL2B-4-H (denoted as H in the drawing).
Fig.44A is a diagram showing results when, in a decomposition reaction of ECL2B peptide with ECL2B-4-L, a concentration of the ECL2B is monitored with HPLC. Fig.44B is a diagram showing results when, in a decomposition reaction of ECL2B peptide with ECL2B-4-H, a concentration of the ECL2B is monitored with HPLC. Fig.44C is a diagram showing results when, as a control of the decomposition reaction of ECL2B peptide, a concentration of the ECL2B is monitored with HPLC.
Figs.45A and 45B are diagrams showing results of kinetic analysis in the enzyme decomposition experiment. Fig.45A is a diagram showing relationship between a substrate (ECL2B peptide) concentration and a decomposition rate, and Fig.45B is a diagram showing a result of Hanes-Woolf plot.
Fig.46 is a diagram showing results of decomposition experiments in which TP41-1 (denoted as TP in the drawing) peptide is decomposed with ECL2B-4-L (denoted as L in the drawing) and ECL2B-4-H (denoted as H in the drawing).
Fig.47A is a diagram showing results when, in a decomposition experiment of TP41-1 peptide with ECL2B-4-L, a concentration of the TP41-1 is monitored with HPLC. Fig.47B is a diagram showing results when, in a decomposition experiment of TP41-1 peptide with ECL2B-4-H, a concentration of the TP41-1 is monitored with HPLC. Fig.47C is a diagram showing results when, as a control of a decomposition experiment of TP41-1 peptide, a concentration of the TP41-1 is monitored with HPLC.
Figs.48A and 48B are diagrams showing results of kinetic analysis in the enzyme decomposition experiment. Fig.48A is a diagram showing relationship between a substrate (TP41-1 peptide) concentration and a decomposition rate, and Fig.48B is a diagram showing a result of Hanes-Woolf plot.
Fig.49 is a diagram showing results when catalytic triad residue structures and germline genes are analyzed by use of PDB data.
Fig.50 is a diagram showing results when catalytic triad residue structures and germline genes of clones that the inventors have are analyzed

### Best Mode for Carrying Out the Invention

### [Embodiment 1]

A production process according to the present invention of antibody enzymes and an antibody enzyme produced according to the production process will be described as embodiment 1. However, the present invention is not restricted thereto.

The invention proposes an antibody enzyme production method that not only enables to efficiently obtain natural antibody enzymes but also can produce antibody enzymes by use of a genetic engineering process, and further provides an example of novel and useful antibody enzymes obtained according to the process. In what follows, an antibody enzyme production process involving the invention will be described, followed by describing an example of obtained antibody enzymes and gene thereof, functions of the antibody enzyme, and applications thereof.

### (1) Antibody enzyme and catalytic triad residue structure

The inventors analyzed in detail features of nature and structure of several kinds of antibody enzymes having the activity of cleaving and/or decomposing polypeptides and antigen proteins. As a result, the inventors for the first time revealed that the antibody enzymes having the activity of cleaving and/or decomposing polypeptides and antigen proteins all have a catalytic triad residue structure in a stereostructure thereof.

Specifically, that a catalytic triad residue structure is present in a stereostructure thereof means that in a stereostructure of an antibody or an antibody fragment a serine residue, an aspartate residue and a histidine residue or a glutamate residue are present stereostructurally in proximity, and, more specifically, it is better that distances between a serine residue, an aspartate residue, and a histidine residue or a glutamate residue, each other, are at least in the range of 3 to 20 Å, preferably in the range of 3 to 10 Å. This is because when distances between functional groups that constitute the catalytic triad residue structure are in the range of 3 to 20 Å, in particular, in the range of 3 to 10 Å, the catalytic triad residue structure and a substrate (such as polypeptide and antigen protein) are considered to be reactable.

In an antibody enzyme involving the invention, a site where the catalytic triad residue structure is present is not particularly restricted. An antibody is constituted of a heavy chain (H chain) and a light chain (L chain). The light chain is constituted of a variable region (VR) and a constant region (CR), and the variable region includes a complimentarity determining region (CDR). As shown in an example described later, it is revealed that the catalytic triad residue structure is mainly present in light chains; however it is present as well in heavy chains though not so much as in the light chain. Furthermore, also experimentally, it is revealed that an antibody that has the catalytic triad residue structure in a stereostructure has the enzymatic activity of cleaving and/or decomposing a polypeptide or an antigen protein.

Accordingly, it is better that in the antibody enzyme production process involving the invention, whether an antibody has the catalytic triad residue structure therein or not can be judged.

### (2) Outline of antibody enzyme production process

An antibody enzyme production process involving the invention may include at least an antibody structure analysis process that carries out a stereostructure estimation step where from amino acid sequences a stereostructure of an antibody is estimated; and a catalytic triad residue structure confirmation step where whether a catalytic triad residue structure is present in the estimated stereostructure of an antibody or not is judged. As is obvious from the knowledge in the (1), when the catalytic triad residue structure is included in a stereostructure of an antibody, the antibody is very probable to be an antibody enzyme. Accordingly, thereby, antibody enzymes can be efficiently screened.

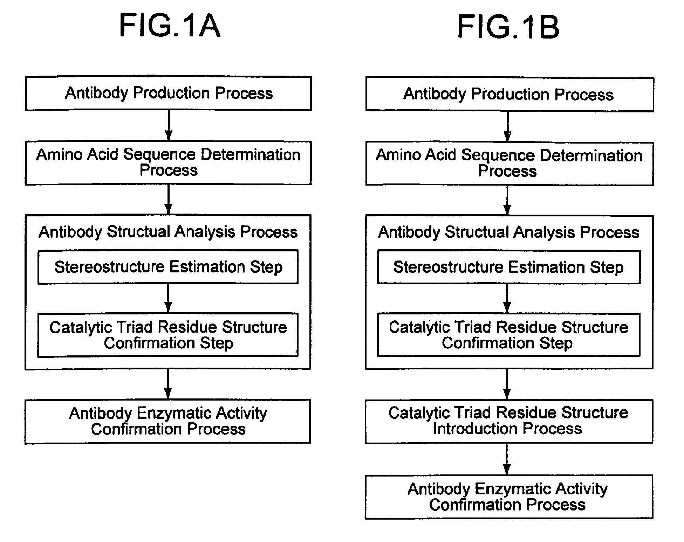

When an example of the antibody enzyme production process involving the invention is more specifically described, as shown in Fig. 1A, the production process may include an antibody production step, an amino acid sequence determination step, an antibody structure analysis step and an antibody enzymatic activity confirmation step.

### (2-1) Antibody production step

The antibody production step, without restricting to particular one, may produce monoclonal antibodies from hybridomas obtained by fusing mouse spleen lymph corpuscles immunized with antigens and mouse myeloma cells, alternatively, antibodies obtained from a library by use of a phage display method may be used.

Specifically, when, as an immunogen, a desired antigen, or a fragment containing an antigenic determinant (epitope) thereof, or a derivative thereof, or an analogue thereof, or a cell that can express these is used, an antibody can be produced. These are carried out according to an ordinary immunization operation, or a hybridoma method (reference literature 5: Kohler, G. and Milstein, C., Nature 256, 495-497 (1975)), a trioma method, a human B cell hybridoma method (reference literature 6: Kozbor, Immunology Today 4, 72, 1983), and an EBV-hybridoma method (reference literature 7: Monoclonal Antibodies and Cancer Therapy, Alan R. Liss, Inc., pp. 77-96, 1985).

Here, as far as the antigen is a polypeptide or a protein, or a polysaccharide, there is no particular restriction.

Specifically, when the antigen is a hapten, it does not have the capability of producing antibodies; accordingly, it cannot produce the antibody. However, when an antigen is covalently bonded with a carrier made of a biopolymer such as a protein derived from a different kind to obtain an antigen protein and this is used to immunize, the antibody production can be induced. As the carrier, without restricting to particular one, various kinds of proteins so far known in the field such as ovalbumin, gamma globulin, and hemocyanine can be preferably used.

The "polypeptide" here includes short peptides in which several amino acids are bonded and proteins. Furthermore, the polypeptide includes naturally existing ones and chemically, biologically synthesized ones. Still furthermore, the "polysaccharide" here means ones in which several of so-called monosaccharides such as glucose, maltose, acetylglucosamine, and glucuronic acid are bonded. Kinds of monosaccharide that constitutes the "polysaccharide" are not particularly restricted as far as these are so far known ones.

### (2-2) Amino acid sequence determination step

The amino acid sequence determination step, as far as an amino acid sequence can be determined from an antibody obtained in the antibody production step, is not particularly restricted. Specifically, the amino acid sequence may be determined by directly reading an amino acid sequence from the obtained antibody, or an amino acid sequence may be estimated from a base sequence of a gene of the antibody. The amino acid sequence may be determined by use of an Edman method. Furthermore, the base sequence may be determined by use of a so far known sequencer, and the amino acid sequence may be estimated by use of a so far known soft wares and so on. These are not particularly restricted.

### (2-3) Antibody structure analysis process

The antibody structure analysis process, as far as it includes a stereostructure estimation step and a catalytic triad residue structure confirmation step, is not particularly restricted. Thereby, whether a desired antibody includes a catalytic triad residue structure or not can be efficiently confirmed.

The stereostructure estimation step, as far as it can estimate a stereostructure from data of the amino acid sequences obtained in the amino acid sequence determination step, is not particularly restricted. Specifically, all that is necessary is to be able to estimate a secondary structure and a tertiary structure in a protein. That is, in the invention, all that is necessary is to clarify up to a tertiary structure of an IgG class antibody.

As a quaternary structure in an antibody, like for instance IgA and IgM class, a structure in which a plurality of subunits (IgG class antibody) having a tertiary structure meets to form an oligomer can be cited. However, such a structure is not particularly necessary in a later catalytic triad residue structure confirmation step; accordingly, it is not necessary to estimate in the invention. However, when an analysis result including a result of the quaternary structure can be effectively utilized in the production of antibody enzymes, without being restricted to the above, a quaternary structure of an antibody may be estimated in the stereostructure estimation step.

When the stereostructure estimation step is carried out, a so far known higher-order structure analysis soft ware may be used, and a specific procedure thereof is not particularly restricted.

In the catalytic triad residue structure confirmation step, as far as whether the catalytic triad residue structure described in (1) is present in a stereostructure of an antibody or not can be judged, there is no particular restriction. Accordingly, when the catalytic triad residue structure is judged, presence of the abovementioned structure may be judged directly or indirectly.

As a direct index that is used in the judgment of the catalytic triad residue structure, the respective indices below can be cited. As a step prior to judgment by use of the respective indices below, a more simple method where whether a histidine residue is present in an amino acid sequence of an antibody or not is investigated may be used to judge whether the catalytic triad residue structure is present or not. This is because in many antibodies, usually, in comparison with the serine residue, the histidine residue is less contained.

In a first index, whether the catalytic triad residue structure has a structure same as a catalytic triad residue structure common in known antibody enzymes or not, alternatively, when, among the known catalytic triad residue structures, only one residue thereof uses an amino-acid residue in a different position, whether it is estimated that a catalytic triad residue structure can be constituted or not is made an index, the former case is called a type ①, and the latter one is called a type ①*.

In a second index, a case where in a single antibody heavy chain (H chain) or a single antibody light chain (L chain), in a stereostructure, in the range of 3 to 20 Å, the respective functional groups of a serine residue, an aspartate residue, and a histidine residue or glutamate residue are present is made an index, and this is called a type ② index.

In the next place, in the catalytic triad residue structure confirmation step, as an indirect index that is used to judge a catalytic triad residue structure, though not particularly restricted, in the embodiment, an index that makes use of an analysis result of germline genes can be preferably used.

The antibody light chain (L chain) can be classified into two kinds of classes, κ and λ. In the case of a mouse, it is said that substantially 95% is the κ chain. Furthermore, since analysis of germline genes of mouse κ chains is being advanced, information thereof can be advantageously obtained. As will be described in an example described later, by analyzing the germlines, a very important knowledge that suggests that in particular germlines an antibody L chain having the enzymatic activity is already prepared is found (example 1-3 and Table 3). In this connection, when germlines from which known antibody enzymes are derived are investigated in advance and antibodies having the germlines are selected, of the light chains, antibody enzymes can be efficiently obtained at a high probability. This is made a type ③ index.

As the germlines that can be used for the light chains, specifically, according to a classification due to Thiebe et al., bb1, cr1, bl1, cs1, bd2, bj2 or 19-25 can be cited. In the present embodiment and example, in the case of light chains, the germlines will be classified according to Thiebe et al.

Furthermore, also of the heavy chains (H chain), when a method of thinking of the light chains is applied, when antibodies having Families of VH1, VH5 and VH10 are selected, antibody enzymes can be efficiently obtained at a very high probability. In the aging process of antibodies, mutation is caused, and some may lose the catalytic triad residue structure; however, when, after the removal of these, the germline or the Family is identified, substantially completely antibodies having the enzymatic activity can be obtained.

In addition, a structurally characteristic constitution obtained from known antibody enzymes as well can be used as an index. Specifically, as will be described in an example described below, a feature that a complimentarity determining region 1 (CDR1) is constituted of 16 amino-acid residues and a histidine residue is present at the 93^{rd} according to the Kabat numbering scheme is remarkable for the antibody enzyme. Accordingly, this can be used as an index of type ④. Furthermore, a feature that a complimentarity determining region 1 (CDR1) is constituted of 11 amino-acid residues and a histidine residue is present at the 91^{st} or 55^{th} according to the Kabat numbering scheme is also remarkable for the antibody enzyme; accordingly, this can be used as an index of type ④*. In the present embodiment and example, an amino acid sequence of an antibody is expressed according to the Kabat numbering scheme.

More sure indexes are the types ① · ①* or ②; accordingly, in order to heighten the assuredness at the selection, these indices are preferably used, and type ③ and types ④ and ④* may be used complementarily. However, without using the indices of the types ①, ①* and ②, the indices of type ③ and types ④ and ④* alone may be used singularly or in a combination.

As means for carrying out an antibody structure analysis process including the catalytic triad residue structure confirmation step, in the embodiment, an antibody structure analysis system described later in (3) can be cited. However, it goes without saying that the invention is not restricted thereto.

Furthermore, in the case of a stereostructure of an antibody being directly estimated from data of base sequences of a gene by use of an antibody structure analysis system, the amino acid sequence determination step may be contained as one step of the antibody structure analysis process. That is, as described in the amino acid sequence determination step, an amino acid sequence determination step where an amino acid sequence is estimated from base sequences of a gene of an antibody may be carried out as one step of the antibody structure analysis process.

### (2-4) Antibody enzymatic activity confirmation step

The antibody enzymatic activity confirmation step, as far as it can confirm, after the analysis in the antibody structure analysis step, whether an antibody that has a catalytic triad residue structure and is very high in the likelihood of being an antibody enzyme actually has the activity of cleaving and/or decomposing a polypeptide and an antigen protein or not, is not particularly restricted. Specifically, a polypeptide or an antigen protein that includes a structure that becomes an antigen or an antigen determinant may be reacted with an antibody to check the activity. At this time, reaction conditions such as a concentration of the antigen, a concentration of the antibody, a reaction temperature and a reaction time, and a concentration of a base (kind of buffer used) are not particularly restricted.

### (2-5) Catalytic triad residue structure introduction step

As another example of the antibody enzyme production method involving the invention, as shown in Fig.1B, a catalytic triad residue structure introduction step may be included. That is, even antibodies that inherently do not have a catalytic triad residue structure, when a catalytic triad residue structure is introduced therein according to a genetic engineering process, can produce antibody enzymes with high efficiency. Accordingly, after the antibody structure analysis step, a catalytic triad residue structure introduction step may be carried out.

In the catalytic triad residue structure introduction step, all that is necessary is to introduce a catalytic triad residue structure, by use of stereostructure information obtained in the antibody structure analysis process, in an antibody according to a genetic engineering process, and a specific procedure is not particularly restricted. Specifically, so far known methods such as a method of preparing a deletion mutant by use of an exonuclease and site-directed mutagenesis can be preferably applied.

The catalytic triad residue structure introduction step may be carried out after the antibody structure analysis process, and it is very preferable that after the catalytic triad residue structure introduction step, an antibody enzymatic activity confirmation step is carried out to judge whether, when the catalytic triad residue structure is introduced, the activity of cleaving and/or decomposing a polypeptide or an antigen protein can be imparted or not.

### (3) System of carrying out antibody structure analysis process

### (3-1) Example of specific constitution of antibody structure analysis system

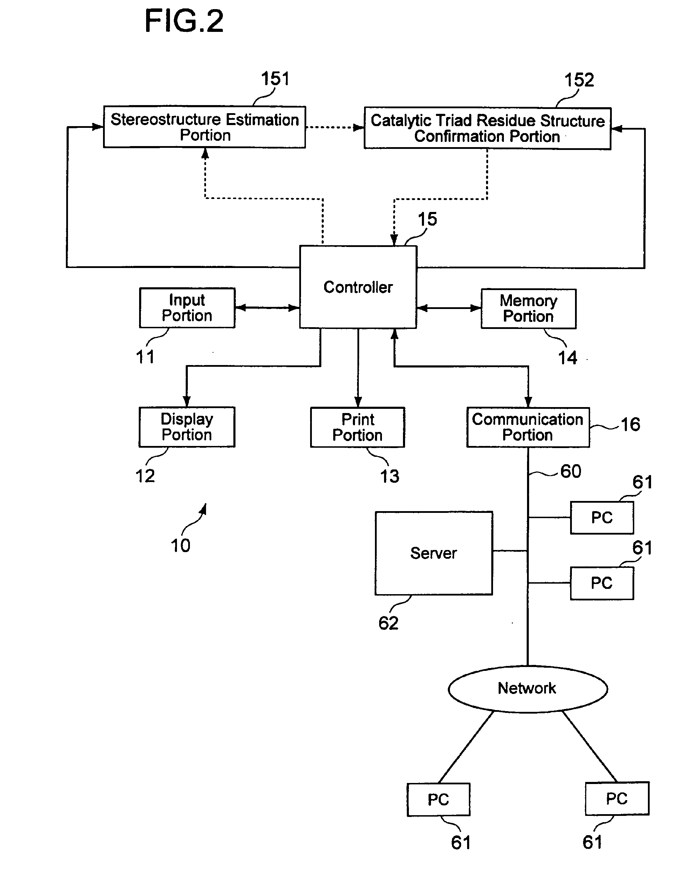

Means of carrying out the antibody structure analysis process according to the invention may be an antibody structure analysis system that includes a computer. For instance, as shown in Fig. 2, an antibody structure analysis system 10 including an input portion 11, a display portion 12, a printing portion 13, a memory portion 14, a controller 15, a stereostructure estimation portion 151 and a catalytic triad residue structure confirmation portion 152 can be cited.

The input portion 11, as far as it enables to input information involving an operation of the antibody structure analysis system 10, is not particularly restricted. So far known input means such as a keyboard, a tablet or a scanner can be preferably used.

The display portion 12 displays various kinds of information such as information involving an operation of the antibody structure analysis system 10 and selection results including an estimated stereostructure of the antibody and a confirmation result of the catalytic triad residue structure. Specifically, various kinds of display devices such as known CRT displays and liquid crystal display devices can be preferably used; however, there is no particular restriction.

The printing portion 13 records (prints or picturizes) various kinds of information that can be displayed by the display portion 12 on recording materials such as PPC paper. Specifically, known image formation devices such as an ink jet printer and a laser printer can be preferably used; however, there is no particular restriction.

The display portion 12 and printing portion 13 in combination can be represented as output means. That is, the display portion 12 is means for outputting various kinds of information in a soft copy and the printing portion 13 is means for outputting various kinds of information as a hard copy. Accordingly, as the outputting means used in the invention, without restricting to the display portion 12 and the printing portion 13, other outputting means may be provided.

The memory portion 14 memorizes various kinds of information (such as control information, selected results and other information) that is used in the antibody structure analysis system 10. Specifically, so far known various kinds of memory means such as semiconductor memories such as RAMs and ROMs, magnetic discs such as floppy discs and hard discs, disc types of optical discs such as CD-ROM/MO/MD/DVD and card types such as IC cards (including memory cards)/optical cards can be preferably used.

Furthermore, the memory portion 14 may be integrated with the antibody structure analysis system 10 to form one device, or isolated as an external memory device, or constituted so as to have both the integrated memory portion 14 and the external memory device. For instance, as the integrated memory portion 14, a built-in type hard-disc drive and a floppy disc-drive, a CD-ROM drive or a DVD-ROM drive incorporated in a device can be cited, and, as an external memory device, an external hard disc and external types of the abovementioned various kinds of disc-drives can be cited.

The controller 15 controls an operation of the antibody structure analysis system 10. Specifically, as shown in Fig.2 with solid arrow marks, to the respective means of the input portion 11, the display portion 12, the printing portion 13, the memory portion 14, the stereostructure estimation portion 151 and the catalytic triad residue structure confirmation portion 152, the control information is outputted from the controller 15. On the basis of the control information, the respective means operate in combination to drive the antibody structure analysis system 10 as a whole. Furthermore, to the controller 15, instruction information to drive the antibody structure analysis system 10 can be inputted from the input portion 11. Accordingly, in Fig.2, the solid arrow marks that show an exchange of the control information are bi-directional.

The stereostructure estimation portion 151 and the catalytic triad residue structure confirmation portion 152 work in combination as analysis means. Specifically, to the stereostructure estimation portion 151, the amino acid sequence data inputted from the input portion 11 are inputted through the controller 15 (dotted line in the drawing). Then, data of the stereostructure generated at the stereostructure estimation portion 151 are outputted to the catalytic triad residue structure confirmation portion 152 (dotted line in the drawing). In the catalytic triad residue structure confirmation portion 152, from the stereostructure data, whether the catalytic triad residue structure is present or not is confirmed, and based on the confirmation result, final analysis data are generated (dotted line in the drawing). The analysis data, as far as the data can be outputted by outputting means such as the display portion 12 and/or printing portion 13, are not particularly restricted.

The data that are used to analyze a stereostructure in the stereostructure estimation portion 151 may be at least data of the amino acid sequences; however, it may be data of the base sequences. In this case, all that are necessary for the stereostructure estimation portion 151 are to estimate amino acid sequences from the data of base sequences to generate a stereostructure based on the data.

Specific constitutions of the controller 15, the stereostructure estimation portion 151 and the catalytic triad residue structure confirmation portion 152 are not particularly restricted; that is, so far known calculation means can be preferably used. The respective means may be independently constituted from each other; however, the respective means are preferably integrated into one calculation means to be an integrated control and analysis system. Specifically, it is very preferable that the respective means are integrated as a central processing unit (CPU) of a computer and an operation thereof is carried out according to a computer program.

Accordingly, the stereostructure estimation portion 151 may carry out a calculation where a CPU estimates a stereostructure according to a so far known computer program that estimates a stereostructure of a protein; and the catalytic triad residue structure confirmation portion 152 may carry out a calculation where a CPU determines at least whether a catalytic triad residue structure is present or not according to a computer program. In addition, though not shown in the drawing, the antibody structure analysis system 10 may include other analysis means and, as analysis data, other data other than that of the catalytic triad residue structure and the stereostructure.

Furthermore, the antibody structure analysis system 10 may be constituted so that, by providing a communication portion 16 as shown in Fig.2, various kinds of information may be inputted and outputted through a communication network including the Internet. The communication portion 16 is connected with a communication network and can send and receive various kinds of information. In Fig.2, the antibody structure analysis system 10, a personal computer (PC) 61 and a server 62 in the same premise are connected to a communication line 60 to form a bus type LAN (local area network), and further the LAN is connected with a PC 61 in other area through the Internet.

A specific configuration of the communication portion 16 is not particularly restricted. Known LAN cards, LAN boards, LAN adaptors and modems can be preferably used.

As the PC 61, known personal computers provided with communication means such as a modem can be preferably used and the PC 61 is not restricted to desktop type and note-type ones. The PC 61 has a fundamental constitution that has a display portion such as a CRT display or a liquid crystal device and an input portion such as a keyboard or a mouse. For convenience of explanation, a display portion and input portion that are provided to the PC 61 and not shown in the drawing are expressed as a PC display portion and a PC input portion.

The PC 61 may be provided with a hardware (various kinds of input means such as a scanner and various kinds of output means such as a printer) that can be externally connected to a general personal computer.

A specific configuration of the server 62 is neither particularly restricted and may be a computer that can offer a service to the PCs 61 that are clients constituting the LAN and the antibody structure analysis system 10. Furthermore, the server 62 may combine a data base server and a file service server.

A specific configuration of the communication line 60 is neither particularly restricted; that is, so far known general communication lines can be used. Furthermore, a type and a bus type of the LAN that is constituted with the communication line 60 are neither particularly restricted; that is, so far known type such as a star type and a ring type may be used.

Furthermore, though not shown in the drawing, the LAN may include shared printers and other terminals. Still furthermore, through not shown in the drawing, in the communication network including the LAN, various kinds of communicable and portable terminals may be included.

In a network having the abovementioned configuration, for instance, after the antibody structure analysis system 10 performed the antibody structure analysis process, the selected results can be not only outputted simply in the antibody structure analysis system 10 (that is, such as the display portion 12 and printing portion 13) but also transmitted through the LAN to the PCs 61. In the PC 61, results obtained from the antibody structure analysis system 10 can be displayed on the PC display portion and printed out by use of a printer, and furthermore owing to an input from the PC input portion the selected results can be processed as well.

That is, the communication portion 16 can function not only as communication means but also as input means of the antibody structure analysis system 10. Furthermore, in particular, when, by use of the PC61, through the Internet, at an area remote from a location where the antibody structure analysis system 10 is located, information (such as amino acid sequences) involving implementation of the antibody structure analysis process is transmitted or selected results are received, to optional customers, a service that provides the antibody structure analysis process can be provided.

Furthermore, in the case of the PCs 61 being connected through the LAN with the antibody structure analysis system 10, when one antibody structure analysis system 10 is in, for instance, a research facility, other researchers can share the antibody structure analysis system 10 through an information terminal such as the PC 61. Accordingly, the present invention can be more efficiently carried out.

Still furthermore, in the case of the server 62 combining a database server and a file server, selected results of the antibody structure analysis process that is performed through a communication network can be stored through the communication network in the server 62. As a result, the selected results can be more efficiently used.

In addition, in the invention, the antibody structure analysis process in the invention can be carried according to a program on a computer. However, in a recording medium that records the program, a medium that carries a program in a volatile manner such as downloading from a communication network is also included. For instance, when a program of the antibody structure analysis process is recorded in recording means of the server 62, the antibody structure analysis system 10 may appropriately download the program of the antibody structure analysis process from the server 62 to use. However, when the antibody structure analysis system 10 downloads the program from a communication network, a program for downloading is stored in advance in a body of the antibody structure analysis system 10 or can be installed from a separate recording medium.

Furthermore, like the PC 61, when it is connected through a communication network to the server 62, when a program of the antibody structure analysis process is downloaded from the server 62, the PC 61 itself can be used as the antibody structure analysis system 10.

### (3-2) Example of antibody structure analysis process carried out by the antibody structure analysis system

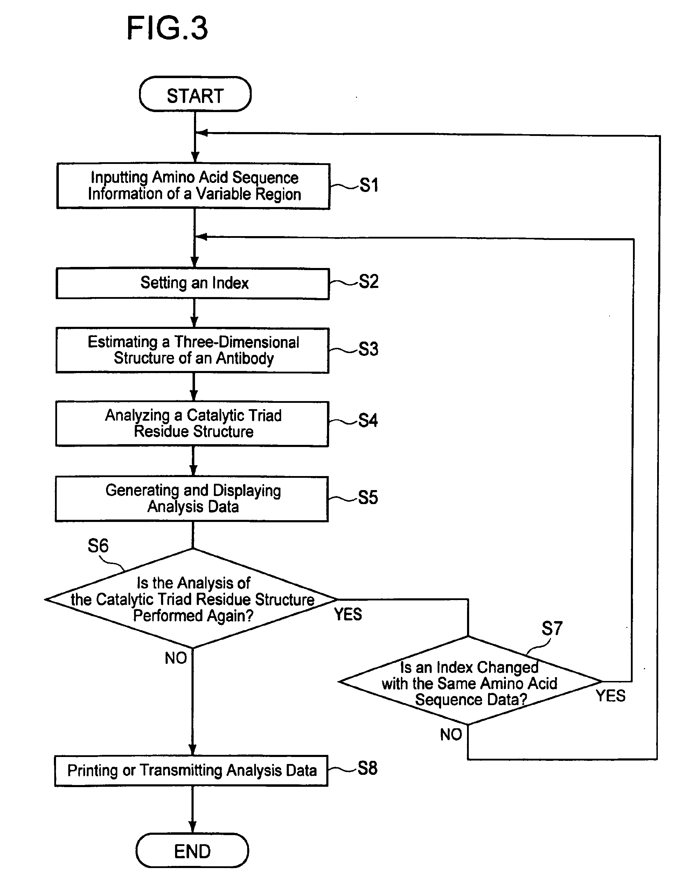

In the next place, a specific operation of the antibody structure analysis system 10, that is, an example of an antibody structure analysis process in the invention will be described with reference to a flowchart shown in Fig.3.

Firstly, as a prior stage, base sequences of a monoclonal antibody obtained in the antibody production step are determined to finally obtain an amino acid sequence. Subsequently, as a step 1 (hereinafter, the step is abbreviated as S), of the amino acid sequence, an amino acid sequence of a variable region is inputted from the input portion 11. Then, as S2, an index for confirming a catalytic triad residue structure in a catalytic triad residue structure confirmation portion 152 is set. As the index, types ① · ①*, ②, ③, ④, and ④* explained in the (2-3) can be cited. The indices may be used singularly or in a combination of a plurality thereof.

In the next place, as S3, by use of inputted data of the amino acid sequence of the variable region, a stereostructure of an antibody is estimated by means of the stereostructure estimation portion 151. Then, as S4, from the data of the stereostructure obtained by the stereostructure estimation portion 151, the catalytic triad residue structure confirmation portion 152, with the data of the amino acid sequence and the index set in the S2, analyses a layout of amino-acid residues that are estimated to constitute the catalytic triad residue structure and a situation of variation thereof.

When a catalytic triad residue structure or a structure that is estimated to be a variation structure thereof is confirmed, the antibody is high in the likelihood of being an antibody enzyme. When any of the above structures is not found, the antibody is low in the likelihood of being an antibody enzyme. Subsequently, in S5, from the analysis data in the S4, final analysis data are generated and displayed at the display portion 12.

Further thereafter, in S6, whether an analysis of the catalytic triad residue structure is once more carried out or not is judged. In the case of the analysis being performed again (YES in the drawing), in S7, whether an analysis is carried out with the data of the same amino acid sequence and with a different index or not is judged. In S7, in the case of data of the same amino acid sequence being used (YES in the drawing), the step is returned to S2, and when data of a new amino acid sequence are inputted (NO in the drawing), the step is returned to S1.

On the other hand, in S6, in the case of the catalytic triad residue structure analysis being not carried out again (NO in the drawing), in S8, obtained analysis data are, when there being a plurality thereof, printed together in the printing portion 13, or sent through a communication interface to other PC 61.

The antibody structure analysis system 10 in the above-described embodiment may be realized on a computer with a program that functions the antibody structure analysis process including above explained steps from S1 to S8.

The program may be stored in a recording medium that can be read with a computer. Specifically, the memory portion 14 shown in Fig.3, specifically, for instance, a ROM itself may be a program medium. When the memory portion 14 is provided with a program reader, it may be a program medium that can be read when a recording medium is inserted therein. As the program medium, known configurations cited as specific examples of the memory portion 14 can be preferably used.

In all cases, the stored program may be carried out when the controller 15 accesses thereto, or the program is read, the read program is downloaded in a not shown program memory area and the program may be carried out. A downloading program is stored in the memory portion 14 beforehand. Furthermore, a content stored in the recording medium, without restricting to the program, may be other data for instance.

Thus, in the invention, a computer program that carries out the antibody structure analysis process on a computer and a machine-readable recording medium in which a computer program that makes a computer execute the program is recorded are included. Accordingly, in order to execute the antibody structure analysis process in the invention on a computer according to a program, the computer itself can be made the antibody structure analysis system 10. As a result, the versatility of the invention can be heightened and the invention can be readily used on a communication network.

### (4) Example of antibody enzyme according to the invention

In the next place, the antibody enzyme involving the invention includes an antibody enzyme having a stereostructurally unique structure that can be applied in the production process, and furthermore, includes also an antibody enzyme that can be produced according to the abovementioned production method. In the embodiment, in what follows, an example of an antibody enzyme involving the invention will be detailed.

### (4-1) Antibody enzyme involving the embodiment

An antibody enzyme involving the embodiment is (a) an antibody enzyme having a variable region made of an amino-acid sequence described in sequence No. 1, 3, 5 or 7 or (b) an antibody enzyme having a variable region made of an amino-acid sequence in which in an amino-acid sequence described in sequence No. 1, 3, 5 or 7, at least one amino acid is replaced, deleted, inserted and/or added and the activity of cleaving and/or decomposing a polypeptide or an antigen protein. In the embodiment, ① a heavy chain of a i41SL1-2 antibody (i41SL1-2-H) that has a variable region having an amino acid sequence shown in the sequence No. 1 and made of 117 amino-acid residues, ② a light chain of a i41SL1-2 antibody (i41SL1-2-L) that has a variable region having an amino acid sequence shown in the sequence No. 3 and made of 114 amino-acid residues, ③ a heavy chain of a i41-7 antibody (i41-7-H) that has a variable region having an amino-acid sequence shown in the sequence No. 5 and made of 120 amino-acid residues, and ④ a light chain of a i41-7 antibody (i41-7-L) that has a variable region having an amino acid sequence shown in the sequence No. 7 and made of 109 amino acid residues can be exemplified.

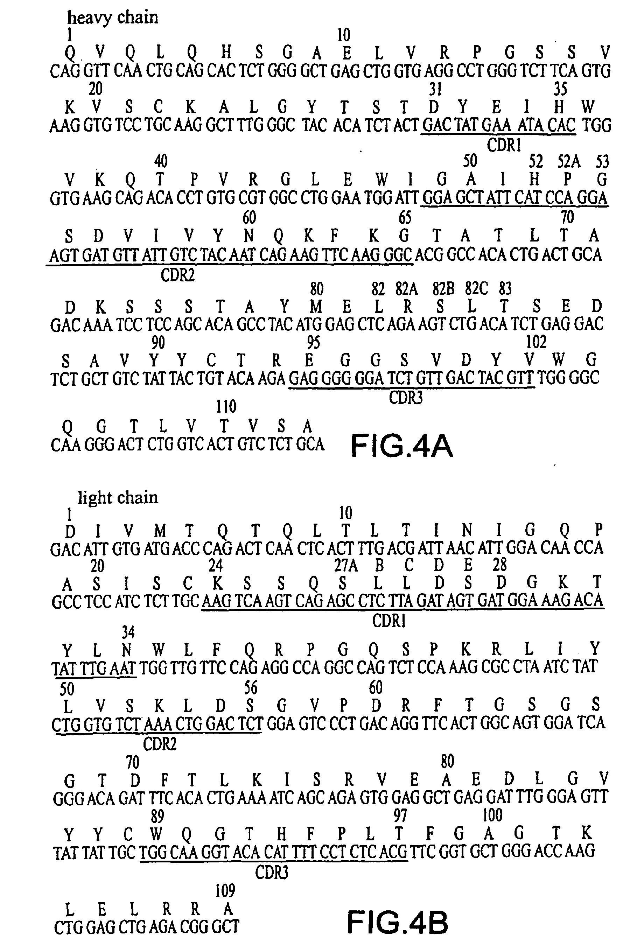

The i41SL1-2 antibody is a monoclonal antibody against a synthetic peptide (shown in the sequence No. 9) of a complimentarity determining region 1 (CDR 1) of a light chain of an antibody 41S-2 that recognizes a constant region of envelope protein gp-41 of HIV virus. Amino acid sequences of variable regions of the heavy and light chains of the i41SL1-2 antibody and base sequences that code the amino acid sequences are shown in Figs. 4A and 4B.

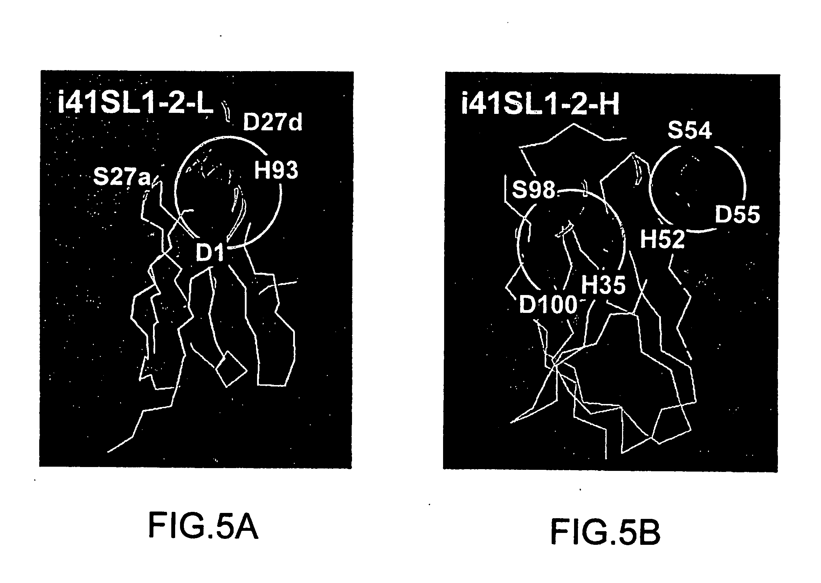

A stereo structure of each of the light chain (i41SL1-2-L) and heavy chain (i41SL1-2-H) of the i41SL1-2 antibody is estimated and analyzed with a computer. As a result, as shown in Figs. 5A and 5B, it was revealed that the i41SL1-2 antibody has a catalytic triad residue structure in both i41SL1-2-H and i41SL1-2-L. That is, the i41SL1-2-L, as shown in Figs. 4B and 5A, is estimated to have a catalytic triad residue structure constituted of, according to Kabat numbering scheme, an aspartate residue at the 1^{st} (D1), a histidine residue at the 93^{rd} (H93), and a serine residue at the 27A (S27a) or a catalytic triad residue structure constituted of, according to Kabat numbering scheme, an aspartate residue at the 27D (D27d), a histidine residue at the 93^{rd} (H93), and a serine residue at the 27A (S27a).

Furthermore, the i41SL1-2-H, as shown in Figs. 4A and 5B, is estimated to have a catalytic triad residue structure constituted of, according to Kabat numbering scheme, an aspartate residue at the 55^{th} (D55), a histidine residue at the 52^{nd} (H52), and a serine residue at the 54^{th} (S54) or a catalytic triad residue structure constituted of, according to Kabat numbering scheme, an aspartate residue at the 100^{th} (D100), a histidine residue at the 35^{th} (H35), and a serine residue at the 98^{th} (S98). Locations of the catalytic triad residue structures are in the neighborhood of the complimentarity determining regions for both the light and heavy chains.

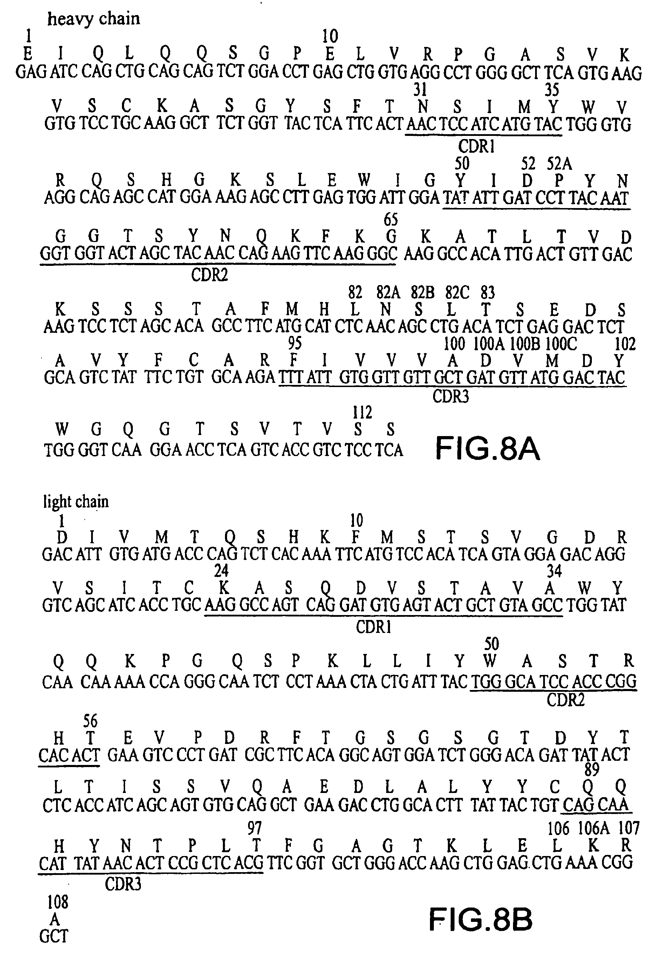

The i41-7 antibody is a monoclonal antibody against a light chain of antibody 41S-2. Amino acid sequences of variable regions of the heavy and light chains of the i41-7 antibody and base sequences that code the amino acid sequences are shown in Figs. 8A and 8B.

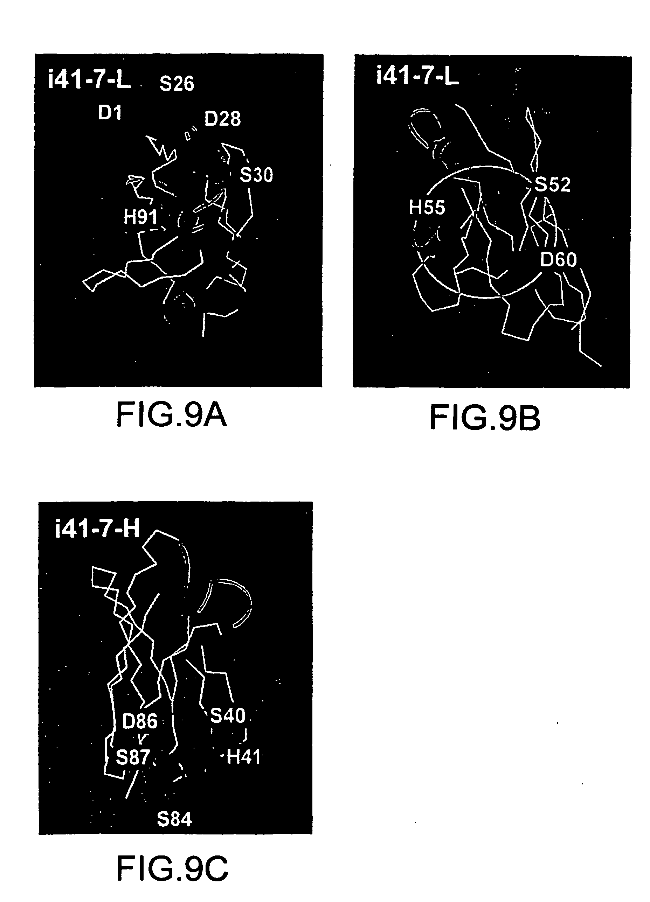

A stereo structure of each of the light chain (i41-7-L) and heavy chain (i41-7-H) of the i41-7 antibody is, similarly to the i41SL1-2 antibody, estimated and analyzed with a computer. As a result, as shown in Figs. 9A, 9B and 9C, it was revealed that the i41-7 antibody has a catalytic triad residue structure in each of the i41-7-L and i41-7-H. That is, the i41-7-L, as shown in Figs. 8B and 9A, is estimated to have a catalytic triad residue structure constituted of, according to Kabat numbering scheme, an aspartate residue at the 1^{st} (D1), a histidine residue at the 91^{st} (H91), and a serine residue at the 26^{th} (S26) or a catalytic triad residue structure constituted of, according to Kabat numbering scheme, an aspartate residue at the 28^{th} (D28), a histidine residue at the 91^{st} (H91), and a serine residue at the 30^{th} (or 26^{th}) (S30 or S26). Locations of the catalytic triad residue structures are in the neighborhood of a CDR1 and CDR3 that are the complimentarity determining region (CDR).

Furthermore, the i41-7-L, as shown in Figs. 8B and 9B, is estimated to have a catalytic triad residue structure constituted of, according to Kabat numbering scheme, an aspartate residue at the 60^{th} (D60), a histidine residue at the 55^{th} (H55), and a serine residue at the 52^{nd} (S52). This is located in the neighborhood of a CDR2.

Still furthermore, the i41-7-H, as shown in Figs. 8A and 9C, is estimated to have a catalytic triad residue structure constituted of, according to Kabat numbering scheme, an aspartate residue at the 86^{th} (D86), a histidine residue at the 41^{st} (H41), and a serine residue at the 40^{th} (or 84^{th} or 87^{th}) (S40 or S84, or S87).

### (4-2) Gene involving the embodiment

A gene involving the embodiment may be (a) a gene that codes an antibody enzyme having a variable region made of an amino-acid sequence shown in sequence No. 1, 3, 5 or 7, or (b) a gene that codes an antibody enzyme that has a variable region made of an amino-acid sequence that is obtained by replacing, deleting, inserting and/or adding at least one amino acid in an amino acid sequence shown in sequence No. 1, 3, 5 or 7 and has the activity of cleaving and/or decomposing a polypeptide and an antigen protein. In the embodiment, ① a gene (i41SL1-2-H gene) having a variable region made of a base sequence (cDNA) shown in sequence No.2, ② a gene (i41SL1-2-L gene) having a variable region made of a base sequence (cDNA) shown in sequence No.4, ③ a gene (i41-7-H gene) having a variable region made of a base sequence (cDNA) shown in sequence No.6, and ④ a gene (i41-7-L gene) having a variable region made of a base sequence (cDNA) shown in sequence No.8 can be exemplified. The genes are derived from a mouse (Mus musculus).

The "gene" according to the invention includes an RNA and a DNA. The RNA includes an mRNA, and the DNA includes a cDNA or a genome DNA that can be obtained by, for instance, cloning or chemical synthesis technology or a combination thereof. Furthermore, the DNA may be a double-stranded one or a single-stranded one. The single-stranded DNA may be a code DNA that becomes a sense strand or an antisense code that becomes an antisense strand (antisense strand can be used as a probe or an antisense drug). Furthermore, the "gene" according to the invention includes, other than sequences that code proteins of the (a) or (b), sequences such as a sequence of an untranslated region (UTR) or a sequence of a vector sequence (including an expression vector sequence).

### (4-3) Function of antibody enzymes i41SL1-2 and i41-7

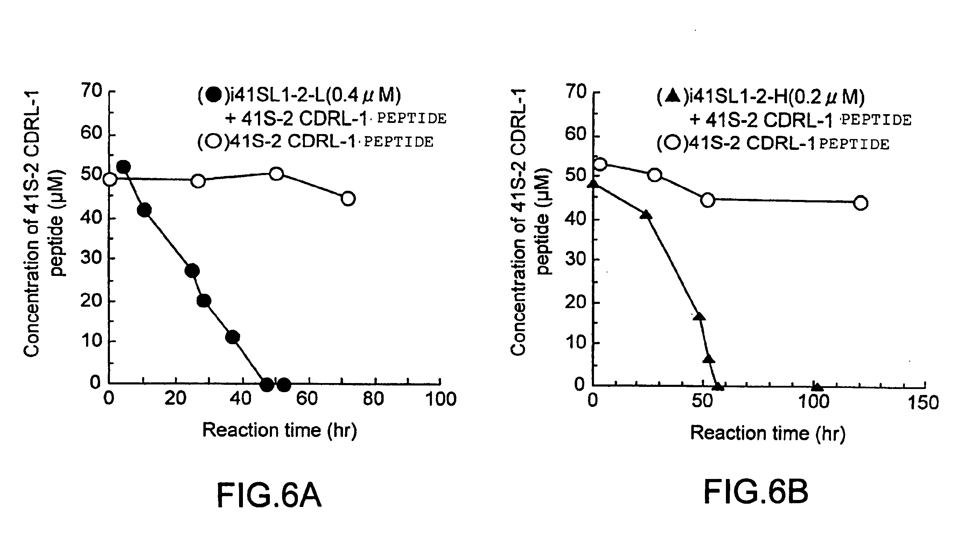

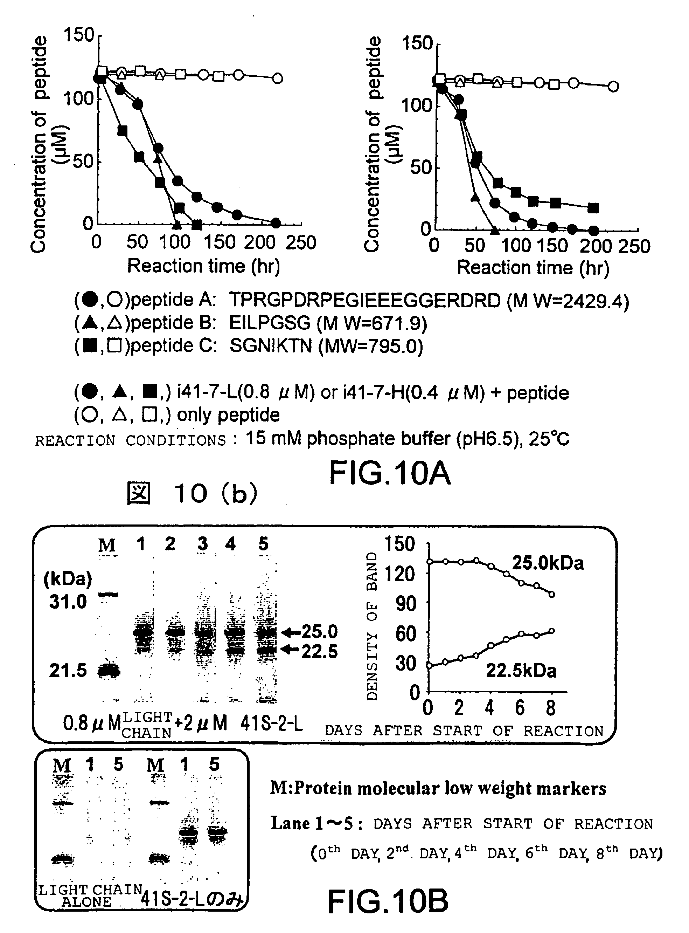

In the i41SL1-2 and i41-7 antibodies, as shown in an example described later, it was experimentally confirmed that both heavy chains (i41SL1-2-H and i41-7-H) and light chains (i41SL1-2-L and i41-7-L) have the activity of cleaving and/or decomposing a polypeptide or an antigen protein (Figs. 6A and 6B and Figs. 10A and 10B). In addition, though results are not shown, when as a control protein HAS was reacted with the i41SL1-2-H, i41-7-H, i41SL1-2-L and i41-7-L, the HAS was not decomposed. From this, it was shown that, in the i41SL1-2 antibody and i41-7 antibody, both heavy chains and light chains have high substrate specificity.

Furthermore, in particular, as shown in Figs. 10A and 10B, it was found that both the heavy and light chains of the i41-7 antibody have the peptidase activity, and, when a peptide is short, irrespective of the specificity, though different in the reaction rate, cleave and/or decompose the peptide. Still furthermore, shorter peptides were larger in the reaction rate than longer peptides. This is considered that, irrespective of the specificity, the shorter peptide is also small in the molecular weight and can readily access a bonding site of the i41-7 antibody.

In Fig. 10B, the i41-7-L decomposes 41S-2-L (25kDa) that is an antigen protein and a band of 22.5kDa appears. Furthermore, the i41-7-L did not decompose an irrelevant protein such as BSA; accordingly, it can be said it specifically decompose the antigen protein.

That is, it is considered that the i41SL1-2-H, i41-7-H, i41SL1-2-L and i41-7-L, when a substrate is a protein, specifically cleave and/or decompose; when the substrate is a polypeptide (polypeptides more than substantially 30 mer or more), to a certain extent specifically, cleave and/or decompose; and, when the substrate is a more shorter polypeptide (substantially 30 mer or less), rather non-specifically cleave and/or decompose.

Accordingly, it was made clear that the i41SL1-2-H, i41-7-H, i41SL1-2-L and i41-7-L are antibody enzymes that have excellent substrate recognition ability of antibody and substrate transforming capacity of enzyme in combination.

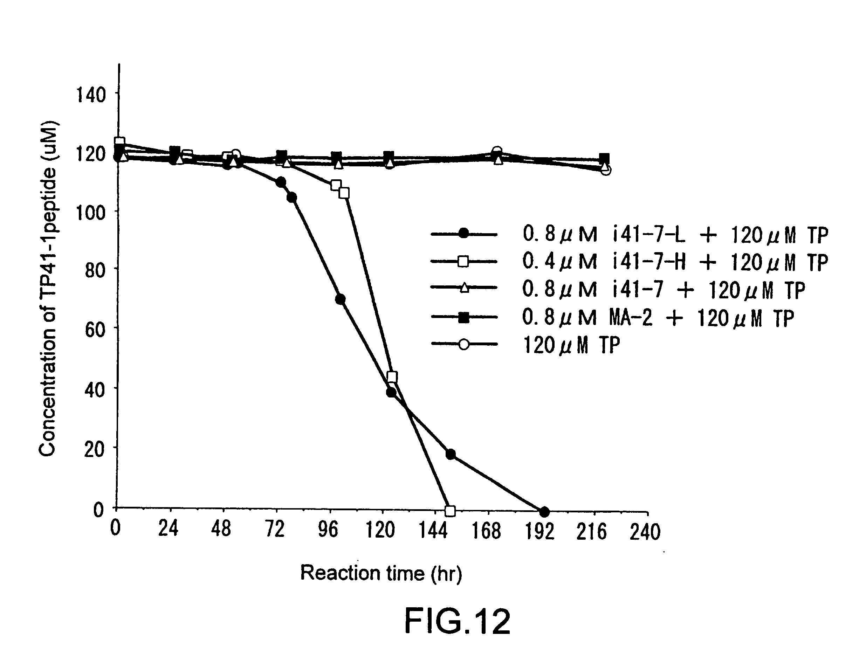

An antibody that does not have a catalytic triad residue structure in a stereostructure, MA-2 (monoclonal antibody against methamphetamine) does not at all cause the antigen decomposition reaction (Fig. 12). Furthermore, as described later, heavy chains of HpU-9 and HpU-18 antibodies do not have His; accordingly, these cannot have a catalytic triad residue structure. Both heavy chains did not at all exhibit the peptidase activity.

Accordingly, it was revealed that an antibody enzyme that has a catalytic triad residue structure in a stereostructure has the activity of cleaving and/or decomposing a polypeptide and an antigen protein.

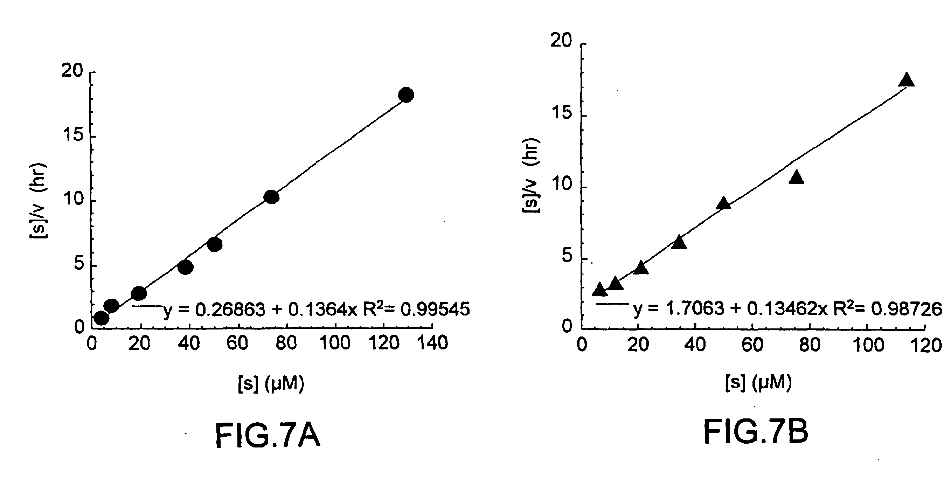

Furthermore, as will be shown in an example described later, the decomposition reactions of antigen peptides owing to the light and heavy chains of the i41SL1-2 antibody are kinetically analyzed (Figs. 7A and 7B and Table 5). As a result, in the case of i41SL1-2 antibody, the light chain exhibited such a high catalyst efficiency (kcat/Km) same as that of tripsin and the heavy chain exhibited a value substantially one tenth that of tripsin. Accordingly, in the i41SL1-2, both the light and heavy chains can be said have the high enzymatic activity comparable to that of a natural protease. Furthermore, it was revealed that the i41SL1-2-L and i41SL1-2-H, with the nature as an antibody that has high affinity to a substrate remaining, exhibit the enzymatic activity of decomposing a polypeptide and an antigen protein.

Accordingly, the antibody enzyme involving the invention was experimentally demonstrated that it has the activity identical as that of a natural protease and high affinity and specificity to a substrate unique to an antibody.

### (4-4) Production process of antibody enzymes involving the embodiment

The antibody enzymes involving the embodiment can be produced by use of the abovementioned antibody enzyme production process involving the invention. In particular, in the embodiment, according to the stereostructure analysis due to an analysis result of the germlines and the molecular modeling, antibodies having the enzymatic activity are produced.

Specifically, among mouse germlines, in light chains derived from bd2, 19-25 and hf24 by Thiebe et al. classification, the enzymatic activity was confirmed (In particular, bd2 and 19-25 constitute a catalytic triad residue structure from amino-acid residues derived from germlines. In hf24, a histidine residue is generated by variation). In addition, antibody light chains derived from cr1 (HpU18) and cs1 (HpU9) (both are assumed to form a catalytic triad residue structure from amino-acid residues derived from germlines) are also recognized to have the decomposing activity to peptides. HpU-2-H (J558.h) lacks a histidine residue, forms a catalytic triad residue structure from Asp86, Glu85 and Ser84 (orSer87) and exhibits the activity of decomposing polypeptides and antigen proteins. This is a very important finding that suggests that there are many antibodies having the enzymatic activity in nature and that the germline can be an index when this kind of antibody is searched. A detail thereof will be described in an embodiment described below.

### (4-5) Antibody enzyme involving the invention and application of its gene

Antibody enzymes and genes involving the invention are novel antibody enzymes that can be produced based on a so far unknown knowledge that a catalytic triad residue structure is present in a stereostructure. Accordingly, these have the availability below.

When the invention is applied, for instance, from an amino acid sequence of an antibody prepared according to a so far known immunological process (for instance, an amino-acid sequence estimated from a base sequence that is determined by use of a so far known genetic engineering process), a stereostructure of the antibody is estimated by use of a computer, whether a structure that can be a catalytic triad residue structure is present in the stereostructure thereof or not is investigated to estimate an antibody enzyme that has the activity of cleaving and/or decomposing a polypeptide and an antigen protein, and thereby the antibody enzymes are enabled to efficiently obtain.

Furthermore, when the invention is applied, for instance, by use of a so far known genetic engineering process, a structure that can be a catalytic triad residue structure is introduced into a stereostructure, and also thereby an antibody enzyme having the activity of cleaving and/or decomposing a polypeptide and an antigen protein can be artificially prepared.

The so far known genetic engineering procedure is neither particularly restricted. For instance, by use of a known variant protein preparation process such as the site-directed mutagenesis (reference literature 8: Hashimoto-Gotoh, Gene 152, 271-275 (1995)), a method in which by use of a PCR method and so on a variation is introduced in a base sequence, or a mutant preparation method due to insertion of transposon, in a base sequence of a gene obtained as mentioned above, a modification is applied so as to replace, delete, insert and/or add at least one base, and thereby a modified base sequence can be prepared. When a variant protein is produced, a commercially available kit can be used.

The antibody enzyme obtained, prepared according to a process as mentioned above has highly specific substrate recognizing capability of the antibody and the enzymatic activity. Accordingly, it is considered that to infectious diseases caused, for instance, by intrusion of bacteria or viruses into a living body, when antibody enzymes specific to causative bacteria or viruses are obtained or prepared, the antibody enzymes can be used to diagnose or cure. Furthermore, when, for instance, to cancer as well, antibody enzymes specifically recognizing peptides that are specifically expressed in cancer cells are obtained or prepared, the cancer can be diagnosed and cured by use of the antibody enzymes.

Furthermore, without restricting only to applications to the abovementioned medical products, toward so far unknown novel drugs, in future, there is likelihood of leading to a development of epoch-making drugs that can conquer incurable diseases and the drug resistance.

In immunological diagnosis, at present, RIA (Radioimmunoassay) and ELISA (Enzyme-Linked Immunosorbent Assay) are frequently used. However, by obtaining a target antibody enzyme and by utilizing the antibody enzyme, a novel clinical diagnosis may be developed.

Furthermore, the antibody enzyme that has, in combination, high molecule recognition capability of the antibody and the substrate variation capability that the enzyme has can be applied also to a novel biosensor as a new biomaterial and used in the diagnosis of disease and inspections such as environmental measurement.

Still furthermore, it is considered that the antibody enzyme involving the invention, having the activity substantially same as that of a natural enzyme, by making use of the catalytic activity (enzymatic activity) thereof, can be developed as well in a reaction promoter such as catalysts used in food industries and chemical industries.

The invention includes a gene that codes the abovementioned antibody enzyme or a variant of the gene. The gene involving the invention or the variant of the gene can be introduced in various kinds of host cells to express an antibody enzyme involving the invention or a variant protein thereof. The gene that is introduced and involves the invention or the variant of the gene may be present as a vector in the host cell or contained in a genome DNA of the host cell as an "external" DNA or "additional" DNA. The "external" DNA here means a DNA that is not present naturally in a genome of the host cell but is inserted in the genome of the host cell according to an artificial operation. The "additional" DNA means a DNA that is present naturally in a genome of a particular host cell but is further additionally inserted in the genome of the host cell according to an artificial operation.

Specific kind of the vector, without being restricted to particular one, may be appropriately selected as one that can express in the host cell. That is, in accordance with the kind of the host cell, in order to assuredly express the gene, a promoter sequence is properly selected, this and the gene involving the invention are incorporated in various kinds of plasmids and so on, and resultant ones may be used as an expression vector. Furthermore, in the expression vector, not only a promoter sequence but also a terminator sequence may be included.

Furthermore, in order to confirm whether the gene or the variant of gene is introduced in the host cell or not, and further whether the gene or the variant of gene is assuredly expressed in the host cell or not, various kinds of markers may be used. For instance, by use of a gene that is deleted in the host cell as a marker, a plasmid or the like containing the marker is introduced in the host cell together with the expression vector that contains the gene involving the invention. Thereby, from the expression of the marker gene, the introduction of the gene involving the invention can be confirmed.

The host cell is not restricted to particular one, and so far known various kinds of cells can be preferably used. Specifically, for instance, bacteria such as Escherichia coli, yeasts (such as budding yeast, Saccharomyces cerevisiae or fission yeast, Schizosaccharomyces pombe), oocytes of Xenopus laevis, cultured cells of various kinds of mammals, or cultured cell of insects can be cited; however, there is no particular restriction.

A method of introducing the expression vector in a host cell, that is, a transformation process is neither particularly restricted; that is, so far known methods such as the electroporation method, the calcium phosphate method, the liposome method, and the DEAE dextran method can be preferably used.

As mentioned above, the antibody enzymes involving the invention and the genes thereof can be applied to not only diagnosis and remedy of various kinds of infectious diseases and cancers but also various kinds of research reagents.

When the genes and antibody enzymes involving the invention are rendered medicines, so far known processes can be applied; that is, there is no particular restriction. For instance, in the case of research reagents, the genes involving the invention may be rendered a transformation kit or the antibody enzymes involving the invention may be mass-produced and purified in a hetero-expression system.

### [Embodiment 2]

In the embodiment 2, as a more specific example of an antibody enzyme involving the invention, an antibody enzyme against Helicobacter Pylori urease will be explained. The present invention is not restricted to the descriptions below.

### (1-1) About antibody enzyme involving the embodiment and gene

About an antibody enzyme involving the invention, antibody fragments of monoclonal antibodies HpU-18, HpU-9 and HpU-2 of the HP urease will be exemplified and described. The antibody fragments of HpU-18, HpU-9 and HpU-2 are specifically a variable region of an L chain of HpU-18 antibody, a variable region of an L chain of HpU-9 antibody, and a variable region of an H chain of HpU-2 antibody, respectively. These three antibody enzymes are obtained according to the abovementioned antibody enzyme production process. That is, from a plurality of monoclonal antibodies of the HP urease, by applying the molecular modeling to amino acid sequences of the variable regions thereof to estimate stereostructures thereof, these are found out as amino acid sequences (antibody fragment) that can constitute a catalytic triad residue structure made of a serine residue, a histidine (or glutamate) residue and an aspartate residue.

A variable region of an L chain of the HpU-18 antibody (hereinafter, referred to as HpU-18-L), a variable region of an L chain of the HpU-9 antibody (hereinafter, referred to as HpU-9-L), and a variable region of an H chain of the HpU-2 antibody (hereinafter, referred to as HpU-2-H) work as the degrading enzyme of the HP urease. This is, as will be shown in an example described later, obvious from a result that these variable regions can completely decompose a peptide that corresponds to a portion of region of the HP urease. The peptide decomposed is one of important regions when the HP urease exhibits the enzymatic activity. Accordingly, when the region is decomposed and thereby a function of the HP urease is completely destroyed, the HP bacteria are rendered incapable of living in a strongly acidic stomach. That is, the antibody enzyme according to the invention that can decompose the peptides can efficiently eliminate the HP bacteria.

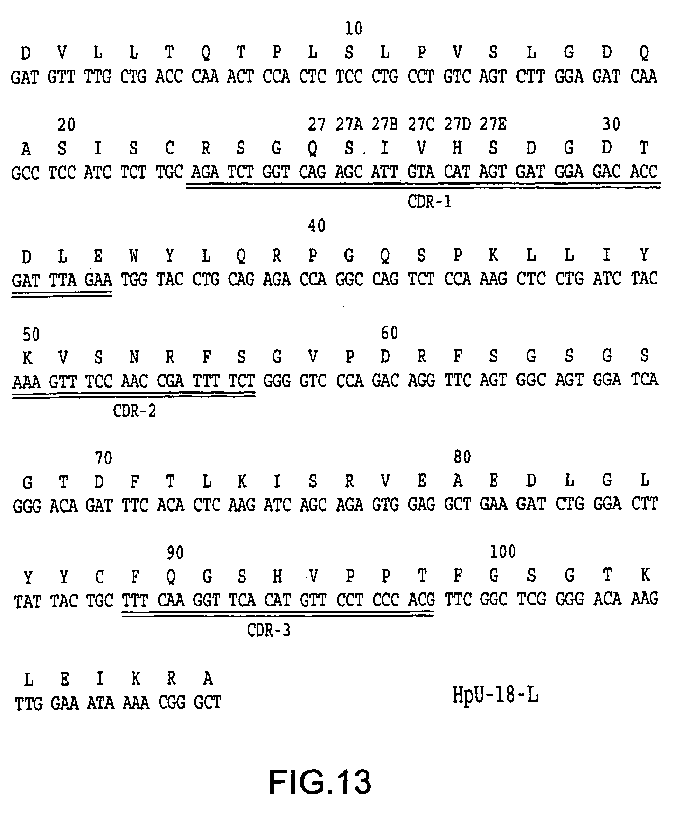

Subsequently, a structure of the HpU-18-L will be detailed below. The HpU-18-L is, as mentioned above, a variable region of an L chain of HpU-18 that is one of monoclonal antibodies of the HP urease and has an amino acid sequence shown in sequence No.14 as a primary structure. In Fig.13, an amino acid sequence of the HpU-18-L and therebelow an example of a base sequence of a gene that codes the amino acid sequence are shown. The base sequence is a base sequence of a gene of the HpU-18 antibody L chain cloned in the embodiment. In Fig.13, complimentarity determining sites that form a complimentary stereostructure with an antigen molecule (HP urease) and determine the complimentarity of the antibody are shown with double underlines as CDR-1, CDR-2 and CDR-3.

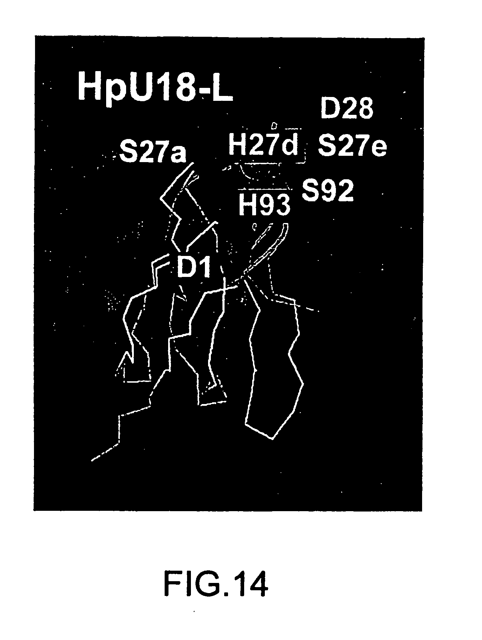

In Fig.14, after the molecular modeling is applied to amino acid sequence of the HpU-18-L, an estimated stereostructure is schematically shown. As shown in Fig.14, in an amino acid sequence shown in sequence No.14, an aspartate residue at the 1^{st} (in Fig. 14, shown as D1 by Kabat numbering scheme), a histidine residue at the 98^{th} (in Fig.14, shown as H93 by Kabat numbering scheme), and a serine residue at the 97^{th} (in Fig.14, shown as S92 by Kabat numbering scheme) or a serine residue at the 28^{th} (in Fig.14, shown as S27a by Kabat numbering scheme) are estimated to form a catalytic triad residue structure. Alternatively, in an amino acid sequence shown in sequence No.14, an aspartate residue at the 33^{rd} (in Fig.14, shown as D28 by Kabat numbering scheme), a histidine residue at the 31^{st} (in Fig.14, shown as H27d by Kabat numbering scheme), and a serine residue at the 32^{nd} (in Fig.14, shown as S27e by Kabat numbering scheme) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No.14, that is, a variant of the HpU-18-L and works as a degrading enzyme of the HP urease. Still furthermore, the antibody enzyme may be one that is obtained by arbitrarily adding a remaining amino acid sequence of the HpU-18 antibody L chain to a C terminal side of the amino acid sequence shown in sequence No.14 or an entire length of the HpU-18 antibody L chain.

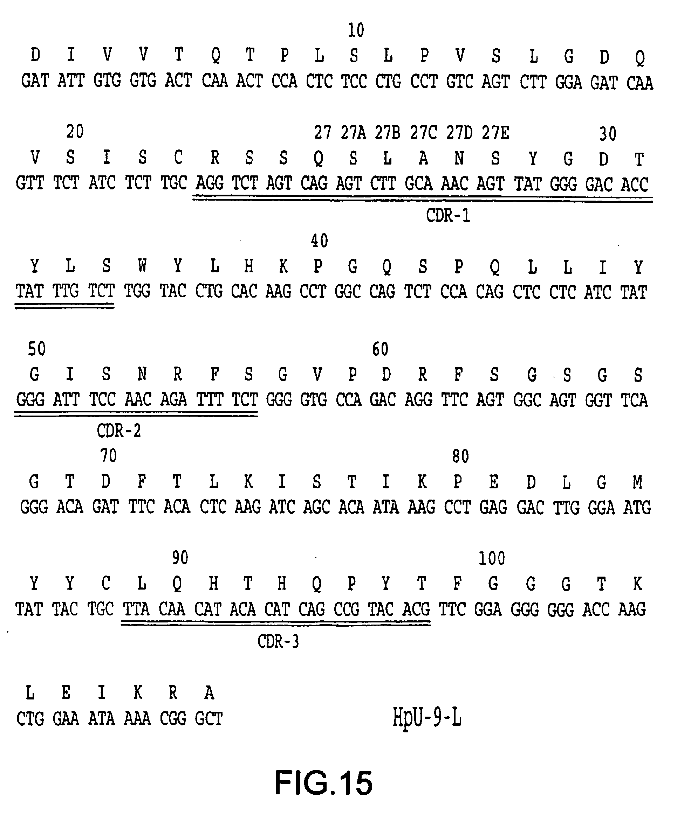

In the next place, a structure of the HpU-9-L will be detailed below. The HpU-9-L is, as mentioned above, a variable region of an L chain of HpU-9 that is one of monoclonal antibodies of the HP urease and has an amino acid sequence shown in sequence No.15 as a primary structure. In Fig.15, an amino acid sequence of the HpU-9-L and therebelow an example of a base sequence of a gene that codes the amino acid sequence are shown. The base sequence is a base sequence of a gene of the HpU-9 antibody L chain cloned in the embodiment. In Fig.15, complimentarity determining sites are shown with double underlines as CDR-1, CDR-2 and CDR-3.

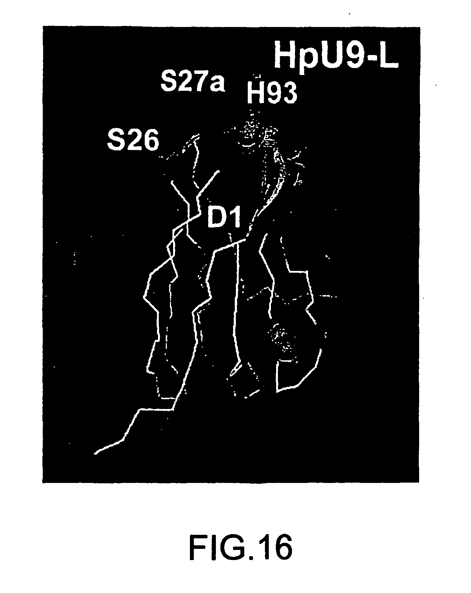

In Fig.16, after the molecular modeling is applied to an amino acid sequence of the HpU-9-L, an estimated stereostructure is schematically shown. As shown in Fig.16, in an amino acid sequence shown in sequence No.15, an aspartate residue at the 1^{st} (in Fig. 16, shown as D1 by Kabat numbering scheme), a histidine residue at the 98^{th} (in Fig.16, shown as H93 by Kabat numbering scheme), and a serine residue at the 26^{th} (in Fig.16, shown as S26 by Kabat numbering scheme) or a serine residue at the 28^{th} (in Fig.16, shown as S27a by Kabat numbering scheme) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No.15, that is, a variant of the HpU-9-L, and works as a degrading enzyme of the HP urease. Still furthermore, the antibody enzyme may be one that is obtained by arbitrarily adding a remaining amino acid sequence of the HpU-9 antibody L chain to a C terminal side of the amino acid sequence shown in sequence No.15 or an entire length of the HpU-9 antibody L chain.

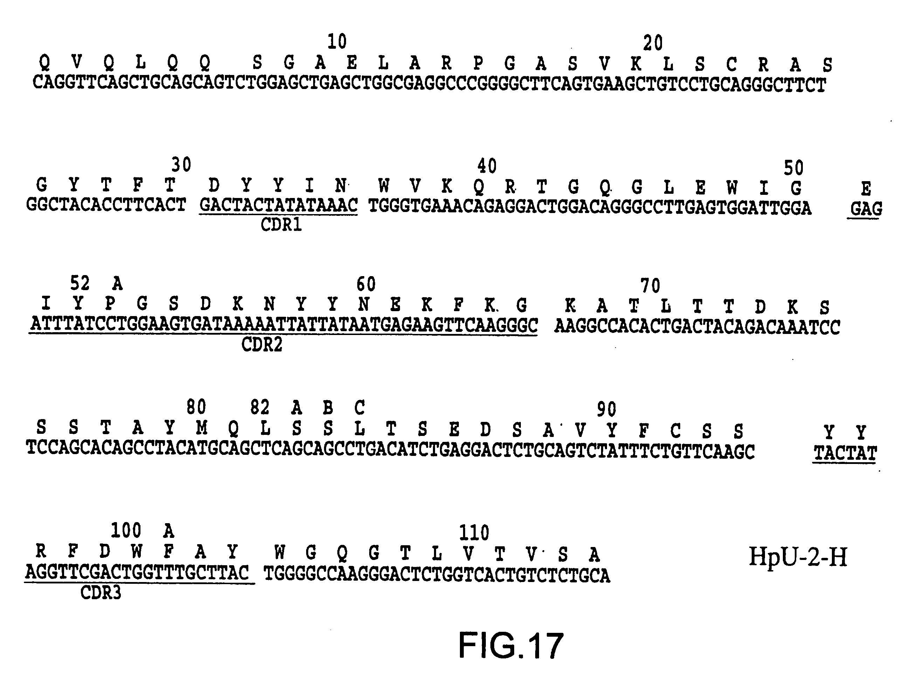

In the next place, a structure of the HpU-2-H will be detailed below. The HpU-2-H is, as mentioned above, a variable region of an H chain of HpU-2 that is one of monoclonal antibodies of the HP urease and has an amino acid sequence shown in sequence No.16 as a primary structure. In Fig.17, an amino acid sequence of the HpU-2-H and therebelow an example of a base sequence of a gene that codes the amino acid sequence are shown. The base sequence is a base sequence of a gene of the HpU-2 antibody H chain cloned in the embodiment. In Fig.17, complimentarity determining sites are shown with double underlines as CDR-1, CDR-2 and CDR-3.

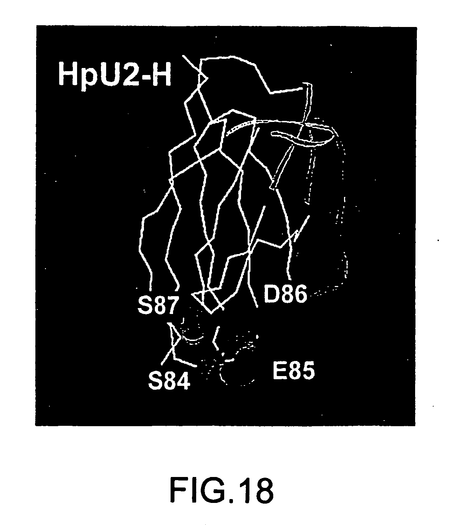

In Fig.18, after the molecular modeling is applied to an amino acid sequence of the HpU-2-H, an estimated stereostructure is schematically shown. As shown in Fig.18, in an amino acid sequence shown in sequence No.16, an aspartate residue at the 90^{th} (in Fig.18, shown as D86 by Kabat numbering scheme), a glutamate residue at the 89^{th} (in Fig.18, shown as E85 by Kabat numbering scheme), and a serine residue at the 88^{th} (in Fig.18, shown as S84 by Kabat numbering scheme) or a serine residue at the 91^{st} (in Fig.18, shown as S87 by Kabat numbering scheme) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No.16, that is, a variant of the HpU-2-H, and works as a degrading enzyme of the HP urease. Still furthermore, the antibody enzyme may be one that is obtained by arbitrarily adding a remaining amino acid sequence of the HpU-2 antibody H chain to a C terminal side of the amino acid sequence shown in sequence No.16 or an entire length of the HpU-2 antibody L chain.

The genes involving the invention are genes that code the abovementioned antibody enzymes, and, for instance, genes made of base sequences shown in sequence Nos.47, 48 and 49 can be cited. However, genes involving the invention, without restricting thereto, may be various genes that code antibody enzymes having amino acid sequence Nos.14, 15 and 16, and furthermore genes that code variants thereof.

### (1-2) Another example of antibody enzyme according to the embodiment

Subsequently, as another example of the antibody enzyme according to the invention, an antibody fragment of monoclonal antibody HpU-20 of the HP urease will be exemplified and described. The antibody fragment of HpU-20 is specifically a variable region of an L chain of HpU-20 antibody and a variable region of an H chain of HpU-20 antibody. These two antibody enzymes as well, similarly to the HpU-18-L and so on, are obtained from a plurality of monoclonal antibodies of the HP urease as amino acid sequences (antibody fragments) that can constitute a catalytic triad residue structure made of a serine residue, a histidine residue and an aspartate residue when the molecular modeling is applied to amino acid sequences of variable regions of the monoclonal antibodies to estimate stereostructures thereof.

A variable region of an L chain of the HpU-20 antibody (hereinafter, referred to as HpU-20-L) and a variable region of an H chain of the HpU-20 (hereinafter, referred to as HpU-20-H) work as a degrading enzyme of the HP urease. This is, as will be shown in an example described later, obvious also from a result that these variable regions decompose a β-subunit of the HP urease. The peptide decomposed is one of important regions when the HP urease exhibits the enzymatic activity. Accordingly, by decomposing the region to completely destroy a function of the HP urease, resultantly the HP bacteria are rendered incapable of living in a strongly acidic stomach. That is, the antibody enzyme according to the invention that can decompose the peptides can efficiently eliminate the HP bacteria.

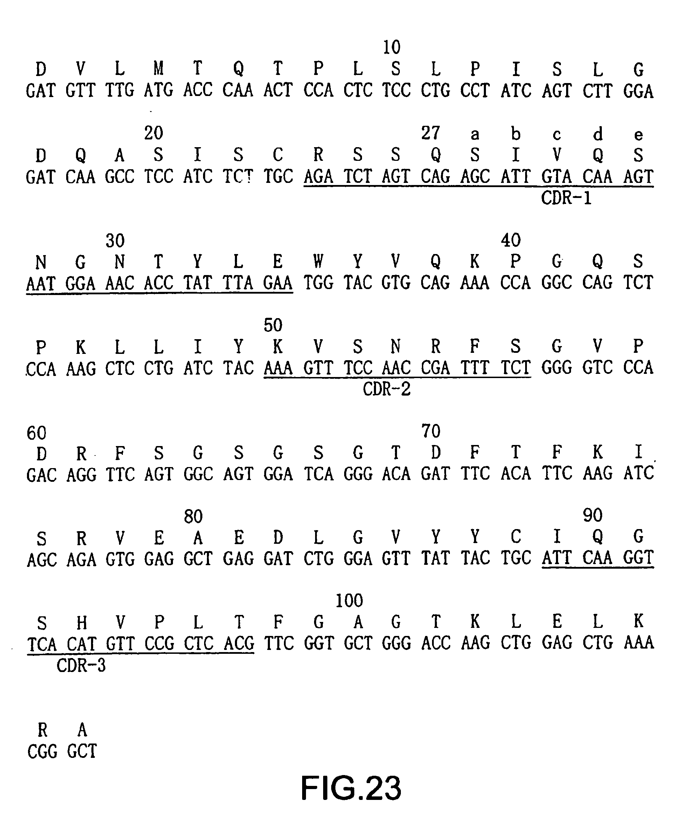

Subsequently, a structure of the HpU-20-L will be detailed below. The HpU-20-L is, as mentioned above, a variable region of an L chain of HpU-20 that is one of monoclonal antibodies of the HP urease and has an amino acid sequence shown in sequence No.17 as a primary structure. In Fig.23, an amino acid sequence of the HpU-20-L and therebelow an example of a base sequence of a gene that codes the amino acid sequence are shown. The base sequence is a base sequence of a gene of the HpU-20 antibody L chain cloned in the embodiment. In Fig.23, complimentarity determining sites that form a complimentary stereostructure with an antigen molecule (HP urease) and determine the complimentarity of the antibody are shown with double underlines as CDR-1, CDR-2 and CDR-3.

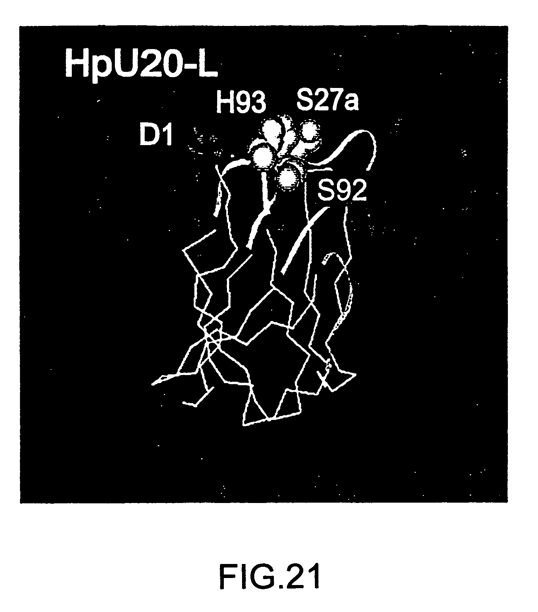

In Fig.21, a stereostructure estimated after the molecular modeling is applied to amino acid sequences of the HpU-20-L is schematically shown. As shown in Fig.21, in an amino acid sequence shown in sequence No.17, an aspartate residue at the 1^{st} (in Fig.21, shown as D1 by Kabat numbering scheme), a histidine residue at the 98^{th} (in Fig.21, shown as H93 by Kabat numbering scheme), and a serine residue at the 97^{th} (in Fig.21, shown as S92 by Kabat numbering scheme) or a serine residue at the 28^{th} (in Fig.21, shown as S27a by Kabat numbering scheme) are estimated to form a catalytic triad residue structure. The germline of the HpU-20-L is cr1.

Furthermore, the antibody enzyme according to the invention may be one that is made of an amino acid sequence that is obtained by replacing, deleting, inserting and/or adding at least one amino acid in an amino acid sequence shown in sequence No.17, that is, a variant of the HpU-20-L, and works as a degrading enzyme of the HP urease. Still furthermore, the antibody enzyme may be one that is obtained by arbitrarily adding a remaining amino acid sequence of the HpU-20 antibody L chain to a C terminal side of the amino acid sequence shown in sequence No.17 or an entire length of the HpU-20 antibody L chain.

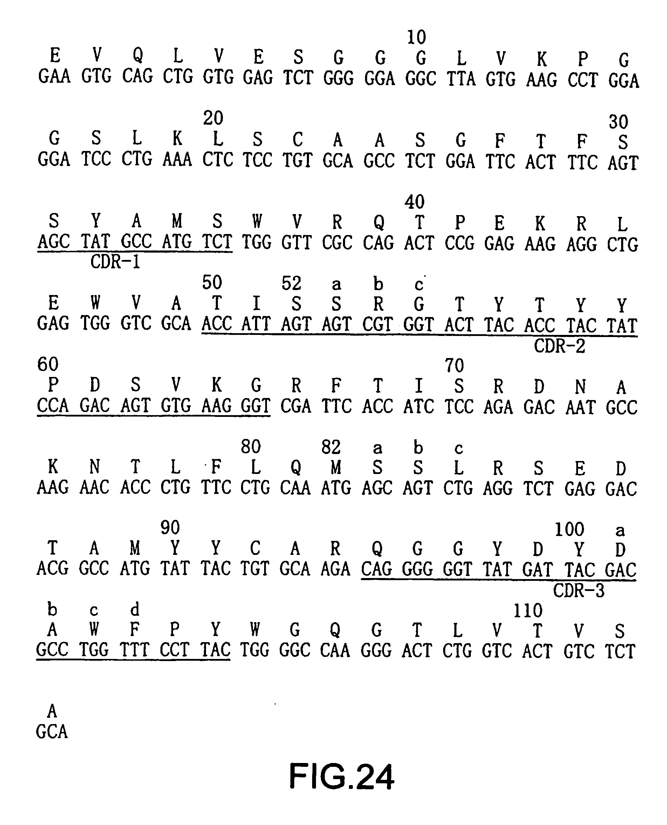

In the next place, a structure of the HpU-20-H will be detailed below. The HpU-20-H is, as mentioned above, a variable region of an H chain of HpU-20 that is one of monoclonal antibodies of the HP urease and has an amino acid sequence shown in sequence No.19 as a primary structure. In Fig.24, an amino acid sequence of the HpU-20-H and therebelow an example of a base sequence of a gene that codes the amino acid sequence are shown. The base sequence is a base sequence of a gene of the HpU-20 antibody H chain cloned in the embodiment. In Fig.24, complimentarity determining sites are shown with double underlines as CDR-1, CDR-2 and CDR-3.

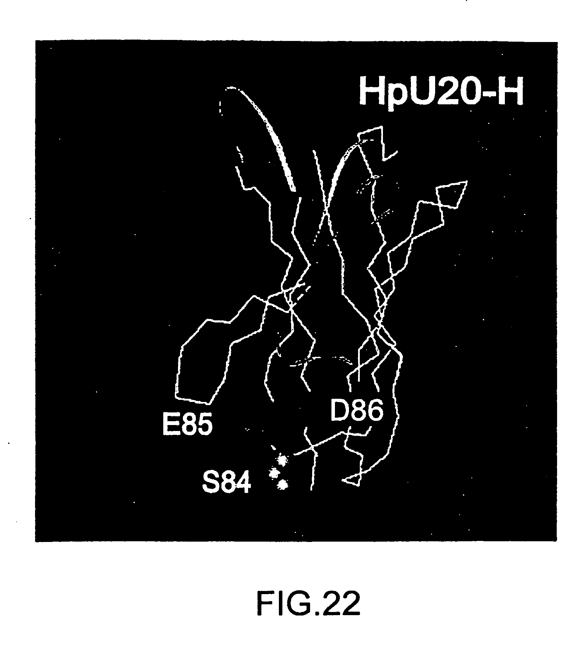

In Fig. 22, a stereostructure estimated by applying the molecular modeling to an amino acid sequence of the HpU-20-H is schematically shown. As shown in Fig. 22, in an amino acid sequence shown in sequence No.19, an aspartate residue at the 90^{th} (in Fig. 22, shown as D86 by Kabat numbering scheme), a glutamate residue at the 89^{th} (in Fig.22, shown as E85 by Kabat numbering scheme), and a serine residue at the 88^{th} (in Fig.22, shown as S84 by Kabat numbering scheme) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No.19, that is, a variant of the HpU-20-H, and works as a degrading enzyme of the HP urease. Still furthermore, the antibody enzyme may be one that is obtained by arbitrarily adding a remaining amino acid sequence of the HpU-20 antibody H chain to a C terminal side of the amino acid sequence shown in sequence No.19 or an entire length of the HpU-20 antibody L chain.

The genes involving the invention are genes that code the antibody enzymes, and, for instance, genes made of base sequences shown in sequence Nos.18 and 20 can be cited. A gene made of a bas sequence shown in sequence No.18 is one of base sequences of a gene (cDNA) that codes a variable region of an L chain of the HpU-20, and a gene made of a base sequence shown in sequence No.20 is one of base sequences of a gene (cDNA) that codes a variable region of an H chain of the HpU-20. However, genes involving the invention, without restricting thereto, may be various genes that code antibody enzymes having amino acid sequences shown in sequence Nos. 17 and 19, and furthermore may be genes that code variants thereof.

### (1-3) Still another example of antibody enzyme according to the embodiment

Subsequently, as still another example of an antibody enzyme according to the invention, an antibody fragment of monoclonal antibody UA-15 of the HP urease will be exemplified and described. The antibody fragment of UA-15 is specifically a variable region of an L chain of the UA-15 antibody. The antibody enzyme as well, similarly to the HpU-18-L and so on, is obtained from a plurality of monoclonal antibodies of the HP urease as an amino acid sequence (antibody fragment) that can constitute a catalytic triad residue structure made of a serine residue, a histidine residue and an aspartate residue when the molecular modeling is applied to amino acid sequences of variable regions of the monoclonal antibodies to estimate the stereostructure.

The variable region of an L chain of the UA-15 antibody (hereinafter, referred to as UA-15-L) works as a degrading enzyme of the HP urease. Then, by decomposing the HP urease to completely destroy a function of the HP urease, resultantly the HP bacteria are rendered incapable of living in a strongly acidic stomach. That is, the antibody enzyme according to the invention that can decompose the peptides can efficiently eliminate the HP bacteria.

Then, a structure of the UA-15-L will be detailed below. The UA-15-L is, as mentioned above, a variable region of an L chain of UA-15 that is one of monoclonal antibodies of the HP urease and has an amino acid sequence shown in sequence No.21 as a primary structure.

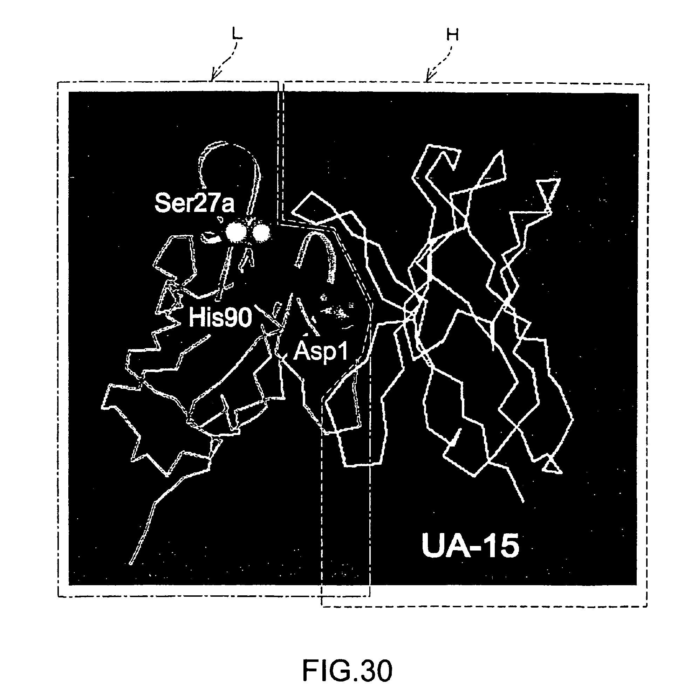

In Fig. 30, a stereostructure estimated by applying the stereostructure modeling (molecular modeling) to a variable region of UA-15 that is made of light and heavy chains is schematically shown. In Fig.30, a light chain is shown with L and a heavy chain is shown with H. As shown in Fig.30, in an amino acid sequence shown in sequence No.21, an aspartate residue at the 1^{st} (in Fig.30, shown as Asp1 by Kabat numbering scheme), a serine residue at the 28^{th} (in Fig.30, shown as Ser27a by Kabat numbering scheme) and a histidine residue at the 94^{th} (in Fig.30, shown as His90 by Kabat numbering scheme) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No.21, that is, a variant of the UA-15-L, and works as a degrading enzyme of the HP urease. Still furthermore, the antibody enzyme may be one that is obtained by arbitrarily adding a remaining amino acid sequence of the UA-15 antibody L chain to a C terminal side of the amino acid sequence shown in sequence No.21 or an entire length of the UA-15 antibody L chain.

The genes involving the invention are genes that code the abovementioned antibody enzymes, and, for instance, a gene made of a base sequence shown in sequence No.22 can be cited. The gene made of a base sequence shown in sequence No.22 is one of base sequences of a gene (cDNA) that codes a variable region of an L chain of the UA-15. However, genes involving the invention, without restricting thereto, may be various genes that code antibody enzymes having an amino acid sequence shown in sequence No.22, and furthermore may be genes that code variants thereof.

A variable region of a heavy chain of the UA-15 has an amino acid sequence shown in sequence No.23 as a primary structure; however, since, in the heavy chain of UA-15, a structure that is estimated to be a catalytic triad residue structure is not present, the UA-15 does not exhibit the activity as an antibody enzyme. Furthermore, a base sequence of a gene that codes the variable region of the heavy chain of the UA-15 is also shown as a sequence No. 24.

### (2) Method of obtaining antibody enzyme involving the embodiment

When an antibody enzyme involving the invention is an entire length of an H chain or an L chain of an antibody of the HP urease such as an HpU-18 L chain, an HpU-2 H chain, or L and H chains of HpU-20, or a UA-15 L chain, by making use of an existing antibody obtaining process, monoclonal antibodies of the corresponding HP urease are obtained followed by separating to H chains and L chains. Furthermore, when the antibody enzyme involving the invention is an antibody fragment of the HP urease, firstly corresponding monoclonal antibody is obtained, followed by cleaving the monoclonal antibody with a proper protease so as to obtain a target antibody fragment.

Furthermore, as to antibody enzymes of which amino acid sequences and gene sequences that code the amino acid sequences are known like the HpU-18-L, HpU-9-L, HpU-2-H, HpU-20-L, HpU-20-H and UA-15-L, a technology of genetic manipulation can be applied to obtain. In this case, a process in which a gene that codes the antibody enzyme is incorporated in a vector or the like, followed by expressibly introducing in a host cell to purify translated peptides in the cell can be adopted. When a gene that codes the antibody enzyme is incorporated together with a proper promoter that can express a lot, a target antibody enzyme may be efficiently obtained.

When an amino acid sequence of the antibody enzyme is not made clear, as will be shown in an example described later, firstly, an mRNA is obtained from a monoclonal antibody producing cell, a cDNA is synthesized from the mDNA, followed by reading a gene sequence thereof. Thereafter, an amino acid sequence is estimated from the gene sequence, a three-dimensional structure is estimated by the molecular modeling, and thereby whether a catalytic triad residue structure is contained or not is confirmed. Thereby, an antibody fragment in which the catalytic triad residue structure is contained can be obtained as an antibody enzyme.

When, of variants of HpU-18-L, HpU-9-L, HpU-2-H, HpU-20-L, HpU-20-H and UA-15-L, an arbitrary number of amino acid sequences remaining from an L chain or an H chain of the respective antibodies is added to a C terminal of the variants, for instance, in experiment 7 described later, a primer on 3' side may be appropriately changed.

Furthermore, as to a gene that codes an antibody enzyme involving the invention, in the case a base sequence thereof being made clear, after obtaining a cDNA thereof (or genome DNA), with the cDNA as a template, PCR is carried out with appropriate primer to multiply a corresponding region, thereby the gene can be obtained. Still furthermore, when a gene in which mutation is introduced is obtained by making use of the site-directed mutagenesis, in transformants in which the gene is introduced, variants of antibody enzymes made of amino acid sequences shown in sequence Nos.14, 15, 16, 17, 19 and 21 can be obtained as translated products.

### (3) Of application method of antibody enzyme according to the invention and gene that codes the antibody enzyme

Thus obtained antibody enzyme, with the nature as the antibody remained, can decompose the HP urease that is an antigen. Accordingly, it can be used as a disinfect agent of the HP bacteria that specifically works the HP urease. Furthermore, it is considered that since the antibody enzyme destroys the urease that is indispensable for the existence of the HP bacteria, different from the antibiotics that have been used as disinfect agents, the antibody enzyme does not impart the anti-drug resistance to the HP bacteria. Furthermore, the antibody enzyme not only can be developed as a drug, but also has likelihood, being able to be orally taken, by blending in healthy drinks or blending in milk products such as yogurt and butter, in the development of healthy foods for preventing HP bacteria infection.

Furthermore, a curative drug involving the invention for HP bacteria-infected patients contains an antibody enzyme having the abovementioned nature; accordingly, it is superior to the antibiotics that have been used as curative drugs. That is, since the abovementioned curative drug is high in the specificity to the HP urease, side effects can be reduced. Still furthermore, an existing curative drug, after it is taken, is absorbed in a stomach, followed by being circulated to a whole body to intrude without prejudice into the respective cells; on the other hand, the curative drug according to the invention, when it is taken orally, is sent into a stomach and can directly attack HP bacteria living in the stomach. Accordingly, although it goes without saying that the side effects are slight, it is considered that an effect can be instantaneously exhibited. Furthermore, the antibody enzyme contained in the curative drug, after decomposing the HP urease, is anyway decomposed by other protease; accordingly, the side effects are expected to be slight.

Still furthermore, the antibody enzyme according to the invention can be used to prepare a transformant by introducing a gene that codes the antibody enzyme into a proper host. That is, a transformant involving the invention is a transformant into which a gene that codes the antibody enzyme is introduced. Here, that "gene is introduced" means that a gene is expressibly introduced in a target cell (host cell) by use of a known genetic engineering procedure (genetic manipulation technology). The transformant can express the antibody enzyme in its body.

Furthermore, the "transformant" includes transformed plants. When the gene is expressibly introduced in a plant, a transformed plant can be obtained. In a category of the transformant and the transformed plant, individual living matters; various kinds of organs such as roots, stalks, leaves and sex organs (including flower organs and seeds); various tissues; cells; and so on are included, and furthermore protoplasts, induced calluses, regenerated individuals and offspring thereof are included as well. Such transformed plants can be used as a curative drug against HP bacteria-infected patients or an infection preventive agent against the HP bacteria. In the case of the transformed plants being produced, when for instance tomato, cucumber or carrot is used as a host plant, there is likelihood of obtaining a high-performance plant containing the antibody enzyme. When this is taken on a daily basis, the HP bacteria can be inhibited from infecting. The high-performance plant can be expected to commercialize as functional foods for preventing the infection of the HP bacteria.

### [Embodiment 3]

In the present embodiment 3, as a more specific example of an antibody enzyme involving the invention, an antibody enzyme against chemokaine receptor CCR-5 will be explained. The invention is not restricted to the description.

### (1-1) About antibody enzyme involving the embodiment and gene

Here, as an example of antibody enzymes according to the invention, antibody fragments of monoclonal antibodies ECL2B-2 and ECL2B-3 of the chemokine receptor CCR-5 are exemplified and explained. The antibody fragments of monoclonal antibodies ECL2B-2 and ECL2B-3 are, more specifically, a variable region of a light chain of ECL2B-2 (that is, ECL2B-2-L) and a variable region of a light chain of ECL2B-3 (that is, ECL2B-3-L).

The two antibody enzymes are obtained from monoclonal antibodies obtained by using peptides (RSSHFPYSQYQFWKNFQTLK (sequence No.38) or RSQKEGLHYTCS (sequence No.39)) that form an extracellular region of the chemokine receptor CCR-5 as an immunogen. When the molecular modeling is applied to amino acid sequences in variable regions of the monoclonal antibodies to estimate a stereostructure thereof, the abovementioned antibody enzymes were found out as amino-acid sequences (antibody fragment) that can constitute a catalytic triad residue structure made of a serine residue, a histidine residue (or glutamate residue) and an aspartate residue.

The antibody enzymes ECL2B-2-L and ECL2B-3-L according to the invention work as degrading enzyme of the chemokine receptor CCR-5. This, as will be shown also in an example described below, is obvious from a result that the ECL2B-2-L and ECL2B-3-L completely decompose an extracellular region peptide of the chemokine receptor CCR-5 (peptide made of an amino acid sequence shown in sequence No.38 or 39).

Now, the chemokine receptor CCR-5 (hereinafter, referred as CCR-5) will be briefly explained.

The CCR-5 is a coreceptor of AIDS virus HIV-1, and considered to induce a conformational change in an HIV-1 envelope to cause membrane fusion between virus and host cell; accordingly, it is an important element when a human is infected with the HIV-1. A human having a defective mutant CCR-5 gene exhibits resistance to the infection and crisis of the HIV-1.

Furthermore, the CCR-5 is one and only one that can be used as a coreceptor in the infection of a macrophage-directed clone of the HIV-1 that is responsible for the infection from a human to another human. From this, it is expected that when the function of the CCR-5 can be suppressed, the HIV can be prevented from infecting and disease can be inhibited from progressing.

The abovementioned two antibody enzymes can completely decompose the CCR-5 and can delete the function thereof. A human who has a defective mutant CCR-5 gene has no obvious immunity deficiency and tissue lesion and has no problem in health; accordingly, the antibody enzymes that can delete the function of the CCR-5 are considered effectively utilized as an anti-HIV drug that prevents the HIV from infecting and AIDS symptom from progressing.

Subsequently, a structure of the ECL2B-2-L will be detailed below. The ECL2B-2-L is a variable region of a light chain of monoclonal antibody ECL2B-2 of which immunogen is an extracellular region peptide of the CCR-5 and has an amino acid sequence shown in sequence No.26 as a primary structure.

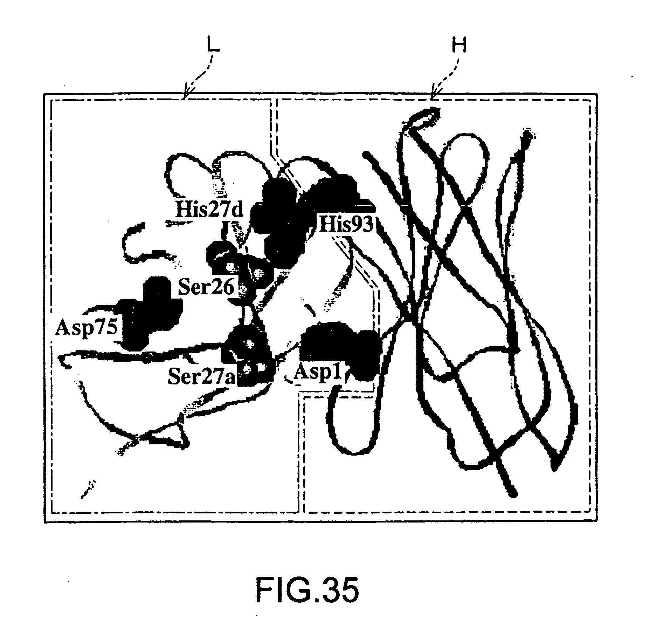

In Fig.35, a stereostructure estimated after the stereostructure modeling (molecular modeling) is applied to a variable region of the ECL2B-2 that is made of a light chain and a heavy chain is schematically shown. In Fig.35, a light chain is expressed with L and a heavy chain is expressed with H. As shown in Fig.35, in an amino acid sequence shown in sequence No.26, an aspartate residue at the 1^{st} (in Fig.35, denoted as Asp1 by Kabat numbering scheme), a serine residue at the 28^{th} (in Fig.35, denoted as Ser27a by Kabat numbering scheme), and a histidine residue at the 98^{th} (in Fig. 35, denoted as His93 by Kabat numbering scheme), or, in place of the histidine at the 98^{th}, a histidine residue at the 31^{st} (in Fig. 35, denoted as His27d) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No.26, that is, a variant of the HECL2B-2-L and works as a degrading enzyme of the CCR-5. Still furthermore, the antibody enzyme may be one that is obtained by arbitrarily adding a remaining amino acid sequence of the ECL2B-2 antibody L chain to a C terminal side of the amino acid sequence shown in sequence No.26.

A variable region of a heavy chain of the ECL2B-2 has an amino acid sequence shown in sequence No.28 as a primary structure; however, in the heavy chain of the ECL2B-2, there is no structure that allows estimating a catalytic triad residue structure. Furthermore, a base sequence of a gene that codes the variable region of the heavy chain of the ECL2B-2 is shown together as a sequence No.29.

Subsequently, a structure of the ECL2B-3-L will be detailed below. The ECL2B-3-L is a variable region of a light chain of monoclonal antibody ECL2B-3 of which immunogen is an extracellular region peptide of the CCR-5 and has an amino acid sequence shown in sequence No.30 as a primary structure.

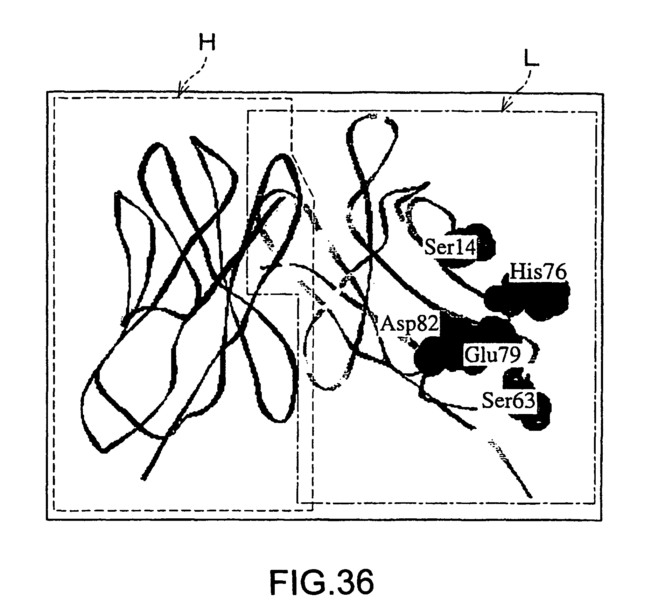

In Fig.36, a stereostructure estimated after the stereostructure modeling (molecular modeling) is applied to a variable region of the ECL2B-3 that is made of a light chain and a heavy chain is schematically shown. In Fig.36, a light chain is expressed with L and a heavy chain is expressed with H. As shown in Fig.36, in an amino acid sequence shown in sequence No.30, an aspartate residue at the 86^{th} (in Fig.36, denoted as Asp82 by Kabat numbering scheme), a serine residue at the 14^{th} (in Fig.36, denoted as Ser14 by Kabat numbering scheme), or in place of the serine residue at the 14^{th}, a serine residue at the 67^{th} (in Fig.36, denoted as Ser63 by Kabat numbering scheme), and a histidine residue at the 80^{th} (in Fig.36, denoted as His76 by Kabat numbering scheme) are estimated to form a catalytic triad residue structure.

Furthermore, the antibody enzyme according to the invention may be one that is made of an amino acid sequence that is obtained by replacing, deleting, inserting and/or adding at least one amino acid in an amino acid sequence shown in sequence No. 30, that is, a variant of the ECL2B-3-L and works as a degrading enzyme of the CCR-5. Still furthermore, the antibody enzyme may be one that is obtained by arbitrarily adding a remaining amino acid sequence of the ECL2B-3 antibody L chain to a C terminal side of the amino acid sequence shown in sequence No.30.

A variable region of a heavy chain of the ECL2B-3 has an amino acid sequence shown in sequence No.32 as a primary structure; however, in the heavy chain of the ECL2B-3, there is no structure that allows estimating a catalytic triad residue structure. Furthermore, a base sequence of a gene that codes the variable region of the heavy chain of the ECL2B-3 is shown together as a sequence No.33.

The gene involving the invention is a gene that codes the antibody enzyme, more specifically, one made of a base sequence shown in sequence No.27 or one made of a base sequence shown in sequence No. 31 can be cited. A gene that is made of a base sequence shown in sequence No.27 is one of base sequences of a gene that codes the variable region of the L chain of the ECL2B-2 and a gene that is made of a base sequence shown in sequence No.31 is one of base sequences of a gene that code the variable region of the L chain of the ECL2B-3.

### (1-2) Another example of antibody enzyme according to the embodiment

Subsequently, as another example of antibody enzyme according to the invention, antibody fragments of monoclonal antibody ECL2B-4 of the chemokines receptor CCR-5 are exemplified and explained. The antibody fragments of monoclonal antibody ECL2B-4 are, more specifically, a variable region of a light chain of ECL2B-4 (that is, ECL2B-4-L) and a variable region of a heavy chain of ECL2B-4 (that is, ECL2B-4-H).

The two antibody enzymes as well, similarly to the ECL2-3-L and the like, are obtained from monoclonal antibodies obtained by using an extracellular region peptide of chemokine receptor CCR-5 as an immunogen. When the molecular modeling is applied to amino acid sequences of a variable region of the monoclonal antibody to estimate a stereostructure thereof, the antibody enzymes were found out as amino acid sequences (antibody fragments) that can constitute a catalytic triad residue structure made of a serine residue, a histidine residue (or glutamate residue) and an aspartate residue.

The antibody enzymes ECL2B-4-L and ECL2B-4-H as well, as will be shown in an example described below, decompose an extracellular region peptide (peptide made of an amino acid sequence shown in sequence No.38) of the chemokine receptor CCR-5; accordingly, these work as the degrading enzyme of the chemokine receptor CCR-5.

That is, the two antibody enzymes as well can completely decompose the CCR-5 and thereby can delete the function thereof. Accordingly, it is considered that the antibody enzymes can be effectively utilized as an anti-HIV drug that can prevent the HIV from infecting and suppress the AIDS from progressing.

In the next place, a structure of the ECL2B-4-L will be detailed below. The ECL2B-4-L is a variable region of a light chain of monoclonal antibody ECL2B-2 of which immunogen is an extracellular region peptide of the CCR-5 and has an amino acid sequence shown in sequence No.34 as a primary structure.

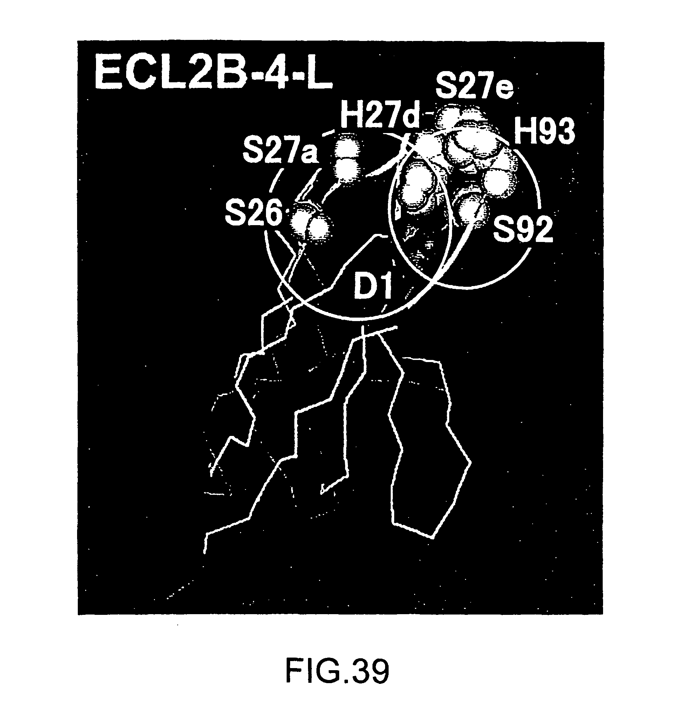

In Fig.39, a stereostructure estimated after the stereostructure modeling (molecular modeling) is applied to a variable region of the ECL2B-4-L is schematically shown. As shown in Fig.39, in an amino acid sequence shown in sequence No.34, an aspartate residue at the 1^{st} (in Fig.39, denoted as D1 by Kabat numbering scheme), a serine residue at the 28^{th} (in Fig.39, denoted as S27a by Kabat numbering scheme), a histidine residue at the 31^{st} (in Fig.39, denoted as H27d by Kabat numbering scheme) are estimated to form a catalytic triad residue structure. In addition, in place of the histidine residue at the 31^{st}, a histidine residue at the 98^{th} (in Fig.39, denoted as H93 by Kabat numbering scheme) can be used. Furthermore, in place of the serine residue at the 31^{st}, any one of the 28^{th} (in Fig.39, denoted as S27a), the 32^{nd} (in Fig. 39, denoted as S27e), and the 97^{th} (in Fig.39, denoted as S92) can be used.

Furthermore, the antibody enzyme according to the invention may be one that is made of an amino acid sequence that is obtained by replacing, deleting, inserting and/or adding at least one amino acid in an amino acid sequence shown in sequence No.34, that is, a variant of the HECL2B-4-L and works as a degrading enzyme of the CCR-5. Still furthermore, the antibody enzyme may be one that is obtained by arbitrarily adding a remaining amino acid sequence of the ECL2B-4 antibody L chain to a C terminal side of the amino acid sequence shown in sequence No.34.

Subsequently, a structure of the ECL2B-4-H will be detailed below. The ECL2B-4-H is a variable region of a heavy chain of monoclonal antibody ECL2B-4 of which immunogen is an extracellular region peptide of the CCR-5 and has an amino acid sequence shown in sequence No.36 as a primary structure.

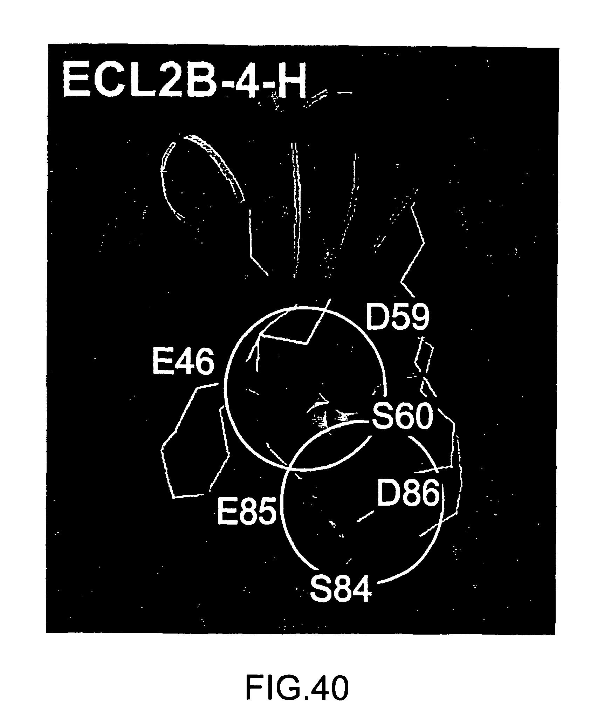

In Fig.40, a stereostructure estimated after the stereostructure modeling (molecular modeling) is applied to a variable region of the ECL2B-4-H is schematically shown. As shown in Fig.40, in an amino acid sequence shown in sequence No.36, an aspartate residue at the 92^{nd} (in Fig.40, denoted as D86 by Kabat numbering scheme), a serine residue at the 65^{th} (in Fig.40, denoted as S60 by Kabat numbering scheme), and a glutamate residue at the 48^{th} (in Fig. 40, denoted as E46 by Kabat numbering scheme) are estimated to form a catalytic triad residue structure. In place of aspartate residue at the 92^{nd}, an aspartate residue at the 64^{th} (in Fig.40, denoted as D59 by Kabat numbering scheme) may be used. Furthermore, in place of serine residue at the 65^{th}, a serine residue at the 90^{th} (in Fig.40, denoted as S84 by Kabat numbering scheme) may be used. Still furthermore, in place of glutamate residue at the 48^{th}, a glutamate residue at the 91^{st} (in Fig.40, denoted as E85) may be used.

Furthermore, the antibody enzyme according to the invention may be one that is made of an amino acid sequence that is obtained by replacing, deleting, inserting and/or adding at least one amino acid in an amino acid sequence shown in sequence No.36, that is, a variant of the ECL2B-4-H and works as a degrading enzyme of the CCR-5. Still furthermore, the antibody enzyme may be one that is obtained by arbitrarily adding a remaining amino acid sequence of the ECL2B-4 antibody H chain to a C terminal side of the amino acid sequence shown in sequence No.36.

The gene involving the invention is a gene that codes the antibody enzyme, and, more specifically, one made of a base sequence shown in sequence No.35 or one made of a base sequence shown in sequence No.37 can be exemplified. A gene that is made of a base sequence shown in sequence No.35 is one of base sequences that code the variable region of the L chain of the ECL2B-4 and a gene that is made of a base sequence shown in sequence No.37 is one of base sequences of a gene that codes the variable region of the H chain of the ECL2B-4.

### (2) Method of obtaining antibody enzyme involving the embodiment

When an antibody enzyme involving the invention is an entire length of an L chain of an antibody of CCR-5 such as an entire length of an L chain of ECL2B-3, an entire length of an L chain of ECL2B-3, or an entire length of an L chain of ECL2B-4, an entire length of an H chain of ECL2B-4, by making use of a so far known antibody obtaining process, monoclonal antibodies of the corresponding CCR-5 are obtained followed by separating H chains and L chains. Furthermore, when the antibody enzyme involving the invention is an antibody fragment of the CCR-5, firstly corresponding monoclonal antibodies are obtained, followed by cleaving the monoclonal antibodies with a proper protease so as to obtain target antibody fragments.

Furthermore, as to antibody enzymes of which amino acid sequences and gene sequences that code the amino acid sequences are clarified like the ECL2B-2-L, ECL2B-3-L, ECL2B-4-L, and ECL2B-4-H, a so far known technology of genetic manipulation can be applied to obtain. In this case, a process in which genes that code the antibody enzymes are incorporated in a vector or the like, followed by expressibly introducing in a host cell to purify translated peptides in the cell can be adopted. When genes that code the antibody enzymes are incorporated together with a proper promoter that can express a lot, target antibody enzymes may be efficiently obtained.

When an amino acid sequence of the antibody enzyme is not clear, firstly, an mRNA is obtained from a monoclonal antibody producing cell or a hybridoma thereof, a cDNA is synthesized from the mDNA, followed by reading a gene sequence thereof. Thereafter, an amino acid sequence is estimated from the gene sequence, a three-dimensional structure is estimated by the molecular modeling, and thereby whether a catalytic triad residue structure is contained or not may be confirmed. Thereby, an antibody fragment in which the catalytic triad residue structure is contained can be obtained as an antibody enzyme.

Furthermore, as to a gene that codes an antibody enzyme involving the invention, in the case a base sequence thereof being made clear, after obtaining a cDNA thereof (or genome DNA), with the cDNA as a template, PCR is carried out with an appropriate primer to multiply a corresponding region, thereby the gene can be obtained. Still furthermore, when, by making use of the site-directed mutagenesis, a proper mutation is introduced into a gene that is made of a base sequence shown in sequence No.27, 31, 35 or 37, in a transformant in which the gene is introduced, a variant of an antibody enzyme made of an amino acid sequence shown in sequence No.26, 30, 34, or 36 can be obtained as a translated product.

### (3) Method of application of antibody enzyme according to the embodiment

So far, anti-HIV drugs that suppress the CCR-5 from working have been developed; however, the antibody enzymes according to the invention, based on an utterly different process from the abovementioned anti-HIV drugs, can attack the CCR-5 directly to delete the function. Accordingly, the antibody enzymes can be utilized as an anti-HIV drug.

An anti-HIV drug containing the present antibody enzymes, owing to a novel mode of action of enzymatically completely decomposing the CCR-5 to delete the function thereof, can inhibit the HIV from infecting and suppress the AIDS from developing. Such an anti-HIV drug specifically aims a CCR-5 molecule alone; accordingly, it is considered that a significant effect can be expected and at the same time the side effects are less. Furthermore, considering from a reaction mechanism of the antibody enzyme, when it is used in combination with other anti-HIV drugs that are now in use, curative effects against the AIDS can be expected to improve.

Furthermore, the antibody enzyme according to the invention, when a gene that codes the antibody enzyme is introduced into an appropriate host, can be used to prepare a transformant. That is, a transformant involving the invention is a transformant into which a gene that codes the antibody enzyme is introduced. Here, that "a gene is introduced" means that a gene is expressively introduced into a target cell (host cell) by means of an existing genetic engineering procedure (genetic manipulation technology). The transformant can express the antibody enzyme in its body.

As the transformant involving the invention, more specifically, one in which a gene made of a base sequence shown in sequence No.27 or 31 (sequence No.35 or 37 may be used) is introduced in a host cell can be cited. As the host cell, usually used ones such as bacteria coli, yeast and baculovirus can be properly used.

The transformant specifically recognizes the CCR-5 in its body and completely decompose to delete the function thereof. Accordingly, it can be used as an anti-HIV drug. Furthermore, when the transformant can express a lot of the antibody enzymes, it is considered that the HIV can be efficiently inhibited from infecting and remedy of the symptom of the AIDS can be efficiently performed; that is, there is likelihood of obtaining a more effective anti-HIV drug.

The amino acid numbers in the amino acid sequences of the respective antibody enzymes shown in Figs.4A, 4B, 8A, 8B, 13, 15, 17, 23, 24, 41 and 42 explained in the embodiment are due to Kabat numbering scheme; accordingly, these are different from numbers of amino acids shown in the corresponding respective sequence numbers.

### [Example 1]

An example relating to the embodiment 1 according to the invention will be described below. In the present example, results of the investigations of the physical properties of antibody enzymes involving the invention: i41SL1-2-H, i41-7-H, i41SL1-2-L and i41-7-L will be explained in turn.

### [Example 1-1: Preparation of antibody]

I41SL1-2 and i41-7 antibodies are prepared according to a method shown below.

Specifically, firstly, a synthesized polypeptide made of an amino acid sequence shown in sequence No.9 or a light chain of antibody 41S-2 shown in sequence No.13, respectively, is coupled with a carrier protein by use of m-maleimidobenzoyl-N-hydroxysuccinimide ester (MBS) or glutaraldehyde, and coupled one is used as an antigen protein to immunize. As the carrier protein, KLH (Keyhole Limpet Hemocyanin) was used. In the next place, by use of the antigen protein, a Balb/c mouse was immunized.

Subsequently, a spleen was taken out of the immunized mouse to prepare spleen cells. The spleen cells and separately prepared myeloma cells derived from mouse bone marrow were fused by use of polyethylene glycol, followed by a HAT selection. Then, by screening by use of an ELISA method, antibody producing-positive cell groups were selected, and the cell groups were cloned to obtain antibody producing-hybridomas. The hybridomas were transplanted in a mouse abdominal cavity and abdominal dropsy was sampled as monoclonal antibodies. As the i41-7 antibodies, in a screening stage, ones well reacted with 41S-2 antibody light chains were selected. Prepared antibodies were purified by means of affinity chromatography.

The purity of the purified antibodies was confirmed by use of SDS-PAGE.

### [Example 1-2: Three-dimensional structure analysis of monoclonal antibody]

Firstly, base sequences of various kinds of monoclonal antibodies were determined. Specifically, mRNAs were extracted from various kinds of antibody producing-cells, followed by synthesizing cDNAs by RT-PCR. Antibody genes were amplified by means of PCR with the cDNAs as a template, incorporated in pGEM-T vector (pGEM-T (registered trade mark) and p-GEM-T (registered trade mark) Easy Vector System, manufactured by Promega Corporation) and sub-cloned in E. coli JM109. After liquid culturing, plasmids in which a target gene was incorporated were purified and base sequences thereof were determined.

In the next place, as reference data, amino acid sequences of variable regions of monoclonal antibodies estimated from the base sequences (35 clones) and amino acid sequences of antibodies registered in Protein Data Bank (PDB) (Research Collaboratory for Structural Bioinformatics) (56 clones) were used. Of these, firstly, by use of software AbM (manufactured by Oxford molecular Ltd.,), three-dimensional structures of the antibodies were formed, followed by minimizing energy by use of Discover (manufactured by Molecular Simulations Inc.,).

### [Example 1-3: Analysis of germline gene]

Among the antibodies thereto the three-dimensional structural analysis was applied, of mouse-derived antibodies in which a class of an L chain is κ type, by use of Ig BLAST (manufactured by National Center for Biotechnology Information), a Vκ germline was searched. The germlines therefrom the respective antibodies were derived were estimated and, of amino-acid residues estimated to constitute a catalytic triad residue structure, layouts of the amino-acid residues and situations of variations from the germlines were analyzed in detail. Specifically, the procedure was as follows.

To each of 56 clones of monoclonal antibodies extracted at random from Protein Data Bank (PDB) and 34 clones of monoclonal antibodies extracted at random from the inventor's laboratory, according to the method described in the example 1-2, the molecular modeling was applied to analyze a stereostructure of a variable region. In the invention, with the sequence data of the PDB, the molecular modeling was carried out to analyze a stereostructure of a variable region. This is because the inventors considered that when all data are analyzed of the stereostructure with the same software, the data of all antibodies could be equally evaluated. That is, when in one case the evaluation was performed with data due to the X-ray crystal structure analysis and in another case, with data due to the molecular modeling, in evaluation, irregular data are considered used.

When 56 clones randomly extracted from the PDB are broken down, 52 clones were mouse-derived ones and 4 clones were human-derived ones. The antibody L chains are classified into two kinds of classes, κ and λ, and it is said that, in the case of mouse, substantially 95% is κ chain. Furthermore, since an analysis of germline genes of mouse κ chains is advanced, information thereof can be advantageously obtained. In this connection, mouse κ chains (47 clones) were analyzed in more detail.

From these, clones relevant to the catalytic triad residue structures were selected and classified as follows. Firstly, ones that constitute the catalytic triad residue structure from Asp1, Ser27A and His93 that are common with the abovementioned i41SL1-2-L were classified as type ⓞ. Then, ones that constitute the catalytic triad residue structure from Asp1, Ser26 and His 93 were classified as type ○, ones that constitute the catalytic triad residue structure from combinations (for instance, His90 or His91 is used) other than combinations of [Asp1, Ser27A or Ser26, and His93] were classified as type ●, ones that, owing to the mutation from the germlines, can not constitute the catalytic triad residue structure were classified as type x, and ones that can constitute the catalytic triad residue structure including amino acids mutated from the germlines were classified as type *.

Firstly, results of the classification of the germlines of all 47 clones are shown in Table 1-1 and Table 1-2. The Table 1-2 is continuation of Table 1-1.

**[Table 1-1]**

| **MOUSE-K** | | | | | |
|---|---|---|---|---|---|
| | | Antigen | H chain | L chain | |
| | | | Vh germline | Vk germline | triad |
| 1 | 1A0Q | Norleucine phenyl phosphonate transition state analog | V23 | gj38c | |
| 2 | 1A3L | Diels-Alder protein | V6 | he24 | |
| 3 | 1A4K | Diels-Alder protein | VGK1A | bb1 | × |
| | | A triad cannot be formed because germline D1 mutated to E1. | | | |
| 4 | 1A5F | E-secretin | V130 | 8-19 | |
| 5 | 1AJ7 | Para-nitrophenyl phosphonate transition-state analog | V130 | cw9 | |
| 6 | 1AY1 | DNA polyI of thermophile | VH36-60 | at4 | |
| 7 | 1B4J (chimeric) | γ-interferon | V3 | 19-20 | |
| 8 | 1BAF | Dinitrophenol | VH36-60 | at4 | |
| 9 | 1BBD | Rhinovirus | V130 | 8-19 | |
| 10 | 1BFV | Steroid, glucuronide | VH283 | bb1 | ⓞ |
| 11 | 1C1E | Diels-Alder protein | VGK1B | bb1 | × |
| | | A triad cannot be formed because germline D1 is mutated to E1. | | | |
| 12 | 1CLO | Cancer embryo antigen | V11 | am4 | |
| 13 | 1DBA | Steroid (androstanedione,progesterone) | VGK1A | bb1 | ⓞ |
| 14 | 2DBL | Progesterone | VGK1A | bb1 | ⓞ |
| 15 | 2DLF | Dansyl chloride | VH22.1 | bb1 | ⓞ |
| 16 | 1DSF | Tumor (including Lewis^{Y}) | VH37.1 | bb1 | ○ |
| | | A triad can be formed with S26 derived from a germline, though germline S27a is mutated to N27a. | | | |
| 17 | 2F58 | HIV-1 gp120, (HIGPGRAFGGG) | VH36-60 | 21-4 | * |
| | | A triad can be formed from D27c-S31-H92 because germline N92 is mutated to H92. | | | |
| 18 | 6FAB | P-azophenylarsonate | V23 | ce9 | |
| 19 | 1FA1 | Arsonic acid | V23 | ce9 | |
| 20 | 1FGN | Blood coagulation III factor | V130 | ba9 | |
| 21 | 1FOR | Rhinovirus | VMU-3.2 | ap4 | |
| 22 | 1FRG | Influenza virus | VH37.1 | 8-19 | |
| 23 | 2FVB | α (1-6), dextrin | V6 | kk4 | |

**[Table 1-2]**

| | | | | | |
|---|---|---|---|---|---|
| 24 | 1GAF | Para-nitrophenyl phosphonate transition-state analog | V130 | cw9 | |
| 25 | 1GGB | HIV-1, (CKRIHIGPGRAFYTTC) | CB17H-1 | 21-4 | |
| 26 | 1GPO | Cystatin, (cysteine inhibitor) | VH36-60 | 23-43 | |
| 27 | 2H1P | PA1 (cryptococcal capsular poly-saccharide) | VH7183.10 | bb1 | ⓞ |
| 28 | 1HFM | Lysozym | VH36-60 | 23-43 | |
| 29 | 1HIN | Influenza virus, hemagglutinin peptide (YDVPDYAS) | VH37.1 VH69.1 | 8-19 | |
| 30 | 1HYY | Phosphonate transition state analog 3 | V-BK | cr1 | * |
| | | Since germline D1 is varied to E1, and S27a to T27a, a triad derived from germlines cannot be formed. However, since N30 is mutated to D30, a triad from H27d or H93-S27e-D30 can be formed. | | | |
| 31 | 1IAl | Anti-peritonitis virus antigen | VGK1A | 19-17 | ● |
| | | A germline-derived triad can be formed from S26-D28-H91. | | | |
| 32 | 1IBG | Digoxin | V0x-1 | 21-12 | ● |
| | | A germline-derived triad can be formed from D1-S26 or S27a-H90. | | | |
| 33 | 1IGC | Protein G | VHMOPC21 | 19-20 | |
| 34 | 1IGF | C helix of myohemerythrins | VH69.1 | cr1 | ⓞ |
| 35 | 1IGI | Digoxin | 45.21.1 | bb1 | ⓞ |
| 36 | 1JEL | Protein containing histidine (85 amino acid) | 45.21.1 | cr1 | ⓞ |
| 37 | 1JHL | Hen egg lysozym | V3 | RF | |
| 38 | 1KB5 | Antigen receptor of T cell | 45.21.1 | 12-44 | ● |
| | | A germline-derived triad can be formed from D1-S26-H90 or H91. | | | |
| 39 | 1KEL | Periodate and nitroaryl sulfide | V11 | cr1 | ⓞ |
| 40 | 1MF2 | HIV-1 protease | VHMOPC21 | 21-2 | |
| 41 | 1MIG | Para-nitrophenyl phosphonate transition-state analog | V0x-1 | 8-24 | |
| 42 | 1MIM | Interleukin 2 receptor (CD25) | V119.13 | kn4 | |
| 43 | 1QBL | Cytochrome C | V130 | 12-41 | ● |
| | | A germline-derived triad can be formed from D1-S26-H90. | | | |
| 44 | 1QNZ | HIV-1 gp120, (RKSIRIQRGPGRAFVTIG) | V102 | 21-4 | |
| 45 | 1SBS | Gonadotropin, (gonadtropic hormone) | VH22.1 | 8-30 | |
| 46 | 1UCB | Tumor, (including Lewis^{Y}) | VH50.1 | cr1 | ○ |
| | | A triad can be formed with S26 derived from germline, though germline S27a is mutated to N27a. | | | |
| 47 | 1YEC | Phosphonate, transition state analog | V6 | bj2 | ⓞ |

As shown in Tables 1 and 2, clones corresponding to ⓞ, ○ and ● were found 16 (34.0% of total). In the mouse-derived L chains of which class is κ chain, 12 clones of 47 clones were fitted to the ⓞ or ○.

From results of classification of the germlines of 47 clones shown in Tables 1-1 and 1-2, ones that can constitute a catalytic triad residue structure are extracted and shown in Table 2.

**[Table 2]**

| No. (serial No through all) | PDB ID | antigen | germline | triad |
|---|---|---|---|---|
| 1 (3) | 1A4K | Diels-Alder protein | bb1 | × |
| 2 (10) | 1BFV | Steroid, glucuronide | bb1 | ⓞ |
| 3 (11) | 1C1E | Diels-Alder protein | bb1 | × |
| 4 (13) | 1DBA | Steroid (androstanedione,progesterone) | bb1 | ⓞ |
| 5 (14) | 2DBL | Progesterone | bb1 | ⓞ |
| 6 (15) | 2DLF | Dansyl chloride | bb1 | ⓞ |
| 7 (16) | 1DSF | Tumor, (including Lewis^{Y}) | bb1 | ○ |
| 8 (27) | 2H1P | PA1 (cryptococcal capsular poly-saccharide) | bb1 | ⓞ |
| 9 (30)* | 1HYY | Phosphonate transition state analog 3 | cr1 | * |
| 10 (31) | 11Al | Anti-peritonitis virus antigen | 19-17 | ● |
| 11 (32) | 11BG | Digoxin | 21-12 | ● |
| 12 (34) | 11GF | C helix of myohemerythrins | cr1 | ⓞ |
| 13 (35) | 11Gl | Digoxin | bb1 | ⓞ |
| 14 (36) | 2JEL | Protein including histidine (85 amino acid) | cr1 | ⓞ |
| 15 (38) | 1KB5 | Antigen receptor of T cell | 12-44 | ● |
| 16 (39) | 1KEL | Periodate and nitroaryl sulfide | cr1 | ⓞ |
| 17 (43) | 1QBL | Cytochrome C | 12-41 | ● |
| 18 (46) | 1UCB | Tumor, (including Lewis^{Y}) | cr1 | ○ |
| 19 (47) | 1YEC | Phosphonate, transition state analog | bj2 | ⓞ |

As shown in the Table 2, there is commonality between antibodies that constitute a catalytic triad residue structure of type ① (or ① *), that is, 9 clones of 19 clones were bb1 and 3 clones were cr1. In addition, the germline of the abovementioned i41SL1-2-L was bd2.

In the next place, characteristics common through the germlines bb1, cr1 and db2 were investigated.

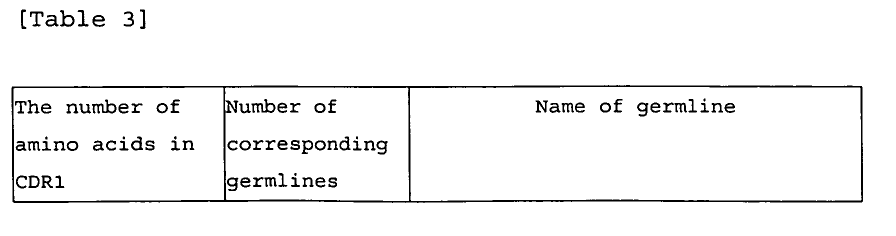

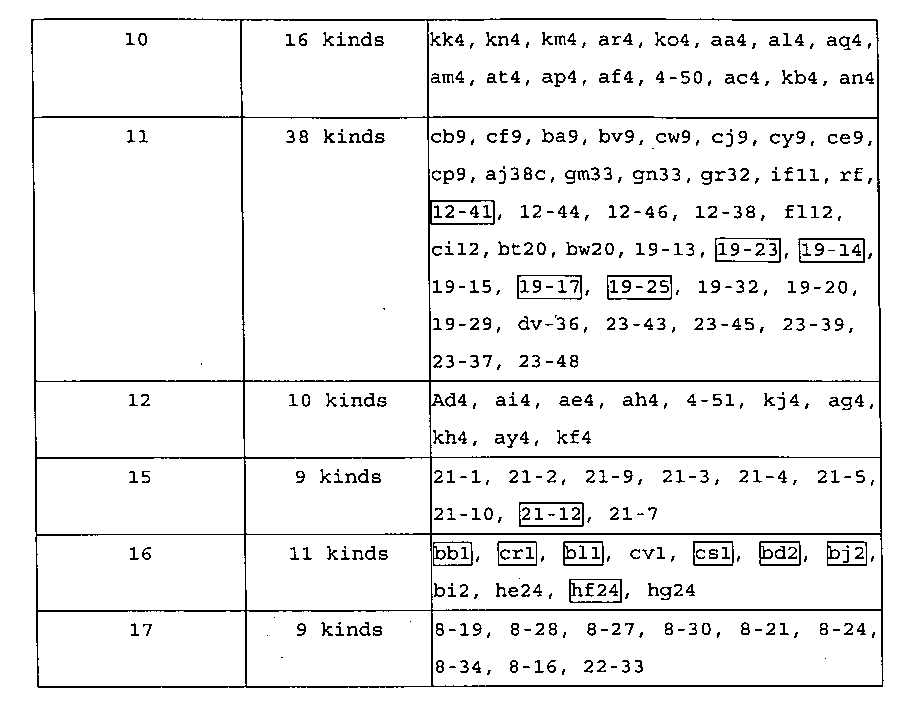

As shown in the above Table 3, in all of these germlines, the complimentarity determining region 1 (CDR1) was formed of 16 amino-acid residues. The CDR sequence is a place where a large mutation is caused so that an antibody may strongly recognize an antigen (this is a reason why it,is called a complimentarity determining region).

Then, the relationship between Asp1, Ser27A and His93 sequences and the germlines was further analyzed.

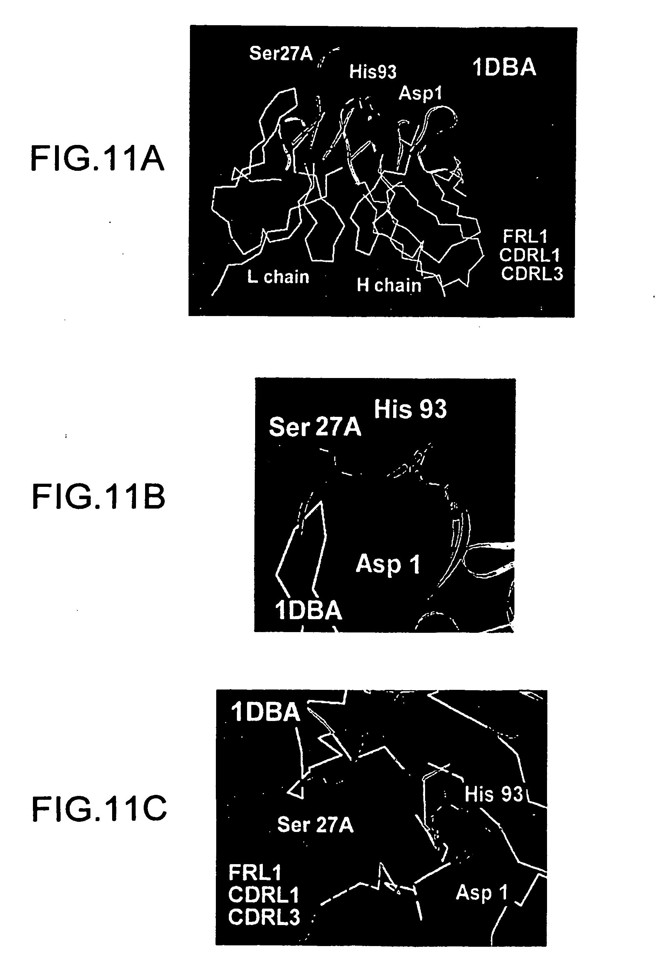

In Figs.11A, 11B and 11C, results of the molecular modeling applied to 1DBA clone of which germline is bb1 by Thiebe and et al. convention are shown. An FR is shown with wire and a CDR is shown with ribbon. The Asp1, Ser27A and His93, respectively, are present in regions FR1, CDR1 and CDR3 different from each other in the primary structure. However, it is found that as shown in Figs.11B and 11C, the three amino-acid residues are located in the neighborhood of a tip end where the His93 of the CDR3 loop is just present and at a height same as that of the His93, that is, on a substantially same plane when seen in a horizontal direction.

These correspond to a tip end portion of a space where loops of the complimentarity determining region concentrate; accordingly, it is considered that this is a position where without stereostructural drawbacks an antigen can be incorporated. Furthermore, as shown in Fig.11A, when 16 amino acids constitute the CDR1, a loop of the CDR1 takes a structure that slowly undulates just in the neighborhood, and the tip end portion (8 amino-acid residues from 27B to 31) higher than the structure protrudes higher than other loops. Accordingly, the antibody enzyme has a catalytic triad residue structure in a position where an antigen peptide can be incorporated without the stereostructural drawbacks; accordingly it is considered that an antigen peptide can be smoothly decomposed. The stereostructural features were common in 10 antibody L chains shown in the Table 2 and the i41SL1-2-L.

In the next place, when occurrence frequencies of Asp1, Ser27A and His93 in the germlines of the data due to Thiebe et al. (reference literature 9: Thiebe R, Schanble KF et al., Eur J Immnol, 29 (7), 2082-2081 (1999)) were investigated, the occurrence frequency of one in which an aspartate residue is used at the 1^{st} was 58 kinds of 93 kinds in total, that is, 62.3% of the whole (the aspartate residue is an amino-acid residue that, without restricting to the 1^{st}, distributes all over the variable region). The serine (Ser) residue, 27A, is in the CDR1; however, a Ser in the neighborhood of the 27A can form a catalytic triad residue structure.

When such Sers are included, germlines containing effective Sers amounted to 92 kinds. That is, there are many germlines in which an Asp1 and a Ser in the CDR1 portion are located in stereostructurally proximity to each other. On the other hand, the occurrence rate of the His is low; that is, germlines where the His is at the 93^{rd} amounted only to 7 kinds (bb1, cr1, b11, cs1, bd2, bj2 and 8-16). Furthermore, 6 kinds thereof (ones other than 8-16) all can form a catalytic triad residue structure and are the germlines such as bb1 in which the CDR1 is constituted of 16 amino-acid residues. That is, 6 kinds of bb1, cr1, b11, cs1, bd2 and bj2 shown in Table 3 can be said that these are germlines in which amino-acid residues are located at positions ideal for decomposing an antigen.

In the next place, whether antibodies produced from the germlines have the antigen degrading activity or not was experimentally investigated. The present inventors have prepared one hundred and several tens kinds of monoclonal antibodies and germlines (antibody light chain (mouse type κ chain)) of 34 clones thereof were determined. Among these, data of ones that were supplied for experiments are summarized in Table 4.

**[Table 4]**

| No | clone | germline | triad | No | clone | germline | triad | No | clone | germline | triad |
|---|---|---|---|---|---|---|---|---|---|---|---|
| 1 | 41S-2 | hf24 | *● | 14 | HpU9 | cs1 | ⓞ | 27 | ECL2B-2 | cr1 | ⓞ |
| 2 | i41SL1-2 | bd2 | ⓞ | 15 | HpU10 | 19-25 | • | 28 | ECL2B-3 | 21-4 | |
| 3 | MA1 | ba9 | | 16 | HpU17 | cr1 | × | 29 | ECL2B-4 | cr1 | ⓞ |
| 4 | MA2 | aa4 | | 17 | HpU18 | cr1 | ⓞ | 30 | ECL2B-5 | cr1 | ⓞ |
| 5 | MA3²⁾ | cr1 | ○ | 18 | HpU19 | bb1 | × | 31 | ECL2B-6 | 21-4 | |
| 6 | MA5 | cr1 | ⓞ | 19 | HpU20 | cr1 | ⓞ | 32 | UA1 | 21-12 | ● |
| 7 | MA8 | ba9 | | 20 | 7C4 | af4 | | 33 | UA7 | 8-30 | |
| 8 | MA12 | ba9 | | 21 | NU24 | hf24 | ● | 34 | UA10 | bb1 | ⓞ |
| 9 | MA14 | cr1 | ⓞ | 22 | 2C12 | 21-12 | ● | 35 | UA15 | 21-12 | ● |
| 10 | MA15 | ba9 | | 23 | 2H5 | 12-46 | | 36 | ECL2A-1 | gj38c ? | |
| 11 | iMO3 | aa4 | | 24 | i41-3 | ce9 | | 37 | ECL2A-19 | bd2 | ⓞ |
| 12 | HpU1 | bd2 | | 25 | i41-7 | 19-25 | ● | | | | |
| 13 | HpU2 | km4 | | 26 | ECL2B-1 | cr1 | | | | | |
| Germlines capable of forming a catalytic triad residue structure | | | | | | | | | | | |
| ⓞ: Triad due to D1-S27a-H93 | | | | | | | | | | | |
| ○: Triad due to D1-S26-H93 | | | | | | | | | | | |
| ●: Triad due to combinations other than "D1-S26 or S27a-H93". H90 or H91 is used. | | | | | | | | | | | |
| x: Triad is lost owing to mutation | | | | | | | | | | | |
| No.36 ECL2A-1 is low in the homology with germlines. | | | | | | | | | | | |
| Catalytic triad residue like structure derived from germlines | | | | | | | | | | | |
| Germlines supposed to be able to form with a layout same as 12-41: 12-44, 12-46 | | | | | | | | | | | |
| Germline supposed to be able to form with a layout same as 19-17: 19-25 | | | | | | | | | | | |
| Germlines supposed to be able to form with a layout same as 21-12: 21-5, 21-10 | | | | | | | | | | | |

| | | | | | | | | | | | |
|---|---|---|---|---|---|---|---|---|---|---|---|
| *: Triad newly obtained owing to mutation | | | | | | | | | | | |

In Tables 1-1, 1-2, 2 and 4, type ⓞ represents ones that constitute a catalytic triad residue structure from Asp1, Ser27A and His93, type ○ represents one that constitutes a catalytic triad residue structure from Asp1, Ser26 and His93, type ● represents ones that constitute a catalytic triad residue structure from combinations other than "Asp1, Ser27A or Ser26 and His93", type * represents one that cannot form a catalytic triad residue structure because of mutation from the germline, and type × represents ones that form a catalytic triad residue structure by including an amino acid mutated from the germlines.

As the clones derived from germlines capable of forming a catalytic triad residue structure that has a His at a tip end portion of a complimentarity determining region as discussed above, 21 clones were found. Furthermore, in two clones (Nos.16 and 18), a catalytic triad residue structure having a His could not be formed.

That is, since in many of antibody subunits (light chain, heavy chain) having the catalytic triad residue structure, the peptitase activity and/or protease activity was recognized, the germlines of the antibodies were investigated. As a result, there were found common points in that the CDR1 is constituted of 16 amino-acid residues and a histidine residue is at the 93^{rd} (or in the neighborhood thereof) by Kabat numbering scheme (type ④). This is an important finding suggesting that in particular germlines an antibody L chain having the enzymatic activity is already prepared.

Furthermore, among antibodies having a catalytic triad residue structure, the enzymatic activity was experimentally investigated of clones 41S-2 (germline: hf24/already disclosed) and i41SL1-2 (bd2). Clones other than the above are now under experiment and a heavy chain of HpU-2 antibody, a light chain of HpU-9 antibody, a light chain of HpU-18 antibody and light and heavy chains of i41-7 were revealed to have the peptitase activity and protease activity.

Still furthermore, the i41-7 antibody belonged to a germline 19-25 of which CDR-1 has 11 amino-acid residues. Then, all germlines of which CDR-1 has 11 amino-acid residues were searched, and, other than 19-25, in 19-14, 19-17, 19-23 and 12-41 (these are shown in a box in Table 3), the catalytic triad residue structure was recognized. When these were investigated in more detail, there were found common points in that the CDR-1 is constituted of 11 amino-acid residues and a histidine residue is present at the 91^{st} (or the 55^{th}) by Kabat numbering scheme (type ④*).

When the above findings are summarized, what follows can be said. ① Natural antibody enzymes are considered present, roughly divided, in two types. One is a type that has, already in a germline stage, sequences advantageous in constituting a catalytic triad residue structure, and, according to Thiebe et al. convention, at least 13 types of bb1, cr1, bl1, cs1, bd2, bj2, hf24, 19-25, 19-14, 19-17, 19-23, 12-41 and 21-12 conform thereto. In the case of hf24, there is likelihood also in that, other than His 91 present in the germline, His 53 appears owing to the mutation, and this constitutes a catalytic triad residue structure. ② There are only 13 kinds (13.9% of 93 kinds in total) that have sequences advantageous for constituting a catalytic triad residue structure according to Thiebe et al. convention.

However, in substantially 20% in search due to the PDB and in substantially 30% in monoclonal antibodies that the inventors have, ones that can be a catalytic triad residue structure are found. That is, at probability beyond expectation, the germlines are recruited.

Furthermore, as conclusions, when a stereostructure is estimated from base sequences of an antibody and an antibody having a germline shown in a box in Table 3 is selected, as to the light chains, the antibody enzyme can be efficiently obtained at rather high probability (conclusion ①).

Still furthermore, when the consideration of the light chain is applied to heavy chains, up to now, heavy chains of the 41S-2, i41SL1-2 and i41-7 antibodies are recognized to constitute the catalytic triad residue structure and have the enzymatic activity. Families of heavy chains of the 41S-2, i41SL1-2 and i41-7 antibodies, respectively, are VH10, VH1 and VH1. On the other hand, in heavy chains of 7C4 and HpU-2 antibodies, there is found no catalytic triad residue structure due to His, Asp and Ser; however, Glu, Asp and Ser constitute a catalytic triad residue structure and the enzymatic activity was recognized. Families of heavy chains of 7C4 and HpU-2 antibodies, respectively, are VH5 and VH1. In particular, both (heavy chains of 7C4 and HpU-2 antibodies) constitute a catalytic triad residue structure from the utterly same combination (Ser84, Clu85 and Asp86). That is, as to the heavy chain, VH1, VH5 and VH10 can be said families that can efficiently exhibit the enzymatic activity as an antibody enzyme (conclusion ②).

Still furthermore, it can be said from the present invention that even in an antibody that inherently has not a catalytic triad residue structure, when a catalytic triad residue structure corresponding to a germline or a Family is introduced according to a genetic engineering method, antibody enzymes can be efficiently obtained (conclusion ③).

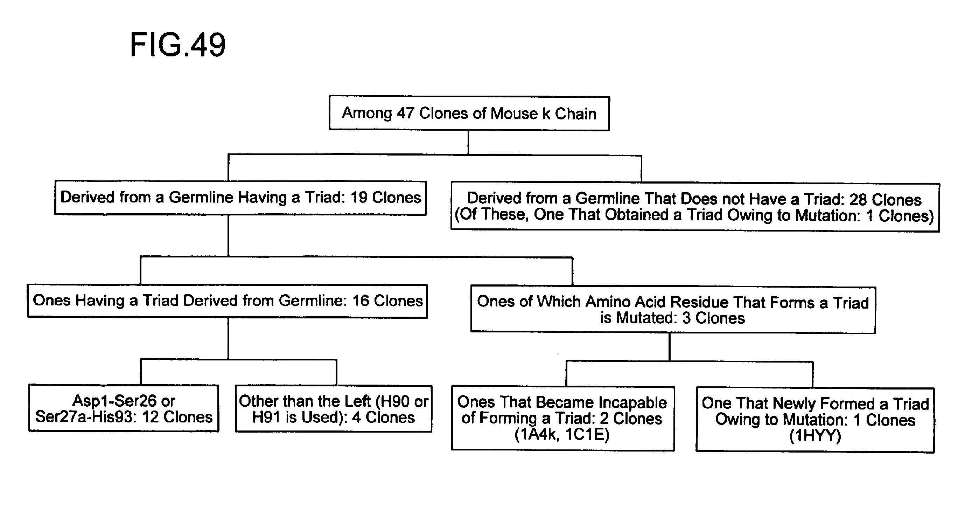

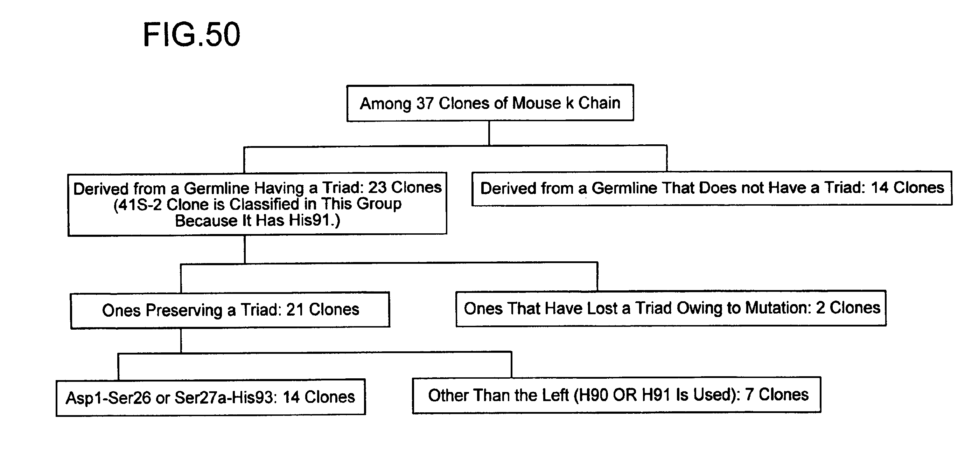

Furthermore, as shown in Fig.49, among 47 kinds of antibody light chains (κ type) of the PDB data, 18 kinds had a catalytic triad residue structure having a His (38.3%) . Still furthermore, as shown in Fig.50, among data of 37 kinds of antibody light chains (κ type) that the inventors have, 20 kinds had a catalytic triad residue structure having a His (50.4%). When these are summed up, among the antibody light chains found from the stereostructures, a detection rate of the antibody light chains having the enzymatic activity is 45.2%. This is general probability capable of detecting antibody light chains.

However, when the germlines are specified as mentioned above, antibodies having a catalytic triad residue structure having a His that is considered to have the enzymatic activity are found at 88.1% (PBD date; 16/19 = 84.2% (Fig.49), data relating to clones that the inventors have; 21/23 = 91.3% (Fig.50), and in total; (16 + 21)/(19 + 23) = 37/42 = 88.1%) ; that is, the probability approaches 100%. A reason for 100% being not attained is that one that inherently has a catalytic triad residue structure undergoes the mutation in the course of ripening of the antibody to lose a His to result in losing the catalytic triad residue structure. One kind of the antibodies due to the PDB data and two kinds of antibodies that the inventors possess underwent the mutation. That is, when the germline is specified, substantially completely antibodies that have the enzymatic activity can be obtained (conclusion ④).

### [Example 1-4: Preparation of light and heavy chains

Ultrafiltration was applied repeatedly to purified antibodies to remove low molecular weight protease inhibitors. Subsequently, a reducing reaction (15 degrees centigrade and 3 hr) with 0.2 M β-mercaptoethanol and an alkylation reaction (15 degrees centigrade and 15 min) with 0.3 M iodoacetamide were applied to separate heavy chains and light chains. After these were subjected to ultrafiltration to concentrate, with a Protein-Pak 300 column (Manufactured by Millipore Corporation Waters Chromatography Division), HPLC purification was applied. In a moving phase, a 6 M guanidine solution (pH 6.5) was used, and a flow rate was set at 0.15 mL/min.

Partitioned solutions of heavy and light chains were diluted with a 6 M guanidine solution to prepare, followed by dialyzing against a PBS solution (137 mM NaCl, 2.7 mM KCl, 1.5 mM KH₂PO₄, 8.0 mM Na₂HPO₄ and pH 7.3) to refold, further followed by buffer changing according to experimental conditions.

### [Example 1-5: Measurement of activity of antibody enzyme]

In order to clarify relationship between the catalytic triad residue structure and the enzymatic activity, with i41SL1-2 antibody light chains (i41SL1-2-L derived from germline bd2) and heavy chains thereof (i41SL1-2-H), and i41-7 antibody light chains (i41-7-L derived from germline 19-25) and heavy chains thereof (i41-7-H) that are all estimated to have the catalytic triad residue structure, a reaction of decomposing a peptide substrate was carried out.

Specifically, to each of heavy and light chains of an i41SL1-2 antibody, a CDR1 antigen peptide (RSSKSLLYSNGNTYLY) shown in sequence No.9 was reacted.

The reaction was carried out under the conditions; 5% DMSO, 9 mM HEPES, 7.5 mM phosphate buffer (pH 7.1) and 25 degrees centigrade. Experiments were carried out at concentrations of 0.4 µM, 0.2 µM and 50 µM, respectively, for i41SL1-2-L, i41SL1-2-H and an antigen peptide.

Results are shown in Figs.6A and 6B. (●) shows results when i41SL1-2-L and the antigen peptide were reacted, (○) shows results when only antigen peptide was reacted, and (▲) shows results when i41SL1-2-H and an antigen peptide were reacted. As shown in Figs.6A and 6B, for both the heavy and light chains, with reaction time, concentrations of the peptide go down, finally resulting in being incapable of detecting. Then, when a peak portion newly detected on a HPLC chromatogram with time was partitioned and subjected to mass spectroscopy, it was found to be a fragment on a C terminal side cleaved between an arginine residue at the 1^{st} and a serine residue at the 2^{nd} of the antigen peptide. From this, it became obvious that the i41SL1-2-L and i41S1-2-H decompose an antigen peptide.

Furthermore, to the light and heavy chains of the i41-7 antibody, three kinds of synthesized peptides shown in sequence Nos.10, 11 and 12 were reacted. The i41-7 antibody was obtained with a protein as an antigen and an epitope has not yet been determined. Under normal conditions, the enzymatic activity has to be investigated with a peptide corresponding to an epitope as a substrate. However, antibody enzymes that inventors have obtained so far exhibit high specificity to a protein but are low in the specificity to a short peptide. In this connection, in the present experiment, three kinds of synthesized peptides having molecular weight from several hundreds Da to 2.3 kDa were used as substrate.

Reactions were carried out under conditions of 15 mM phosphate buffer (pH 6.5) and 25 degrees centigrade. Synthesized peptides shown in sequence Nos.10, 11 and 12 were used at a concentration of 120 µM, i41-7-L at 0.8 µM and i41-7-H at 0.4 µM.

Results thereof are shown in Fig.10A. (●) shows results when peptide TP41-1 shown in sequence No.10 and i41SL1-7-L or i41-7-H were reacted, (○) shows results when only peptide TP41-1 shown in sequence No.10 was reacted, (▲) shows results when peptide shown in sequence No.11 and i41-7-L or i41-7-H were reacted, (Δ) shows results when only peptide shown in sequence No.11 was reacted, (■) shows result when peptide shown in sequence No.12 and i41SL1-7-L or i41-7-H were reacted, and (□) shows results when only peptide shown in sequence No.12 was reacted.

As shown in Fig.10A, i41-7-L and i41-7-H decomposed all the peptides.

Furthermore, a decomposition reaction of an antigen protein due to a light chain of i41-7 antibody (i41-7-L) was analyzed by means of SDS-PAGE. One that was used as a substrate was a light chain of an antibody 41S-2 (41S-2-L). The 41S-2-L that was used as a substrate was incomplete in the refolding and did not have the activity as an antibody enzyme.

Decomposition reaction were carried out under the conditions same as that mentioned above, 41S-2-L was used at a concentration of 2 µM, and a light chain of the i41-7 antibody was used at a concentration of 0.8 µM. Results thereof are shown in Fig.10B. A lane M shows a result when a protein molecular weight marker is subjected to electrophoresis and lanes 1 through 5 show results when reaction liquids with i41-7-L and 41S-2-L at 0, 2, 4, 6 and 8 days after the start of the reaction were subjected to the electrophoresis. Bands at a position of 25.0 kDa show antigen protein 41S-2-L and bands at a position of 22.5 kDa show a decomposition product of 41S-2-L. As controls, a result when a light chain of i41-7 alone was reacted and a result when 41S-2-L alone was reacted are shown. On the basis of results due to the SDS-PAGE, the decomposition reaction of the 41S-2-L due to the i41-7-L is graphed and shown together.

From results of SDS-PAGE and a graph in the Fig.10B, it is understood that a band at a position of 25.0 kDa is decomposed to generate a band at a position of 22.5 kDa. Furthermore, from the controls, in the reaction of the 41S-2-L that is a substrate alone, a band at a position of 25.0 kDa is not decomposed. From this, it is demonstrated that the light chain of the i41-7 antibody decomposes the antigen protein 41S-2-L.

In the next place, of an i41-7 intact antibody, a light chain (i41-7-L), a heavy chain (i41-7-H) and a MA-2 antibody, the polypeptide decomposition property was investigated. The i41-7 intact antibody is a complete antibody made of heavy and light chains. The MA-2 antibody (germline: aq4) is a monoclonal antibody against methamphetamine and an antibody that does not have a catalytic triad residue structure in the stereostructure.

The reaction conditions were same as that in the above experiment and, as a substrate, polypeptide TP41-1 (described as "TP" in Fig.12) shown in sequence No.10 was used at a concentration of 120 µM. The i41-7 intact antibody, i41-7-L, and MA-2 antibody were used at a concentration of 0.8 µM and the i41-7-H was used at a concentration of 0.4 µM.

Results are shown in Fig.12. (●) shows results when i41-7-L and TP41-1 were reacted, (□) shows results when i41-7-H and TP41-1 were reacted, (Δ) shows results when i41-7 intact antibody and TP41-1 were reacted, (■) shows results when MA-2 antibody and TP41-1 were reacted, and (○) shows results when only TP41-1 as the control was reacted.

As shown in Fig.12, it was found that both the heavy and light chains of i41-7 antibody have, for a while after the start of the reaction, an induction period during which the peptide is not decomposed, followed by an active period during which the peptide is decomposed. Furthermore, though results are not shown in the drawing, when, after the peptide was completely decomposed, peptide was added again, both the heavy and light chains of i41-7 antibody decomposed the peptide without the induction period. From the results, it is considered that both the heavy and light chains of i41-7 antibody, during transferring from the induction period to the active period, undergo a conformation change. Furthermore, it is considered that after the conformation change, the active type is maintained thereafter.

The intact i41-7 antibody that has both the heavy and light chains did not decompose the polypeptide. This is considered that, in the i41-7 intact antibody, owing to an S-S bond or a non-covalent bond, the light chain and the heavy chain are strongly bonded; accordingly, it cannot undergo the conformation change into a mode that has the activity even when it is brought into contact with the polypeptide.

Furthermore, in the MA-2 antibody that does not have the catalytic triad residue structure in its stereostructure, both heavy and light chains thereof did not decompose the polypeptide. Although results are not shown in the drawing, none of a MA-15 antibody light chain (germline: ba9), a 7C4 antibody light chain (germline: af4), a HpU-9 antibody heavy chain and a HpU-18 antibody heavy chain decomposed the polypeptide and the antigen protein.

From the results, it was demonstrated that an antibody enzyme that has a catalytic triad residue structure in a stereostructure has the activity of cleaving and/or degrading the polypeptide and the antigen protein.

### [Example 1-6: Kinetic analysis decomposition reaction of i41SL1-2-L and i41SL-2-H against antigen peptide

When profiles of decomposition reactions of i41SL1-2-L and i41SL-2-H against a CDR1 antigen peptide were traced in detail, in an initial period of reaction with the antigen peptide, an "induction period" during which a concentration of the peptide hardly changed (peptide is hardly degraded) was recognized (length thereof varies depending on the experimental conditions). However, when the antigen peptide was once more added to an antibody solution that is in an active state after the peptide was once decomposed, this time, the induction period was not recognized. In this connection, a decomposition initial speed in an antigen peptide re-addition reaction was measured and analyzed according to a Michaelis-Menten equation.

As a result, as shown in Figs.7A and 7B, both the heavy and light chains exhibited high correlation in the Hanes-Woolf plots. Accordingly, these can be said an enzyme reaction that follow the Michaelis-Menten equation. Kinetic parameters obtained here are shown in Table 5 comparing with that of trypsin (values obtained with a different synthesized peptide as a substrate).

**[Table 5]**

| | Km (M⁻¹) | kcat (min⁻¹) | kcat/Km (M⁻¹min⁻¹) |
|---|---|---|---|
| i41SL1-2-L | 1.98 × 10⁻⁶ | 6.13 × 10⁻¹ | 3.10 × 10⁵ |
| i41SL1-2-H | 1.27 × 10⁻⁵ | 6.18 × 10⁻¹ | 4.87 × 10⁴ |
| trypsin | 3.49 × 10⁻⁴ | 1.08 × 10² | 3.09 × 10⁵ |

As shown in the Table 5, a catalyst efficiency (kcat/Km) of the light chain is identical as that of trypsin and that of the heavy chain is also such high as substantially one tenth that of trypsin; accordingly, it can be said that both the heavy and light chains of the antibody enzyme have high enzymatic activity comparable with that of natural enzymes. However, when the respective parameters are compared, in the affinity (Km) with the substrate, both the heavy and the light chains of the antibody enzyme are stronger than that of tripsin; on the other hand, in the number of rotation (kcat), both the heavy and light chains are substantially one two-hundredth that of trypsin, there is found a large difference between natures thereof. This result shows that i41AS1-2-L and i41SL1-2-H, with the nature as an antibody that it has high affinity with a substrate remaining, exhibit the enzymatic activity of completely decomposing the polypeptide.

### [Example 2]

An example relating to the embodiment 2 according to the invention will be explained below.

### [Experiment 2-1] (Purification of HP urease)

HP bacteria were cultured in a 7% FBS-containing brucella liquid medium under a nonaerobic atmosphere at 37 degrees centigrade. The cultured HP bacteria were centrifuged to recover, followed by washing with 0.15 M NaCl, further followed by suspending the bacteria in distilled water, still further followed by rigorously agitating, and thereby a bacteria component was extracted. Then, a soluble component after centrifugation and dialysis was made a distilled water extract (DWExtract). This was subjected to anion exchange chromatography that uses a Q Sepharose Fast Flow, followed by gel filtration with Superose 6, and further followed by subjecting to anion exchange chromatography. This was further subjected to gel filtration and the HP urease was confirmed to be highly pure by use of the SDS-PAGE.

### [Experiment 2-2] (Preparation of antibody producing-cell)

With high purity HP urease purified in experiment 2-1 as an antigen, a spleen cell was fused with NS-1 myeloma and polyethylene glycol, after HAT selection, followed by screening and cloning, and thereby three kinds of anti-HP urease mouse monoclonal antibody producing-cells, that is, HpU-2, HpU-9 and HpU-18 were prepared.

### [Experiment 2-3] (Preparation of H and L chains)

To each of the respective purified antibodies (HpU-2, HpU-9 and HpU-19) obtained from the respective antibody producing-cells, ultrafiltration was repeated to remove low molecular weight protease inhibitors. Subsequently, a reducing reaction (15 degrees centigrade and 3 hr) with 0.2 M β-mercaptoethanol and an alkylation reaction (15 degrees centigrade and 15 min) with 0.3 M iodoacetamide were applied to separate the H chain and the L chain. This was subjected to ultrafiltration to concentrate, followed by HPLC purification by use of a Protein-Pak 300 column (manufactured by Millipore Corporation Waters Chromatography Division). In a moving phase, a 6 M guanidine solution (pH 6.5) was used and a flow rate was set at 0.15 ml/min.

Fractionated solutions of the H and the L chains were diluted with a 6 M guanidine solution, followed by dialyzing against PBS to refold, further followed by buffer exchanging in accordance with the experimental conditions.

### [Experiment 2-4] (Cultivation of antibody producing-cell)

Cells that produce three anti-Helicobacter Pylori monoclonal antibodies (HpU-2, HpU-9 and HpU-18) prepared in experiment 2-2 were recovered from frozen stocks. The respective antibody producing-cells were cultivated in an IMDM medium containing 20% cow fetus serum. Under the conditions of a temperature of 37 degrees centigrade and a concentration of carbon dioxide of 5.5%, passage culture was repeated until the number of cells became 5 × 10⁷. The cells were recovered by centrifuging.

### [Experiment 2-5] (Extraction of mRNA from HpU antibody producing-cell)

According to a protocol of quickPrep™ mRNA Purification Kit manufactured by Pharmacia Biotec, an experiment was carried out. To the recovered respective antibody producing-cells, Extraction Buffer was added to homogenize, followed by centrifugation. A supernatant liquid was added to Oligo (dT)-cellulose Spun Column, followed by thoroughly agitating. Slight centrifugation was applied to precipitate gel followed by removing a solution. A washing operation was repeated three times, followed by adding Elution Buffer at the end of the washing, and thereby an eluted solution was recovered. The absorbance at 260 nm was measured of the eluted solution to quantitatively determine mRNA, followed by precipitating with alcohol, further followed by storing at -80 degrees centigrade.

### [Experiment 2-6] (Synthesis of cDNA from mRNA)

According to a protocol of AMW Reverse Trascriptase First-Strand cDNA Synthesis Kit (manufactured by LIFE SCIENCE, INC.), an experiment was carried out. Each mRNA extracted from the respective antibody producing-cells was recovered by applying centrifugation and dissolved in Rnase-free water. Thereto, pd(T)₁₂₋₁₈Primer was added followed by treating at 70 degrees centigrade for 10 min. Thereafter, dithiothreitol and Rnase Inhibitor, AMW Reverse Trascriptase were added and blended, followed by reacting at 41 degrees centigrade for 40 min. Thereby, from the mRNAs of the respective antibody producing-cells, the respective cDNAs were synthesized.

### [Experiment 2-7] (Amplification of antibody gene due to PCR)

With the synthesized cDNA as a template, a primer that complimentarily couples with 5' and 3' sides of variable regions of antibody H and L chains contained in Mouse Ig-Prime Kit (NOVEGEN), dNTP Mix and AmpliTaq DNA polymerase (Roche) were blended and thoroughly agitated. After incubation at 95 degrees centigrade for 10 min, a reaction cycle of 94 degrees centigrade and 1 min, 50 degrees centigrade and 1 min and 72 degrees centigrade and 2 min was repeated 40 times. Amplified products were confirmed by use of agarose gel electrophoresis.

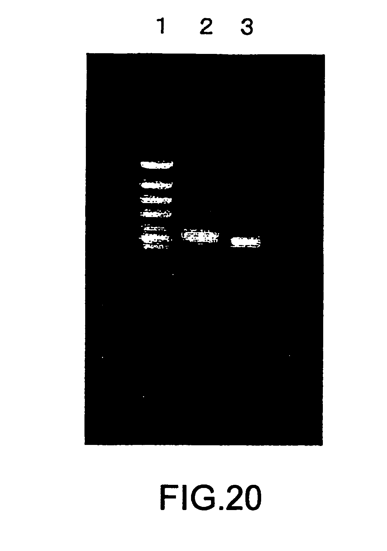

As to the HpU-2, as the results thereof are shown in Fig.20, at estimated positions (at substantially 500 bp in the HpU-2 H chain and at substantially 450 bp in the HpU-2 L chain), strong bands of DNA could be confirmed. In Fig.20, in lane 1, lane 2 and lane 3, respectively, a marker, an H chain and an L chain were electrophoresed. Furthermore, though not shown in the drawing, in the HpU-9 H chain, the HpU-9 L chain, the HpU-18 H chain and the HpU-18, respectively, at substantially 500 bp, substantially 450 bp, substantially 500 bp, and substantially 450 bp, strong bands of DNA were confirmed. The respective DNA fragments were cleaved to purify.

### [Experiment 2-8] (Cloning of purified respective DNA fragments)

A ligation reaction was carried out to the purified fragment and pGEM-T vector (Promega) with T4 DNA polymerase. It was incubated at room temperature for 1 hr, followed by blending with a competent cell JM109 and leaving on ice for 20 min. Thereafter, it was heated at 42 degrees centigrade for 45 sec, left on ice for 2 min, followed by adding a medium, further followed by cultivating at 37 degrees centigrade for 1 hr. On an agar plate coated with IPTG/X-gal, the cultivated bacteria solution was spread and incubated at 37 degrees centigrade overnight. Formed white colonies were optionally selected, followed by liquid culturing at 37 degrees centigrade overnight. According to the alkali-SDS method, plasmid was purified from a fungus body, followed by confirming whether a target fragment was inserted or not.

### [Experiment 2-9] (Determination of base sequence and amino acid sequence of cloned HpU antibody)

According to a protocol of Thermo Sequenase II dye terminator cycle sequencing kit (manufactured by Amersham Pharmacia), an experiment was carried out. To the purified plasmid, a primer and dNTP, the respective ddNTP, and cycle sequencing heat resistant DNA polymerase were blended, followed by lightly agitating. After incubation at 94 degrees centigrade for 1 min, a reaction cycle of 94 degrees centigrade for 1 min, 60 degrees centigrade for 1 min and 72 degrees centigrade for 2 min was repeated 40 times. After the reaction terminated, ethanol precipitation was carried out, and finally DNA was dissolved in a stop solution and subjected to polyacrylamide gel electrophoresis to read a base sequence. Of the variable regions of the HpU-18 (L chain), HpU-9 (L chain) and HpU-2 (H chain), respectively, results obtained by reading base sequences and results obtained by transforming the base sequences into amino acid sequences are shown in Figs.13, 15 and 17. In all sequences, upper case characters and lower case characters, respectively, show an amino acid sequence and a base sequence. Though not shown in the drawing, in variable regions of the HpU-18 (H chain), HpU-9 (H chain) and HpU-2 (L chain) as well, the base sequences and amino acid sequences were determined.

### [Experiment 2-10] (Estimation of three-dimensional structure from determined amino acid sequences)

The amino acid sequences of the H and L chains of the HpU-2, HpU-9 and HpU-18 were inputted in an antibody variable region stereostructure estimation program abM (manufactured by Oxford Molecular Corp.) and stereostructures were constituted. Subsequently, an energy was minimized with Insight II (Discover) (manufactured by MSI Corp.), and thereby a stereostructure of each of the variable regions of the HpU-2, HpU-9 and HpU-18 was obtained.

As a result, a stereo structure of the variable region of the HpU-18 (L chain) estimated by the molecular modeling of the three-dimensional structure, that of the variable region of the HpU-9 (L chain) and that of the variable region of the HpU-2 (H chain), respectively, are schematically shown in Figs.14, 16 and 18. In the abovementioned three antibody fragments each, a catalytic triad residue structure was recognized. On the other hand, though not shown in the drawing, in the HpU-18 (H chain), HpU-9 (H chain), and HpU-2 (L chain), the catalytic triad residue structure was not recognized.

### [Experiment 2-11] (Enzymatic activity test due to HpU antibody)

The H chain of HpU-2 antibody, the H or L chain of HpU-9 and HpU-18 antibodies and HpU2EPI (amino acid sequence: ¹⁸³SVELDIGGNRRIFGNALVD²⁰⁴ (sequence No.25)) that is a partial peptide of the HP urease were reacted in a 15 mM phosphate buffer (PB: pH 6.5). The HpU2EPI is a peptide corresponding to a region from the 183^{rd} to the 204^{th} in an α unit primary structure of a HP urease protein made of 238 amino acids. The operation was carried out in a clean bench. Purified peptide powder was dissolved with 15 mM PB (pH6.5), followed by filtering and sterilizing with Ultrafree MC (0.22 µm, manufactured by Millipore Corp.). Furthermore, each of L or H chain solutions of the HpU-2, HpU-9 and HpU-18 antibodies were blended so that a concentration in a reaction solution was 0.4 µM (20 µg/ml) for the H chain, 0.8 µM (20 µg/ml) for the L chain and 80 µM (184.4 µg/ml) for peptide. A reaction temperature was 25 degrees centigrade and for a reaction vessel a dry hot air sterilized small test tube was used. A reaction solution was analyzed with HPLC and peptide variation with time was traced. Analysis conditions at this time were flow rate: 0.5 ml/min, wavelength: 214 nm, moving phase: 0.1% TFA · 13% acetonitrile · ultrapure water. Fractionation of the reaction solution was carried out in a clean bench, a sample was taken in ultrafree C3 (0.5 µm, manufactured by Millipore Corp.), followed by centrifuging at 10,000 rpm for substantially 1 min, further followed by filtering, still further followed by injecting in the HPLC. Furthermore, as a control, an H chain alone, an L chain alone and a peptide alone were prepared at 15 mMPB (pH 6.5).

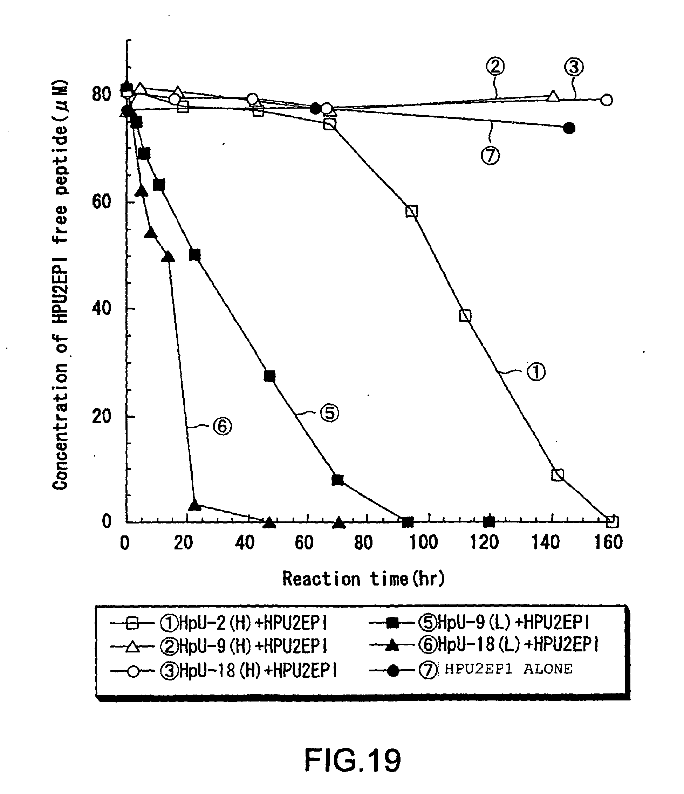

Results thereof are shown in Fig.19. In a graph of Fig.19, a horizontal axis expresses a reaction time (hr), and a vertical axis expresses a concentration (µM) of a peptide (HPU2EPL) that is a substrate. Furthermore, ① shows a case where HpU-2 (H) and the peptide were blended, ② shows a case where HpU-9 (H) and the peptide were blended, ③ shows a case where HpU-18 (H) and the peptide were blended, ⑤ shows a case where HpU-9 (L) and the peptide were blended, ⑥ shows a case where HpU-18 (L) and the peptide were blended, and ⑦ shows a case where the peptide only was used. As shown in Fig.19, when the HpU-18 (L) was blended with the peptide, at substantially 20 hr after the start of the reaction, a concentration of the peptide became substantially zero, that is, the peptide was almost completely decomposed. In the case of the HpU-9 (L), the peptide was completely decomposed after substantially 90 hr. In the case of the HpU-2 (H), during an initial stage of the reaction, an induction period during which the concentration of the peptide hardly changes appeared, through an active period thereafter, after substantially 160 hr, the peptide was completely decomposed.

Ones that exhibited the enzymatic activity against the peptide in the present experiment 2-11 (HpU-2 (H), HpU-9 (L) and HpU-18 (L)) were estimated to have the catalytic triad residue structure in the three-dimensional structure molecular modeling of experiment 2-10. On the other hand, ones that did not exhibit the enzymatic activity in the present experiment 2-11 (HpU-18 (H) and HpU-9 (L)) were estimated in the experiment 2-10 that these could not constitute the catalytic triad residue structure. From this, it is suggested that in an antibody that has a catalytic triad residue structure the enzymatic activity can be recognized.

### [Experiment 2-12] (Preparation of H chain and L chain of HpU-20 and UA-15 antibodies)

Similarly to the experiments 2-1 and 2-2, two kinds of anti-HP urease mouse monoclonal antibody producing-cells, HpU-20 and UA-15, were prepared. Then, according to a method similar to experiment 2-3, H and L chains of HpU-20 and UA-15 were prepared.

### [Experiment 2-13] (Sequence determination and molecular modeling of HpU-20 and UA-15 antibodies)

According to methods similar to that of Experiments 2-4 through 2-8, cDNAs of L and H chains of HpU-20 and L and H chains of UA-15 were cloned respectively. Then, according to a method similar to Experiment 2-9, of cDNAs of variable regions L and H chains of HpU-20 and L and H chains of UA-15, base sequences were determined, and therefrom amino acid sequences were determined as well. Further thereafter, according to a method similar to Experiment 2-10, of the L and H chains of HpU-20 and L and H chains of UA-15, the molecular modeling of the three-dimensional structure was applied to estimate the stereostructures.

A stereostructure estimated as a result of a variable region of HpU-20 (L chain) is schematically shown in Fig.21; a stereostructure of a variable region of HpU-20 (H chain), in Fig. 22; and stereostructures of variable regions of UA-15 (H and L chains), in Fig.30. From the stereostructure estimations, in the L and H chains of HpU-20 and the L chain of UA-15, the catalytic triad residue structure could be recognized. On the other hand, in the UA-15 (H chain), a catalytic triad residue structure could not be recognized.

### [Experiment 2-14-1] (Enzymatic activity test 1 with L and H chains of HpU-20)

The enzymatic activity tests of HpU-20-L and HpU-20-H against TP41-1 peptide (sequence No.42) were carried out. In the decomposition reaction, materials, conditions and procedures below were adopted.

### (Materials)

①: HpU-20 mAb (in 15 mM PB pH 6.5, one that was filtered and sterilized after purification and dialysis.)
②: HpU-20 antibody
   ②-1: Lot.1 [HpU-20-L] (in 15 mM PB pH 6.5, concentration 94.4 µg/ml)
   ②-2: Lot.1 [HpU-20-H] (in 15 mM PB pH 6.5, concentration 70.8 µg/ml)
   ②-3: Lot.2 [HpU-20-L] (in 15 mM PB pH 6.5, concentration 96.7 µg/ml)
   ②-4: Lot.2 [HpU-20-H] (in 15 mM PB pH 6.5, concentration 103.3 µg/ml)
③: Purified TP41-1 Peptide

### (Reaction liquid)

①: HpU-20-L: 0.8 µM (20 µg/ml)
②: HpU-20-H: 0.4 µM (20 µg/ml)
③: HpU-20 mAb: 0.8 µM (120 µg/ml)
④: TP41-1 Peptide: 120 µM (284 Mg/ml)

### (Reaction conditions)

Reaction temperature: 25 degrees centigrade
Small test tube, microchip, 15 mM PB (pH 6.5): sterilized
Experimental operation: in clean bench

### (Method)

(1): TP41-1 is prepared at 1.7 mg/ml with 15 mM PB pH 6.5, followed by sterilizing.
(2) : Subsequently, 1.7 mg TP41-1 of (1) is prepared at 568 µg/ml with 15 mM PB ph 6.5.
(3) : H and L chains of HpU-20 antibody, respectively, are prepared at 40 µg/ml with 15 mM PB ph 6.5.
(4): (2) and (3) are blended at a ratio of 1:1.
(5): A change with time of the peptide is traced by means of HPLC.

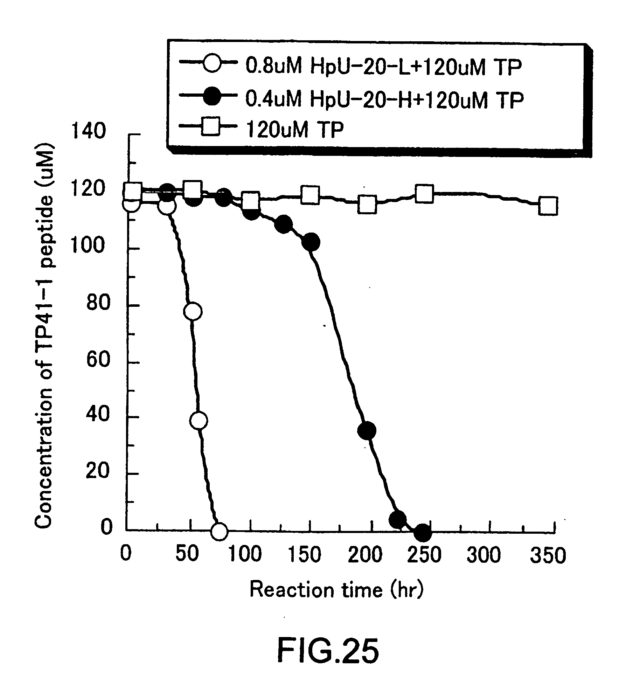

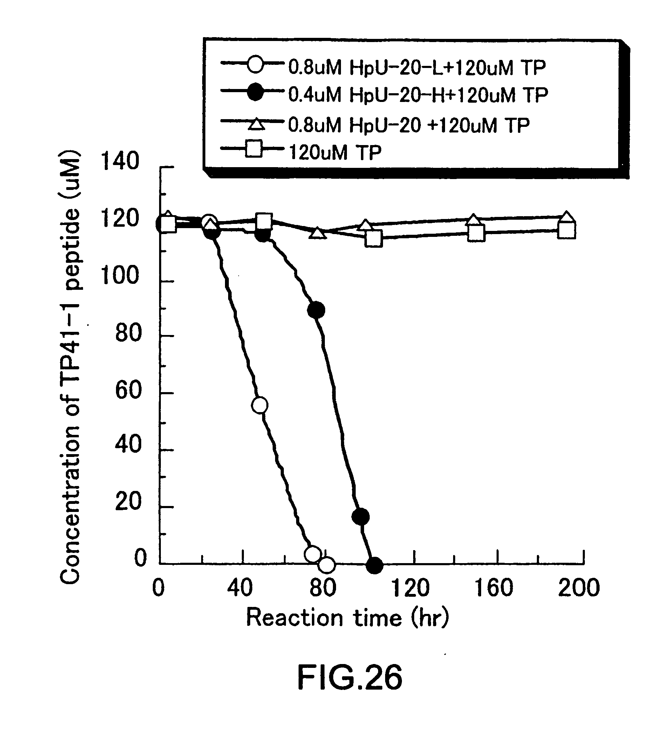

Experimental results thereof are shown in Fig.25 for Lot.1 and in Fig.26 for Lot.2. In graphs of Figs.25 and 26, a horizontal axis denotes a reaction time (hr) and a vertical axis denotes a concentration (µM) of the peptide that is a substrate.

As shown in graphs of Figs.25 and 26, in the prepared Lot.1 and Lot.2, with only a slight difference in the reactivity, both completely decomposed the TP41 peptide with excellent reproducibility. From the results, both the HpU-20-L and HpU-20-H were confirmed to have the enzymatic activity in the decomposition reaction of the peptide.

### [Experiment 2-14-2] (Enzymatic activity test 2 with L and H chains of HpU-20)

Subsequently, with activated L and H chains of HpU-20, a protein decomposition experiment was carried out. In the decomposition experiment, materials, compositions and kinds of reaction liquids, reaction conditions and processes below were adopted.

### (Materials)

①: Activated (once completely decomposed TP41 peptide) Lot.2 [HpU-20-L] (in 15 mM PB pH 6.5, 02. 10. 15 separation, 02. 10. 16 purification, 02. 10. 21 PBS dialysis, followed by buffer exchanging in 02. 10. 21 ~ 22 PB. Storage for 4.2 months, concentration 96.7 µg/ml)
②: HP bacteria urease (M.W=522600) 02. 07. 01 purification, concentration 1.84 mg/ml
③: BSA SIGUMA A-6003 Lot51K7600
④: HSA WAKO 019-10503 LotKSH6886

### (Composition of reaction liquid)

①: HpU-20-L: 0.4 µM (20 µg/ml)
②: HP bacteria urease: 52 nM (28 µg/ml)
③: BSA: 0.4 µM (28 µg/ml)
④: HSA: 0.4 µM (28 µg/ml)

### (Reaction conditions)

Reaction temperature: 25 degrees centigrade
Small test tube, microchip, 15 mM PB (pH 6.5): sterilized
Experimental operation: in clean bench

### (Method)

(1) HSA and BSA are prepared to 2.2 mg/ml with 15 mM PB pH 6.5, followed by filtering and sterilizing.
(2) Subsequently, 1.7 mg/ml TP41-1 of (1) is prepared to 55 µg/ml with 15 mM PB pH 6.5.
(3) 1.84 mg/ml of HP bacteria urease is prepared to 55 µg/ml with 15 mM PB pH 6.5.
(4) Activated HpU-20-L and (2) or (3) are blended at a ratio of 1: 1.
(5) Changes of an antibody and the respective proteins are tracked by means of the SDS-PAGE.

### (Kinds of reaction liquid)

①: 04 µM HpU-20-L + 0.4 µM BSA: 320 µl
②: 0.4 µM HpU-20-L + 0.4 µM HSA: 320 µl
③: 0.4 µM HpU-20-L + 52 nM HP bacteria urease: 320 µl
④: 0.4 µM HpU-20-L alone: 320 µl
⑤: 0.4 µM BSA alone: 320 µl
⑥: 0.4 µM HSA alone: 320 µl
⑦: 52 nM HP urease alone: 320 µl

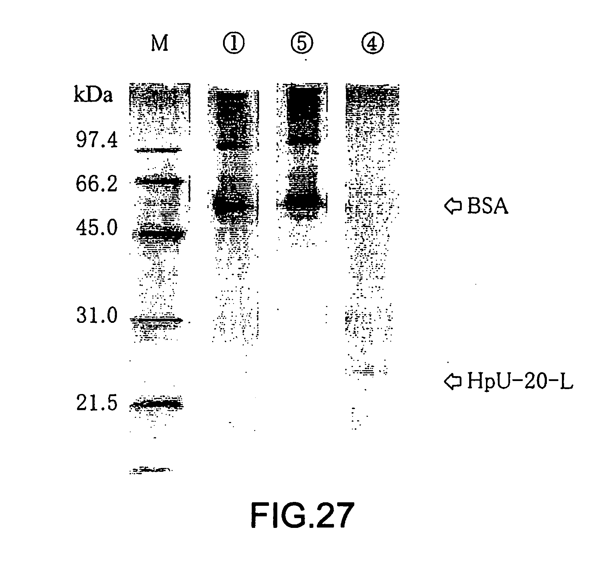

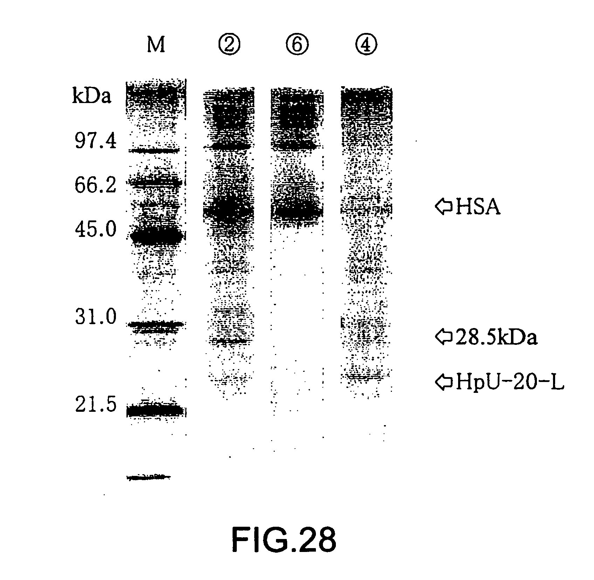

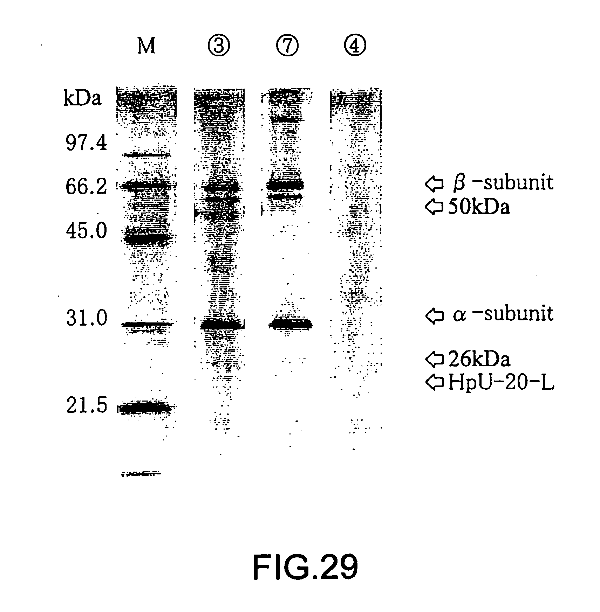

Results of the present experiments are shown in Figs. 27 through 29. Figs. 27 through 29 are diagrams showing results of the SDS-PAGE of samples at 1 hr after the start of the reaction. As to samples in the respective lanes, M denotes a marker and ① through ⑦ denote the abovementioned respective reaction liquids.

From the results shown in Figs.27 through 29, it is found that after substantially 1 hr of reaction the HpU-20-L does not at all decompose the BSA. As to the HSA, although a thin band is found at 28.5 kDa, the HSA as the control hardly exhibits a change; that is, it is found that the HpU-20-L causes only a slight decomposition of the HSA. On the other hand, against the HP bacteria urease, after a reaction of substantially 1 hr, a strong band at 50 kDa and a weak band at 26 kDa were observed. Furthermore, a band of a β-subunit was weakened by 26.7% compared with the control. A α-subunit band hardly exhibited a change before and after the reaction. From the results, it was revealed that the HpU-20-L specifically decomposes the β-subunit of the HP bacteria urease.

### [Experiment 2-15] (Enzymatic activity test with L and H chains of UA-15)

With UA-15-L and UA-15-H, an enzymatic activity test against TP41-1 peptide (sequence No.42) was conducted. The decomposition reactions were carried out with reaction liquids below and under reaction conditions below.

### (Reaction liquid)

①: Lot.1 [UA-15-L]: 0.8 µM (20 µg/ml)
②: Lot.1 [UA-15-H]: 0.4 µM (20 µg/ml)
③: Lot.2 [UA-15-L]: 0.8 µM (20 µg/ml)
④: Lot.2 [HpU-20-H]: 0.4 µM (20 µg/ml)
⑤: TP41-1 Peptide: 120 µM (284 µg/ml)

### (Reaction conditions)

Reaction temperature: 25 degrees centigrade
Small test tube, microchip, 15 mM PB (pH 6.5): sterilized
Experimental operation: in clean bench

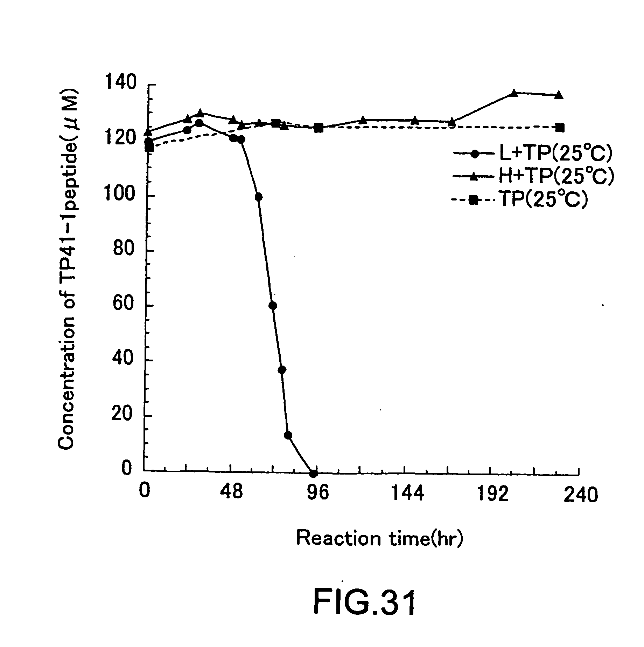

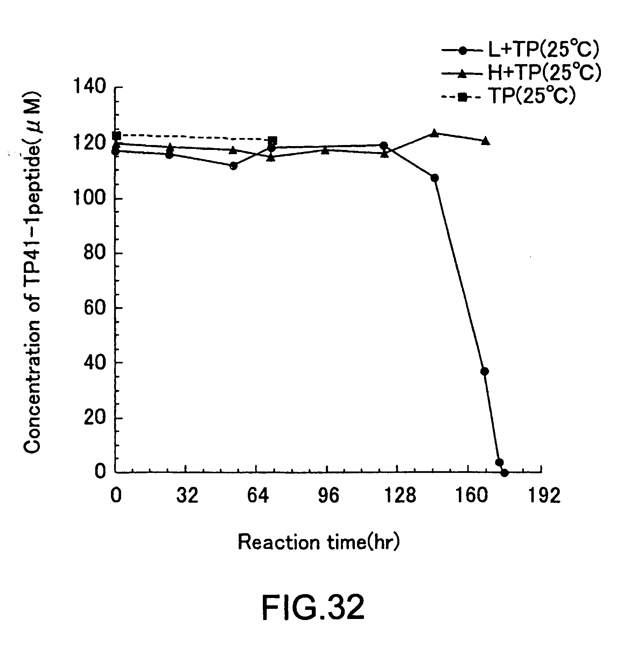

Experimental results thereof are shown in Fig.31 for Lot.1 and in Fig.32 for Lot. 2. In graphs of Figs.31 and 32 , a horizontal axis denotes a reaction time (hr) and a vertical axis denotes a concentration (µM) of the TP41 peptide that is a substrate. As obvious from graphs in Figs.31 and 32 as well, in the L chain of the UA-15, the enzymatic activity of decomposing the TP41-1 peptide was confirmed; however, in the H chain of the UA-15 the enzymatic activity was not confirmed.

Subsequently, with reaction liquids obtained by replenishing the peptide substrate (TP41-1) to a reaction liquid of UA-15-L in Lot.2, the peptide decomposition experiments were carried out at the respective temperatures of 15, 25 and 37 degrees centigrade.

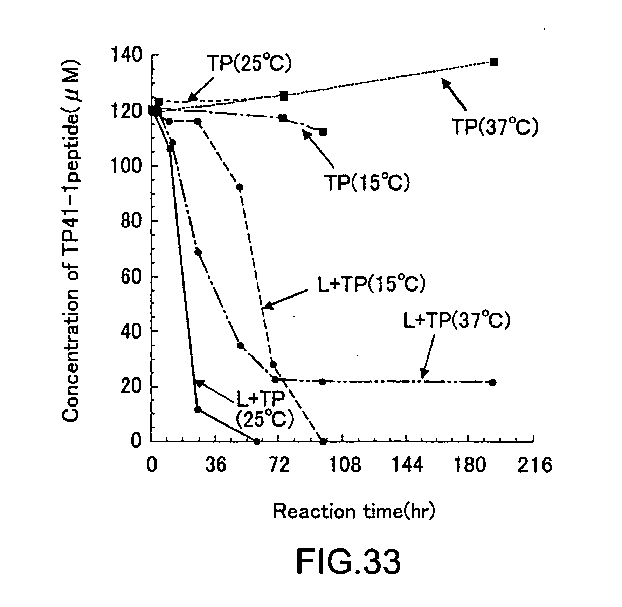

Results are shown in Fig.33. From the results, it was found that in the case of the reaction temperature being 25 degrees centigrade, the enzymatic activity of the UA-15-L was the highest.

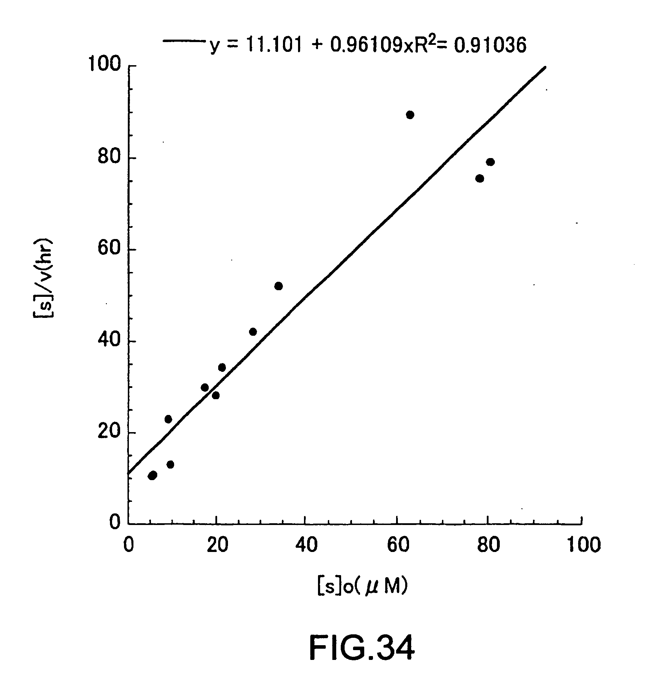

Furthermore, under the above reaction conditions, with Lot.1 [UA-15-L] in a reaction liquid, the TP41-1 peptide was once completely decomposed. Thereafter, to the solution, the TP41-1 peptide was added at the respective concentrations of 5, 10, 15, 20, 30, 65 and 80 µM, followed by applying kinetic analysis due to the UA-15-L. Results thereof are shown in Fig.34 and Table 6.

**[Table 6]**

| Hanes-Woolf plot | | |
|---|---|---|
| Km(M) | kcat (min⁻¹) | kcat/Km (/M·min) |
| 11.6 × 10⁻⁶ | 4.3 × 10⁻² | 3.8 × 10³ |

### [Example 3]

An example relating to embodiment 3 of the invention will be explained below.

### [Experiment 3-1] (Preparation of ECL2B-2 and ECL2B-3 antibodies)

Each of monoclonal antibodies ECL2B-2 and ECL2B-3 was prepared with a partial peptide (RSSHFPYSQYQFWKNFQTLK: sequence No.38) made of 20 amino acids that constitute an extracellular region of CCR-5 as an immunogen.

Firstly, to a C terminal of the partial peptide glycine and cystine residues were introduced to conjugate with Keyhole Limpet Hemocyanin (KLH). In an actual CCR-5 peptide, an N terminal of the partial peptide is a cystine residue; however, in a peptide that is used in the experiment, an arginine residue substituted for the cystine residue. The peptide and a conjugate with KLH of the peptide are ones that are manufactured by Takara Shuzo Co., Ltd.

The peptide conjugated with KLH was immunized in a Balb/c mouse. First and second immunizations were carried out by use of a complete immunoactivator due to Freund at an interval of two weeks. A third immunization was carried out by use of an incomplete immunoactivator due to Freund four weeks after the second immunization. A final booster (additional immunization) was carried out six weeks after the third immunization. On third day after the final booster, cell fusion of a spleen cell of a Balb/c mouse and a myeloma cell (SP/NSI/1-Ag4-1 (NS-1)) was conducted, followed by applying the HAT selection and screening. After the cloning was carried out twice, cell systems that excrete a monoclonal antibody (ECL2B-2 and ECL2B-3; IgG₁(k)) were established.

### [Experiment 3-2] (Purification and isolation of antibody subunit)

ECL2B-2 and ECL2B-3 were purified based on a purification manual of Bio-Rad Protein A MAPS-II kit (manufactured by Nippon Bio-RAD Corp.).

Firstly, abdominal dropsy containing ECL2B-2 and ECL2B-3 was blended with a same volume of an ammonium sulfate saturated aqueous solution. A precipitate thereof was centrifuged, followed by recovering, further followed by adding 5 ml of PBS to the precipitate. The process was repeated twice, followed by applying dialysis to PBS twice. A definite amount of PBS solution containing ECL2B-2 or ECL2B-3 was blended with a same volume of a conjugate buffer MAPS-II, followed by placing a mixture on a base packed with Affi-Gel (protein A), further followed by eluting a conjugated antibody. The eluted antibody was dialyzed twice with a 50 mM Tris/0.15 M NaCl (pH = 8.0) buffer solution (4 degrees centigrade). Thus obtained antibody was subjected thrice to ultrafiltration by use of Centriprep 10 (manufactured by Amicon Corp.). Five milligrams of antibody was dissolved in 2.7 ml of a buffer solution (pH 8.0) made of 50 mM Tris and 0.15 M NaCl, followed by adding 0.3 ml of 2 M 2-mercaputoethanol, further followed by reducing at 15 degrees centigrade for 3 hr.

To the solution, 3 ml of 0.6 M iodoacetamide was added. Thereafter, by use of 1 M Tris, the pH was controlled to 8. Then, the solution was incubated at 15 degrees centigrade for 15 min. Thus obtained solution was subjected to ultrafiltration to be 0.5 ml, thereafter one half thereof was subjected to HPLC (column: Protein-Pak 300, 7.8 × 300 mm, manufactured by Nippon Waters Corp.) with 6 M guanidine hydrochloride (pH = 6.5) as an elution solution at a flow rate of 0.2 ml/min. Each of fractions of the light and heavy chains was gathered, followed by diluting with 6 M guanidine hydrochloride. These, while buffer exchanging 7 times within 3 to 4 days, were dialyzed against PBS at 4 degrees centigrade.

The ECL2B-2 and ECL2B-3 prepared in the abovementioned experiment were identified and quantitatively determined by experiments 3-3 and 3-4 below.

### [Experiment 3-3] (Immunoassay due to ELISA method)

Fifty micro-liters of a partial peptide (RSSHFPYSQYQFWKNFQTLKG (shown in sequence No.40): One that is synthesized with a cystine residue at a C terminal of the CCR-5 partial peptide used in the experiment 3-1 deleted. Hereinafter, the peptide is referred to as ECL2B peptide) of CCR-5 dissolved in a PBS solution (5 µg/ml) was immobilized on an immunization plate (manufactured by Nunc Corp.). The blocking was applied with 2% gelatin at room temperature. After the plate was cleansed, ECL2B-2 or ECL2B-3 was immune reacted, followed by conducting a reaction with anti-mouse Ig (G + A + M) conjugated with alkali phosphatase. After the substrate reaction, by use of an immunization plate reader (trade name NJ-2001, manufactured by InterMed Corp.), the absorbance at 405 nm was measured.

As a result of the immunoassay in the experiment, classes and subclasses of the obtained monoclonal antibodies ECL2B-2 and ECL2B-3 were IgG₁ (κ). The monoclonal antibodies did not cross-react with other proteins such as KLH, HSA, OVA and human hemoglobin. Furthermore, the monoclonal antibodies did not react with peptides such as gp120 V3 cyclic peptide of HIV-1 (CTRPNNNTRKSIHIGPGRAFYTTGEIIGDIRQAHC: sequence No.41), peptide TP41-1 of a retention region of gp41 (HIV-1/TPRGPDRPEGIEEEGGERDRD: sequence No.42), RT-2 (KLLRGTKALTFVIPLTEEAE: sequence No.43), 41S-2mAb CDRL-1 (RSSKSLLYSNGNTYLY: sequence No.44), 1D3 mAb CDRH-2 (DKFNQNYSTAGNYPNIR: sequence No.45) and CA-2 (RSSKSLLYSNGNTYLYGPDKFNQNYSTAGNYPNIR: sequence No.46).

### [Experiment 3-4] (HPLC analysis and mass spectrometry)

In order to monitor a catalytic reaction, 20 µl of the reaction liquid obtained in experiment 3-3, under the conditions of a temperature of 40 degrees centigrade and a constant composition of 13% acetonitrile/0.08% TFA, was injected in a reverse phase (RP)-HLPC (column: Waters Puresil C₁₈ column (4.6 × 150 mm: manufactured by Waters NY, HPLC: manufactured by Jasco Corp.)) at a flow rate of 0.5 ml/min. As a result, the purities of the antibody peptides were 90% or more.

The antibody peptides were identified by use of MALDI-TOF-MASS (trade mark: AUTOFLEX, manufactured by Brucker Ltd.). As a result, masses thereof were same as theoretical ion distributions. The same mass spectrometry conditions were applied to identify fragments found in catalytic reaction due to light chains of the ECL2B-2 and ECL2B-3.

### [Experiment 3-5] (Sequence determination and molecular modeling of ECL2B-2 and ECL2B-3 antibodies)

The mRNA of each of ECL2B-2 and ECL2B-3, by use of an mRNA purification kit (manufactured by Amersham Pharmacia Biotech Corp.), was isolated from a hybridoma having ECL2B-2 or ECL2B-3. Thereafter, cDNAs of the light and heavy chains were synthesized by use of a single strand cDNA synthesis kit (manufactured by Life Science Inc.).

The sequence determination was carried out by use of an Auto Read Sequencing Kit (manufactured by Amersham Pharmacia Biotec) and a DNA Auto Sequencer (trade name: ALF II, manufactured by Amersham Pharmacia Biotech). The computer analysis (molecular modeling) for constituting three-dimensional molecular structures was carried out by use of a workstation (manufactured by Silicon Graphics Inc.) and the AbM software (manufactured by Oxford Molecular Ltd.).

Thus obtained PDB data, by use of a software Discover II (manufactured by Molecular Simulations Inc.), were applied so that a total energy might be minimum. In order to schematize structures, software, Protein Adviser Ver. 3.5 (manufactured by FSQ Ltd.), was used.

According to methods as mentioned above, amino acid sequences of variable regions of light and heavy chains of monoclonal antibodies ECL2B-2 and ECL2B-3 and sequences of cDNAs that code the amino acid sequences were determined.

An amino acid sequence shown in sequence No.26 is an amino acid sequence of an ECL2B-2 light chain, and a base sequence shown in sequence No. 27 is a base sequence of cDNA that codes the amino acid sequence. An amino acid sequence shown in sequence No.28 is an amino acid sequence of an ECL2B-2 heavy chain, and a base sequence shown in sequence No.29 is a base sequence of cDNA that codes the amino acid sequence. An amino acid sequence shown in sequence No.30 is an amino acid sequence of an ECL2B-3 light chain, and a base sequence shown in sequence No.31 is a base sequence of cDNA that codes the amino acid sequence. An amino acid sequence shown in sequence No.32 is an amino acid sequence of an ECL2B-3 heavy chain, and a base sequence shown in sequence No.33 is a base sequence of cDNA that codes the amino acid sequence.

In the next place, on the basis of the respective amino acid sequences of which sequences were determined, the molecular modeling was carried out. In Fig.35, a three-dimensional structure of a variable region of ECL2B-2 estimated according to the molecular modeling was shown. Furthermore, in Fig.36, a three-dimensional structure of a variable region of ECL2B-3 estimated according to the molecular modeling was shown.

In a light chain of ECL2B-2 (ECL2B-2-L), three amino acid residues, Asp¹ (or Asp^{27d}), Ser^{27a} and His⁹³, were estimated to be able to form a catalytic triad residue structure in a molecular structure (Fig.35).

Many serine proteases such as trypsin, thrombin and plasmin have a catalytic triad residue structure made of Asp, Ser and His at an active site that cleaves a peptide bond. A light chain (VIPase-L) of a natural antibody enzyme VIPase that can decompose antigenic vasoactive intestinal peptide (VIP) is reported to have three amino acid residues, Asp¹ (inside of FR-1), Ser^{27a} (inside of CDR1) and His⁹³ (inside of CDR3), in a structure thereof [reference literature 10: S. Paul et al., J. Biol. Chem. 269 (1994), page 32389]. Furthermore, the inventors of the present invention have reported that i41SL1-2-L that is a light chain of monoclonal antibody i41SL1-2 has a catalytic triad residue made of the same Asp¹, Ser^{27a} and His⁹³ and exhibits the enzymatic activity of decomposing CDR-1 peptide that is an antigen thereof [reference literature 11: Y. Zhou, E. Hifumi, H. Kondo and T. Uda, Biological Systems Engineering, ACS symposium Series 830, 200-208 page (2002), and reference literature 12: T. Uda and E. Hifumi, Proceedings of International Symposium on Nano-Intelligent Materials/Systems, pp47 to 52, (2002 Tokyo)].

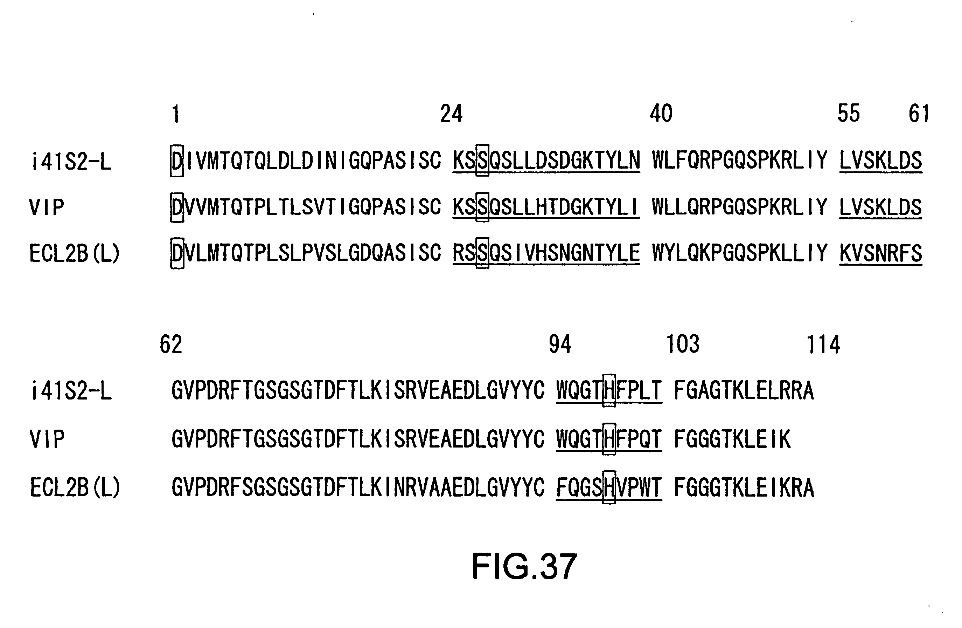

In Fig. 37, amino acid sequences of i41SL1-2-L (denoted as i41S2-L in Fig.37), VIPase-L (denoted as VIP in Fig.37) and ECL2B-2-L (denoted as ECL2B (L) in Fig.37) are compared. In Fig.37, in the respective sequences, each of amino acids that constitute a catalytic triad residue structure is surrounded with a square frame. As shown in Fig.37, the ECL2B-2-L was confirmed to have three amino acid residues at positions same as that of VIPase-L and i41SL1-2-L. These belong the same germline bd2; accordingly, their sequence homology is very high.

It is reported that in the VIPase, owing to the site-directed mutation, a catalytic triad residue made of Asp¹, Ser^{27a} and His⁹³ is an active site [reference literature 10]. From this as well, the ECL2B-2-L is estimated to have the catalytic activity of decomposing the antigenic peptide CCR-5.

On the other hand, a light chain of ECL2B-3 (ECL2B-3-L) was estimated to have a little bit different type of catalytic triad residue, that is, Ser¹⁴ (or Ser⁶³), His⁷⁶ and Asp⁸² (Fig.36).

Subsequently, distances between residues that constitute a catalytic triad residue were confirmed. The molecular modeling, different from the X-ray structural analysis, cannot accurately determine distances between atoms; accordingly, distances between α-carbons in the residues were estimated as approximate distances between the residues that constitute the catalytic triad residue. Thus obtained data and data of other natural antibody enzymes are shown together in Table 7.

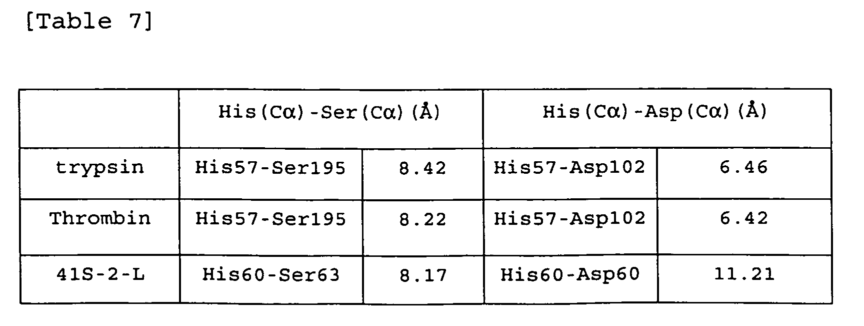

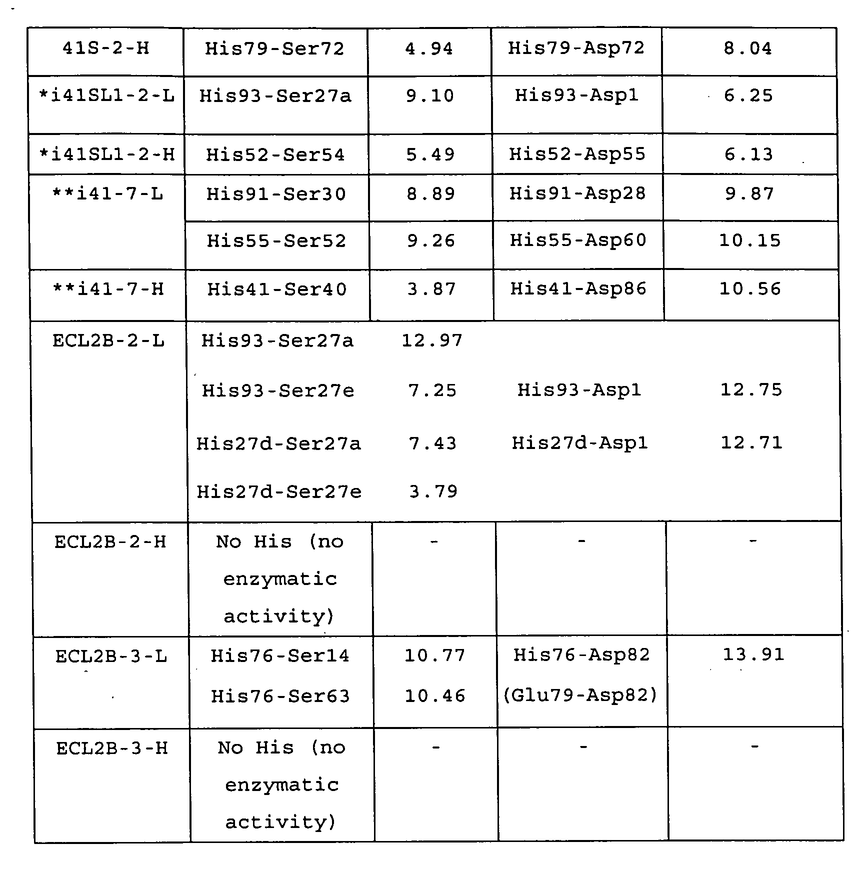

As shown in Table 7, distances between His(Cα) and Ser(Cα) were comparable to that of enzymes (trypsin and Thrombin) and natural antibody enzymes (41S-2-L, 41S-2-H, i41SL1-2-L and i41-7-L). On the other hand, those of 41S-2-H, i41SL-2-H and i41-7-H were such small as substantially 4 Å.

As to distances between α carbons of His and Asp, a little different feature was exhibited. Trypsin, Thrombin, i41SL1-2-L and i41SL1-2-H had substantially same distances, and other than that had 2 to 5 Å longer distances. Antibodysubunits such as 41S-2-L, 41S-2-H, i41SL1-2-H and i41-7-H exhibited the enzymatic activity like the serine protease. From this fact, ECL2B-2-L and ECL2B-3-L are estimated to have the enzymatic activity like the serine protease.

Subsequently, in order to confirm the enzymatic activity of ECL2B-L and ECL2B-3, a peptide decomposition experiment was carried out as follows.

### [Experiment 3-6] (Enzymatic activity test of ECL2B-2 and ECL2B-3)

Before decomposition experiments of an antigenic peptide ECL2B due to monoclonal antibodies ECL2B-2, ECL2B-3, almost all of glass and plastic instruments and buffering solutions were sterilized by heating (at 180 degrees centigrade and for 2 hr) or by treating with an autoclave (121 degrees centigrade and for 20 min) or a 0.2 µm sterilizing filter. Operations in the experiment were carried out in a safety cabinet to inhibit foreign matters in air from contaminating. The decomposition reactions were conducted at a temperature of 25 degrees centigrade and in a 7.5 mM phosphate buffer (pH 6.5). In order to monitor the reaction, 20 µl of a reaction liquid was injected in an RP-HPLC (manufactured by Jasco) under the conditions of a temperature of 40 degrees centigrade and a constant composition.

Firstly, with light chains of the monoclonal antibodies ECL2B-2 and ECL2B-3 (ECL2B-2-L and ECL2B-3-L), catalytic decomposition tests of an antigenic peptide (ECL2B peptide: RSSHFPYSQYQFWKNFQTLKG) were carried out.

In a sterilized test tube, 500 µl of a purified ECL2B-2-L (0.8 µM) or ECL2B-3-L (0.8 µM) -containing solution was blended at 25 degrees centigrade with a same volume of a solution containing 120 µM ECL2B peptide. A time course of the ECL2B peptide was monitored by use of the RT-HPLC. For the sake of reference, with peptide B instead of ECL2B-peptide as a substrate, an experiment was carried out similarly.

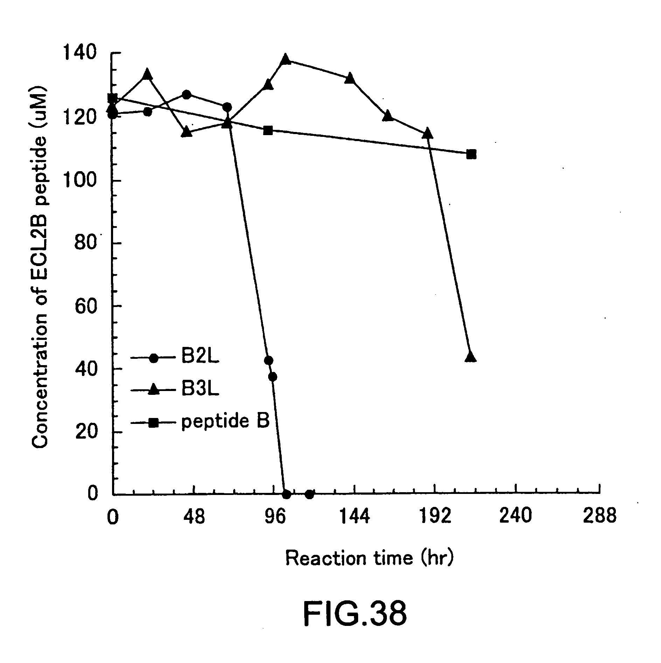

Results thereof are shown in Fig.38. In Fig.38, a horizontal axis shows a reaction time (hr) and a vertical axis shows a concentration (µM) of the ECL2B peptide that is a substrate.

In the case of ECL2B-2-L being used (expressed with ● in Fig.38), the decomposition of the ECL2B peptide began with an induction period of substantially 80 hr after ECL2B-2-L and ECL2B peptide were mixed and thereafter a concentration of the ECL2B peptide rapidly decreased. This kind of induction period is frequently found in many reactions. The ECL2B peptide completely disappeared within substantially 90 hr after the mixing.

In the case of ECL2B-3-L being used (expressed with ▲ in Fig.38), the decomposition of the ECL2B peptide began with such a longer induction period as substantially 190 hr after ECL2B-3-L and ECL2B peptide were mixed. The ECL2B peptide was rapidly degraded within substantially 250 hr after the induction period. In the case of a similar experiment being conducted with peptide B (expressed with ■ in Fig.38), the peptide was not decomposed.

Subsequently, decomposition products obtained when the ECL2B-2-L was used as an enzyme were analyzed with a MALDI-TOF-MASS. As a result thereof, many kinds of peptide fragments derived from the ECL2B peptide were confirmed. At an initial stage of the decomposition, relatively long peptides such as RSSHFPYSQYQFWKNFQ and SSHFPYSQYQFW were observed. At the final stage of the decomposition (a stage where the peptide shown in sequence No.40 disappears from a reaction system), it was confirmed that many small peptides such as RSSHFPYS, RSSHFPY, SSHFPYSQYQ, SSHFPYSQ, HFPYSQYQFWKN, YSQYQFWKN and YSQYQ were generated. Since the abovementioned many peptide fragments were generated, it can be said that the ECL2B-2-L or ECL2B-3-L could continuously decompose the antigenic peptide RSSHFPYSQYQFWKNFQTLKG.

As already mentioned above, three residues that constitute a catalytic triad residue in the ECL2B-2-L are located at positions same as that of three catalytic triad residues of VIPase and i41SL1-2-L. This consistency suggests that the light chain of ECL2B-2 (ECL2B-2-L) has capability of catalytically decomposing the antigenic peptide (ECL2B peptide).

From results mentioned above, it was confirmed that the ECL2B-2-L and ECL2B-3-L were antibody enzymes that can completely decompose the peptide that constitutes an extracellular region of the CCR-5.

Under the conditions same as that of the abovementioned experiment, with heavy chains of monoclonal antibodies ECL2B-2 and ECL2B-3 (ECL2B-2-H and ECL2B-3-H), similar experiments were carried out. However, both these heavy chains did not decompose the antigenic peptide ECL2B. That is, in mass spectrometry, any peptide fragments could not be detected.

Furthermore, a decomposition experiment of an antigenic peptide with intact monoclonal antibody ECL2B-2 containing also a region other than a variable region was carried out under the conditions same as that of the above experiment. However, also in this case, the ECL2B peptide that is a partial peptide of the CCR-5 was not at all decomposed.

### [Experiment 3-7] (Preparation of ECL2B-4 antibody and purification of antibody subunit)

According to a method similar to experiment 3-1, monoclonal antibody ECL2B-4 was prepared with a partial peptide (sequence No.38) made of 20 amino acids that constitute an extracellular region of the CCR-5 as an immunogen.

Subsequently, according to a method similar to experiment 3-2, the ECL2B-4 was purified. The antibody ECL2B-4 purified in the experiment was identified and quantitatively analyzed according to a method similar to the experiment 3-3.

### [Experiment 3-8] (Sequence determination and molecular modeling of ECL2B-4 antibody)

According to a method similar to that of the experiment 3-5, amino acid sequences of variable regions of light and heavy chains of the ECL2B-4 and cDNA sequences that code the amino acid sequences were determined. Thereafter, on the basis of determined amino acid sequences, the molecular modeling was carried out. Results thereof are shown below.

An amino acid sequence shown in sequence No.34 is an amino acid sequence of a light chain of the ECL2B-4 and a base sequence shown in sequence No.35 is a base sequence of a cDNA that codes the amino acid sequence. An amino acid sequence shown in sequence No.36 is an amino acid sequence of a heavy chain of the ECL2B-4 and a base sequence shown in sequence No.37 is a base sequence of a cDNA that codes the amino acid sequence.

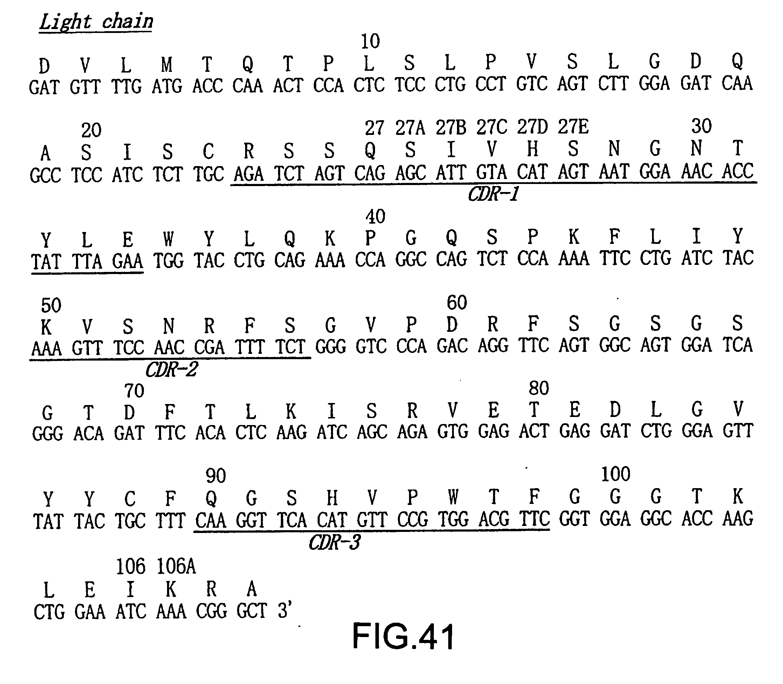

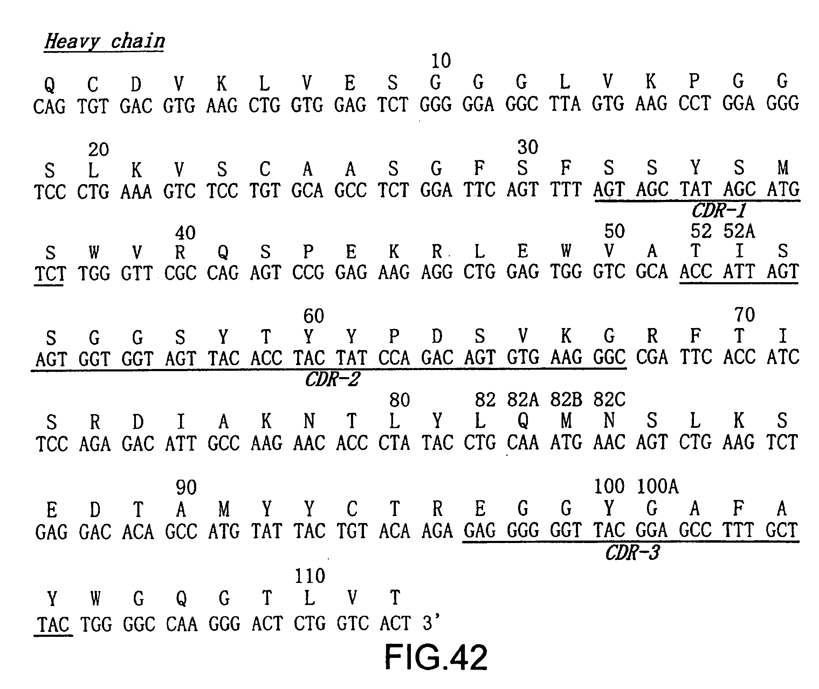

Furthermore, in Fig.41, an amino acid sequence of a light chain of the ECL2B-4 and a base sequence of a cDNA that code the amino acid sequence are shown together and variable regions (CDR-1 through CDR-3) are shown. In Fig.42, an amino acid sequence of a heavy chain of the ECL2B-4 and a base sequence of a cDNA that code the amino acid sequence are shown together and variable regions (CDR-1 through CDR-3) are shown.

In the next place, based on the determined respective amino acid sequences, the molecular modeling was carried out. In Fig.39, a three-dimensional structure of a variable region of the ECL2B-4-L estimated by means of the molecular modeling is shown. Furthermore, in Fig.40, a three-dimensional structure of a variable region of the ECL2B-4-H estimated by means of the molecular modeling is shown.

In the light chain of the ECL2B-4 (ECL2B-4-L), three amino acid residues, S26 (or any one of S27a, S27e and S92), H27d (or H93) and D1, were estimated to be able to form a catalytic triad residue in a molecular structure (Fig.39).

Furthermore, in the heavy chain of the ECL2B-4 (ECL2B-4-H), three amino acid residues, S60 (or S84), E46 (or E85) and D59 (or D86), were estimated to be able to form a catalytic triad residue in a molecular structure (Fig.40).

Subsequently, in order to confirm the enzymatic activity of ECL2B-4-L and ECL2B-4-H, the peptide decomposition experiments were carried out as follows.

### [Experiment 3-9] (Enzymatic activity test of ECL2B-4 antibody)

According to a method similar to the experiment 3-6, the enzymatic activity test was carried out of ECL2B-4-L and ECL2B-4-H.

Firstly, the decomposition reaction of ECL2B peptide (sequence No.38) was carried out in a 15 mM phosphate buffer (pH 6.5) at 25 degrees centigrade. In order to monitor the reaction, 20 µl of a reaction liquid was injected in the RP-HPLC (manufactured by Jasco) under the conditions of a column temperature of 40 degrees centigrade and a constant composition.

As a reaction liquid of the decomposition reaction, ① through ⑤ below were prepared.
① ECL2B-4(L) 0.8 µM + ECL2B peptide: 50 µM: 800 µl prepared
② ECL2B-4(H) 0.4 µM + ECL2B peptide: 50 µM: 800 µl prepared
③ ECL2B peptide: 50 µM: 500 µl prepared
④ ECL2B-4(L): 0.8 µM : 300 µl prepared and
⑤ ECL2B-4(H): 0.4 µM : 300 µl prepared

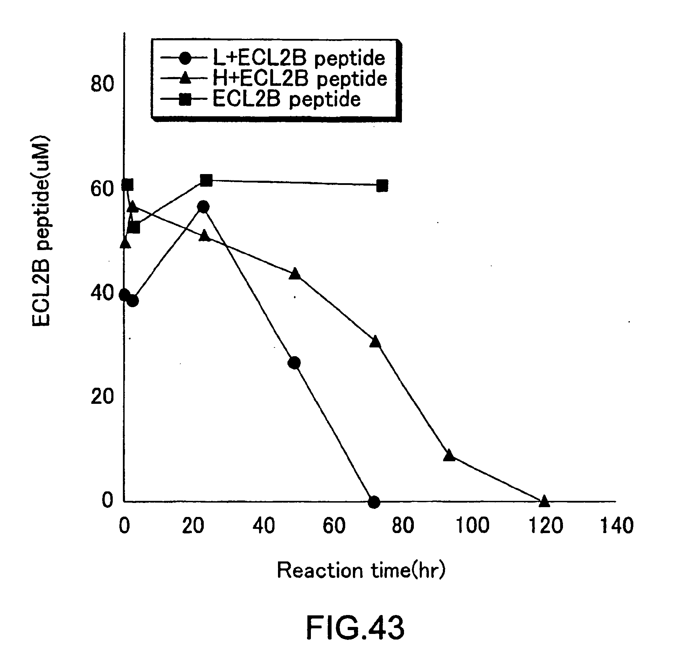

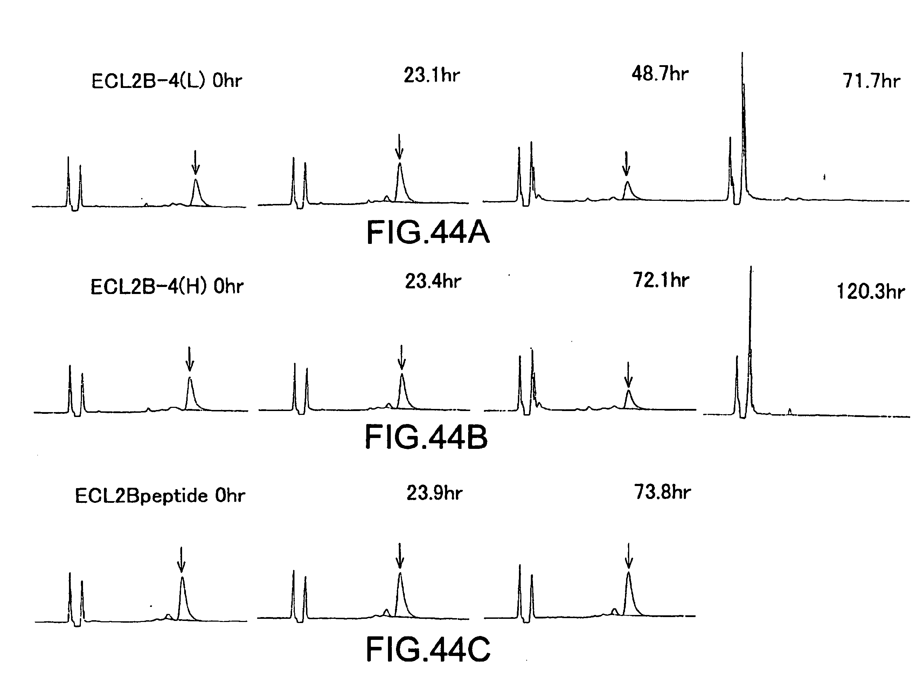

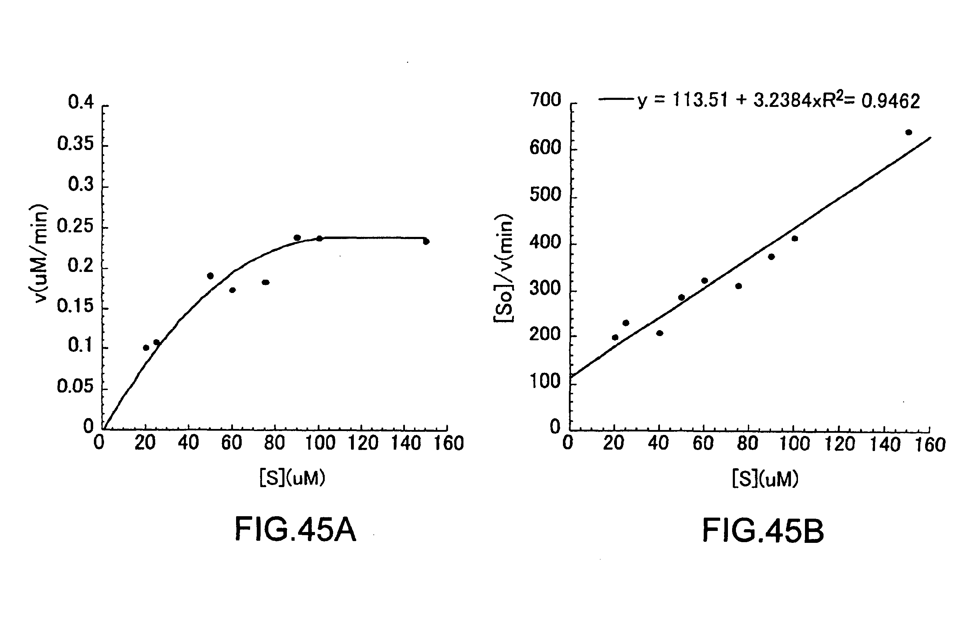

Results thereof are shown in Figs. 43 through 45B. In Fig.43, a horizontal axis expresses a reaction time (hr) and a vertical axis expresses a concentration (µM) of ECL2B peptide that is a substrate. Furthermore, in Figs.44A through 44C, results obtained by monitoring with the HPLC are shown. In Figs.44A through 44C, peaks of the ECL2B peptide are shown with arrow marks. In addition, in Figs.45A and 45B, results of the kinetic analysis in the enzyme decomposition experiment are shown. Fig.45A is a graph showing relationship between a substrate (ECL2B peptide) concentration and a decomposition velocity, and Fig.45B is a graph showing a result of Hanes-Woolf plot.

Then, Vmax, Km, kcat and kcat/Km values (average plot) calculated by use of the Hanes-Woolf plot are shown in Table 8 below.

**[Table 8]**

| Hanes-Woolf plot | | | |
|---|---|---|---|
| Vmax (µM/min) | Km (M) | kcat (min⁻¹) | kcat/Km (/M·min) |
| 0.31 | 3.5 × 10⁻⁵ | 0.72 | 2.2 × 10⁴ |

From the results, it was confirmed that the ECL2B-4-L and ECL2B-4-H had the enzymatic activity in the decomposition reaction of the ECL2B peptide. Accordingly, it was confirmed that the ECL2B-4-L and ECL2B-4-H were antibody enzymes that can target peptide that constitutes an extracellular region of the CCR-5 and completely decompose it.

Subsequently, the enzymatic activity test of the ECL2B-4-L and ECL2B-4-H against TP41-1 peptide (sequence No.42) was carried out. The decomposition reaction was carried out in a 15 mM phosphate buffer (pH 6.5) at 25 degrees centigrade. In order to monitor the reaction, 20 µl of a reaction liquid was injected in the RP-HPLC (manufactured by Jasco) under the conditions of a column temperature of 40 degrees centigrade and a constant composition.

As a reaction liquid of the decomposition reaction, ① through ⑤ below were prepared.
① ECL2B-4(L) 0.8 µM + TP41-1 peptide: 120 µM: 800 µl prepared
② ECL2B-4(H) 0.4 µM + TP41-1 peptide: 120 µM: 800 µl prepared
③ TP41-1 peptide: 120 µM: 500 µl prepared
④ ECL2B-4 (L): 0.8 µM : 300 µl prepared and
⑤ ECL2B-4(H): 0.4 µM : 300 µl prepared

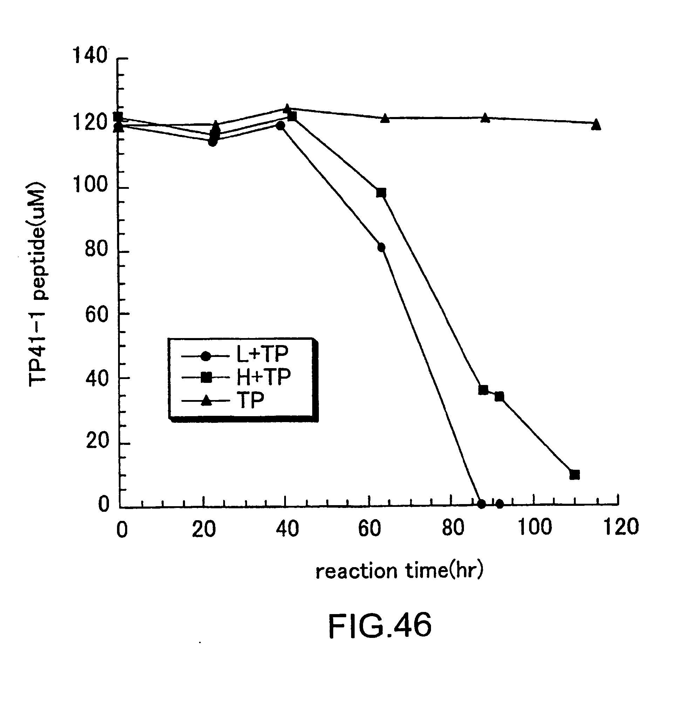

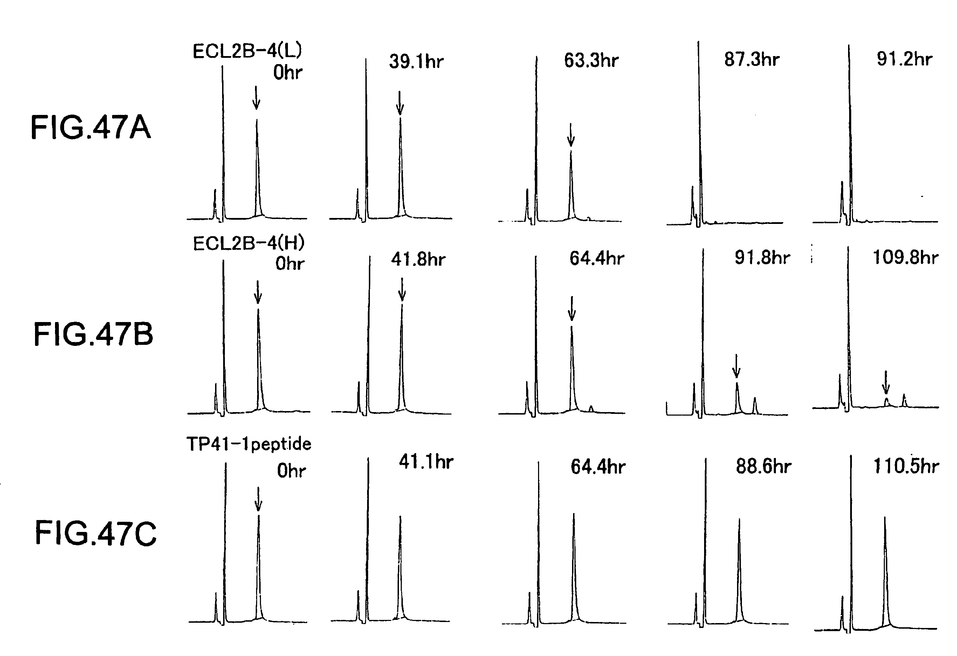

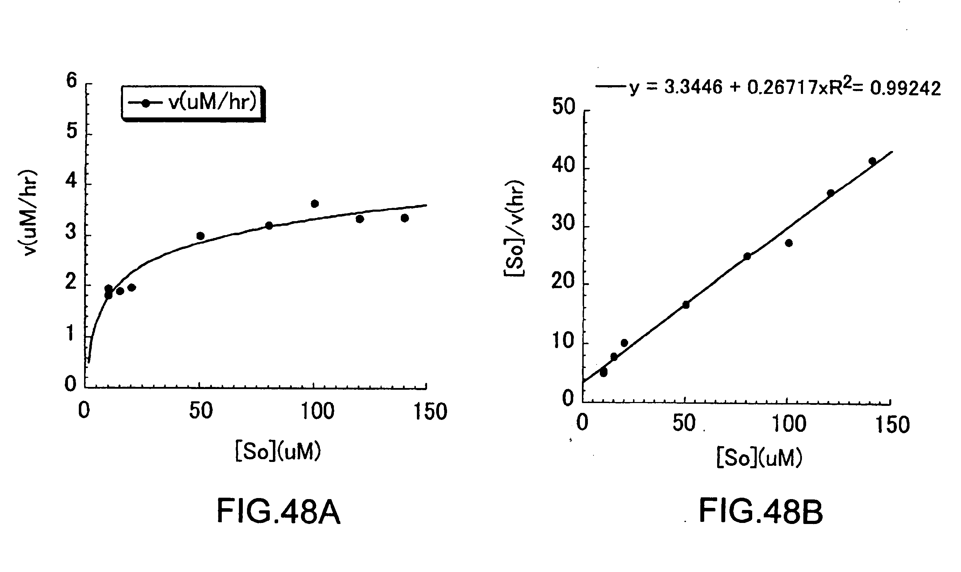

Results thereof are shown in Figs.46 through 48B. In Fig.46, a horizontal axis expresses a reaction time (hr) and a vertical axis expresses a concentration (µM) of TP41-1 peptide that is a substrate. Furthermore, in Figs.47A through 47C, results obtained by monitoring with the HPLC are shown. Furthermore, in Figs.48A and 48B, results of kinetic analysis of the enzyme decomposition test are shown. In Figs.47A through 47C, peaks of the TP41-1 peptide are shown with arrow marks. Fig.48A is a graph showing relationship between a substrate (TP41-1 peptide) concentration and a decomposition velocity, and Fig. 48B is a graph showing a result of Hanes-Woolf plot.

Then, Vmax, Km, kcat and kcat/Km values (average plot) calculated by use of the Hanes-Woolf plot are shown in Table 9 below.

**[Table 9]**

| Hanes-Woolf plot | | | |
|---|---|---|---|
| Vmax (µM/min) | Km (M) | kcat (min⁻¹) | kcat/Km (/M·min) |
| 0.0624 | 1.25 × 10⁻⁵ | 0.16 | 1.25 × 10⁴ |

From the results, it was confirmed that the ECL2B-4-L and ECL2B-4-H had low enzymatic activity also in the decomposition reaction of the TP41-1 peptide. Furthermore, as obvious from comparison of Tables 8 and 9, it was experimentally shown that the ECL2B-4-L decomposed ECL2B peptide that is an inherent antigen faster than the TP41-1 peptide, and it was verified that the ECL2B-4-L was assuredly an antibody enzyme against the ECL2B.

Amino acid sequences of variable regions of antibodies of which sequences were determined in the examples 1 through 3 and base sequences of cDNAs that code the amino acid sequences are summarized and shown in table 10 below.

**[Table 10]**

| Sequence Number | Kind of Sequence |
|---|---|
| 1 | An amino acid sequence of a variable region of I41SL1-2-H |
| 2 | A base sequence of cDNA that codes a variable region of I41SL1-2-H |
| 3 | An amino acid sequence of a variable region of I41SL-2-L |
| 4 | A base sequence of cDNA of a variable region of I41SL1-2-L |
| 5 | An amino acid sequence of a variable region of I41-7-H |
| 6 | A base sequence of cDNA that codes a variable region of I41-7-H |
| 7 | An amino acid sequence of a variable region of I41-7-L |
| 8 | Abase sequence of cDNA that codes a variable region of I41-7-L |
| 14 | An amino acid sequence of a variable region of HpU-18--L |
| 47 | A base sequence of cDNA that codes a variable region of HpU-18-L |
| 15 | An amino acid sequence of a variable region of HpU-9-L |
| 48 | A base sequence of cDNA that codes a variable region of HpU-9-L |
| 16 | An amino acid sequence of a variable region of HpU-2-H |
| 49 | Abase sequence of cDNA that codes a variable region of HpU-2-H |
| 17 | An amino acid sequence of a variable region of HpU-20-L |
| 18 | A base sequence of cDNA that codes a variable region of HpU-20-L |
| 19 | An amino acid sequence of a variable region of HpU-20-H |
| 20 | A base sequence of cDNA that codes a variable region of HpU-20-H |
| 21 | An amino acid sequence of a variable region of UA-15-L |
| 22 | A base sequence of cDNA that codes a variable region of UA-15-L |
| 23 | An amino acid sequence of a variable region of UA-15-H |
| 24 | A base sequence of cDNA that codes a variable region of UA-15-H |
| 26 | An amino acid sequence of a variable region of ECL2B-2-L |
| 27 | A base sequence of cDNA that codes a variable region of ECL2B-2-L |
| 28 | An amino acid sequence of a variable region of ECL2B-2-H |
| 29 | A base sequence of cDNA that codes a variable region of ECL2B-2-H |
| 30 | An amino acid sequence of a variable region of ECL2B-3-L |
| 31 | A base sequence of cDNA that codes a variable region of ECL2B-3-L |
| 32 | An amino acid sequence of a variable region of ECL2B-3-H |
| 33 | A base sequence of cDNA that codes a variable region of ECL2B-3-H |
| 34 | An amino acid sequence of a variable region of ECL2B-4-L |
| 35 | A base sequence of cDNA that codes a variable region of ECL2B-4-L |
| 36 | An amino acid sequence of a variable region of ECL2B-4-H |
| 37 | A base sequence of cDNA that codes a variable region of ECL2B-4-H |

Specific embodiments and examples exemplified in the section of "Best Mode for Carrying Out the Invention" are shown only for clarifying technical contents of the present invention. The present invention should not be construed by narrowly limiting to such specific examples and, within a spirit of the invention and claims described below, can be variously modified and carried out.

### Industrial Applicability

As mentioned above, antibody enzymes and genes involving the present invention can be applied to treatment and diagnosis of diseases such as various infectious symptoms and cancers. Furthermore, when a target antibody enzyme can be obtained, it can lead to a development of a novel clinical diagnosis.

Furthermore, the antibody enzymes and genes involving the invention can be applied to novel biosensors as new biomaterials, and can be applied also to diagnosis of diseases and inspections such as and environmental measurements.

Still furthermore, when the antibody enzymes and genes involving the present invention are used, it can be considered to apply to develop effective synthesis means and so on in the food industry and chemical industry.

Furthermore, according to a production method according to the invention of antibody enzymes, antibody enzymes having the enzymatic activity of cleaving or decomposing polypeptides and antigen proteins selectively among immunologically prepared antibodies can be efficiently obtained.

Still furthermore, according to a production method according to the invention of antibody enzymes, by use of an existing genetic engineering process, antibody enzymes having the enzymatic activity of readily cleaving or decomposing polypeptides and antigen proteins can be efficiently prepared.

Furthermore, according to antibody enzymes according to the invention, HP bacteria urease can be specifically decomposed to efficiently eliminate the HP bacteria. The antibody enzyme is considered that, different from existing bacteria eliminating agents, it does not impart the HP bacteria the drug resistance; accordingly, it can be effectively used as treatment drugs of HP bacteria infectious patients. Still furthermore, the antibody enzymes can, by orally taking, exhibit the effect of eliminating the HP bacteria; accordingly, it can be used as drugs that prevent the HP bacteria from infecting.

Furthermore, the antibody enzyme according to the invention specifically works against chemokine receptor CCR-5 that plays a very important role when the AIDS virus infects with a human and can decompose to delete its function; accordingly, the antibody enzyme is useful as an anti-HIV drug for preventing the HIV from infecting and for treating the AIDS. Antibody enzymes according to the invention decompose the CCR-5 according to a novel mechanism utterly different from existing anti-HIV drugs that suppress the CCR-5 from working; accordingly, it can be expected to effectively use in the treatment of the AIDS against which at present an effective treatment is not found.

## Claims

1. An antibody enzyme production method, comprising:
an antibody structure analysis process, wherein the antibody structure analysis process includes performing a stereostructure estimation step of estimating a stereostructure of an antibody from an amino acid sequence and a catalytic triad residue structure confirmation step where whether a catalytic triad residue structure in which a serine residue, an aspartate residue, and a histidine residue or glutamate residue are present in stereostructurally close proximity is present or not in an estimated stereostructure of the antibody is confirmed.

2. The antibody enzyme production method according to claim 1,
wherein in the catalytic triad residue structure confirmation step, in order to confirm a catalytic triad residue structure, that a serine residue, an aspartate residue and a histidine residue or glutamate residue are present within the range of 3 to 20 Å in a stereostructure is used as an index.

3. The antibody enzyme production method according to claim 1 or 2,
wherein in the catalytic triad residue structure confirmation step, in order to confirm a catalytic triad residue structure, either having a structure same as a catalytic triad residue structure common to existing antibody enzymes or having a structure that is estimated to form a catalytic triad residue structure by using by one amino acid residue in a different position from that of the existing catalytic triad residue structure is used as an index.

4. The antibody enzyme production method according to any one of claims 1, 2, and 3,
wherein in the catalytic triad residue structure confirmation step, in order to confirm a catalytic triad residue structure, that an antibody has a germ cell gene type from which a known antibody enzyme is derived is used as an index.

5. The antibody enzyme production method according to any one of claims 1, 2, 3, and 4,
wherein in the catalytic triad residue structure confirmation step, in order to confirm a catalytic triad residue structure, that a complimentarity determining region 1 (CDR1) is constituted of 16 amino acid residues and a histidine residue is at the 93^{rd} according to Kabat numbering scheme is used as an index.

6. The antibody enzyme production method according to any one of claims 1, 2, 3 and 4,
wherein in the catalytic triad residue structure confirmation step, in order to confirm a catalytic triad residue structure, that a complimentarity determining region 1 (CDR1) is constituted of 11 amino acid residues and a histidine residue is at the 91^{st} or 55^{th} according to Kabat numbering scheme is used as an index.

7. The antibody enzyme production method according to any one of claims 1, 2, 3, 4, 5 and 6, further comprising:
an antibody production process where an antibody is produced from a hybridoma obtained by fusing a mouse spleen lymph corpuscle immunized with an antigen and a mouse myeloma cell.

8. The antibody enzyme production method according to claim 7,
wherein, as the antigen, an antigen protein obtained by coupling a substance that becomes an antigen determinant with a carrier protein is used.

9. The antibody enzyme production method according to any one of claims 1, 2, 3, 4, 5, 6, 7 and 8, further comprising:
a catalytic triad residue structure introducing process where by use of stereostructure information obtained after the antibody structure analysis process, according to a genetic engineering process, a catalytic triad residue structure is introduced in an antibody.

10. Antibody enzyme, comprising:
in a stereostructure, a catalytic triad residue structure in which a serine residue, an aspartate residue and a histidine residue or glutamate residue are stereostructurally in proximity.

11. An antibody enzyme,
wherein a complimentarity determining region 1 (CDR1) is constituted of 16 amino acid residues and a histidine residue is at the 93^{rd} by Kabat numbering scheme.

12. An antibody enzyme,
wherein a complimentarity determining region 1 (CDR1) is constituted of 11 amino acid residues and a histidine residue is at the 91^{st} or 55^{th} by Kabat numbering scheme.

13. Antibody enzyme,
wherein the antibody enzyme is produced from a mouse germ cell gene selected from, by Thiebe et al. convention, bb1, bl1, bd2, cr1, cs1, bj2, hf24, 12-41, 19-14, 19-17, 19-23, 19-25, and 21-12.

14. The antibody enzyme according to any one of claims 10, 11, 12 and 13,
wherein the antibody enzyme is a heavy chain or a light chain of an antibody.

15. The antibody enzyme according to claim 14, comprising:
an amino acid sequence shown in sequence No.1 or an amino acid sequence in which in the amino acid sequence shown in sequence No.1, at least one amino acid is replaced, deleted, inserted and/or added.

16. The antibody enzyme according to claim 14, comprising:
an amino acid sequence shown in sequence No.3 or an amino acid sequence in which in the amino acid sequence shown in sequence No.3, at least one amino acid is replaced, deleted, inserted and/or added.

17. The antibody enzyme according to claim 14, comprising:
an amino acid sequence shown in sequence No.5 or an amino acid sequence in which in the amino acid sequence shown in sequence No.5, at least one amino acid is replaced, deleted, inserted and/or added.

18. The antibody enzyme according to claim 14, comprising:
an amino acid sequence shown in sequence No.7 or an amino acid sequence in which in the amino acid sequence shown in sequence No.7, at least one amino acid is replaced, deleted, inserted and/or added.

19. A gene that codes an antibody enzyme according to any one of claims 10, 11, 12, 13, 14, 15, 16, 17 and 18.

20. The antibody enzyme according to claim 14 that is an antibody of Helicobacter Pylori bacterium urease or an antibody fragment thereof and works as a decomposing enzyme of the urease.

21. The antibody enzyme according to claim 20,
wherein the antibody enzyme includes a variable region of an antibody of the urease.

22. The antibody enzyme according to claim 20 or 21, comprising an amino acid sequence shown in sequence No.14 or an amino acid sequence in which in the amino acid sequence shown in sequence No.14, at least one amino acid is replaced, deleted, inserted and/or added.

23. The antibody enzyme according to claim 20 or 21, comprising an amino acid sequence shown in sequence No.15 or an amino acid sequence in which in the amino acid sequence shown in sequence No.15, at least one amino acid is replaced, deleted, inserted and/or added.

24. The antibody enzyme according to claim 20 or 21, comprising an amino acid sequence shown in sequence No.16 or an amino acid sequence in which in the amino acid sequence shown in sequence No. 16, at least one amino acid is replaced, deleted, inserted and/or added.

25. The antibody enzyme according to claim 20 or 21, comprising an amino acid sequence shown in sequence No.17 or an amino acid sequence in which in the amino acid sequence shown in sequence No.17, at least one amino acid is replaced, deleted, inserted and/or added.

26. The antibody enzyme according to claim 20 or 21, comprising an amino acid sequence shown in sequence No.19 or an amino acid sequence in which in the amino acid sequence shown in sequence No.19, at least one amino acid is replaced, deleted, inserted and/or added.

27. The antibody enzyme according to claim 20 or 21, comprising an amino acid sequence shown in sequence No.21 or an amino acid sequence in which in the amino acid sequence shown in sequence No.21, at least one amino acid is replaced, deleted, inserted and/or added.

28. A curative drug to a Helicobacter Pylori bacteria infected patient, comprising the antibody enzyme according to any one of claims 20, 21, 22, 23, 24, 25, 26 and 27.

29. An infection control agent of Helicobacter Pylori bacteria, comprising the antibody enzyme according to any one of claims 20, 21, 22, 23, 24, 25, 26 and 27.

30. A gene that codes the antibody enzyme according to any one of claims 20, 21, 22, 23, 24, 25, 26 and 27.

31. The gene according to claim 30, comprising a base sequence shown in sequence No.18.

32. The gene according to claim 30, comprising a base sequence shown in sequence No.20.

33. The gene according to claim 30, comprising a base sequence shown in sequence No.22.

34. A transformant, wherein the gene according to any one of claims 30, 31, 32 and 33 is introduced.

35. The transformant according to claim 34, wherein the gene is introduced in a plant to express.

36. An infection control agent of Helicobacter Pylori bacteria, comprising the transformant according to claim 34 or 35.

37. The antibody enzyme according to claim 14,
wherein the antibody enzyme is an antibody of human-derived chemokine receptor CCR-5 or an antibody fragment thereof and decomposes the chemokine receptor CCR-5.

38. The antibody enzyme according to claim 37,
wherein the antibody enzyme includes a variable region of the chemokine receptor CCR-5 antibody.

39. The antibody enzyme according to claim 37 or 38,
wherein the antibody enzyme includes an amino acid sequence shown in sequence No.26 or an amino acid sequence in which in the amino acid sequence shown in sequence No.26, at least one amino acid is replaced, deleted, inserted and/or added.

40. The antibody enzyme according to claim 37 or 38,
wherein the antibody enzyme includes an amino acid sequence shown in sequence No.30 or an amino acid sequence in which in the amino acid sequence shown in sequence No.30, at least one amino acid is replaced, deleted, inserted and/or added.

41. The antibody enzyme according to claim 37 or 38,
wherein the antibody enzyme includes an amino acid sequence shown in sequence No.34 or an amino acid sequence in which in the amino acid sequence shown in sequence No. 34, at least one amino acid is replaced, deleted, inserted and/or added.

42. The antibody enzyme according to claim 37 or 38,
wherein the antibody enzyme includes an amino acid sequence shown in sequence No.36 or an amino acid sequence in which in the amino acid sequence shown in sequence No. 36, at least one amino acid is replaced, deleted, inserted and/or added.

43. An anti-HIV drug that includes the antibody enzyme according to any one of claims 37, 38, 39, 40, 41 and 42 and inhibits AIDS virus from infecting.

44. An anti-HIV drug to an AIDS virus-infected patient, comprising the antibody enzyme according to any one of claims 37, 38, 39, 40, 41 and 42.

45. A gene that codes the antibody enzyme according to any one of claims 37, 38, 39, 40, 41 and 42.

46. The gene according to claim 45, comprising a base sequence shown in sequence No.27.

47. The gene according to claim 45, comprising a base sequence shown in sequence No.31.

48. The gene according to claim 45, comprising a base sequence shown in sequence No.35.

49. The gene according to claim 45, comprising a base sequence shown in sequence No.37.

50. A transformant, wherein the gene according to any one of claims 45, 46, 47, 48 and 49 is introduced.

51. An anti-HIV drug, comprising the transformant according to claim 50.

52. A computer program that lets a computer carry out the antibody structural analysis process according to any one of claims 1, 2, 3, 4, 5 and 6.

53. A machine-readable recording medium, wherein a computer program that lets a computer carry out a program that carries out the antibody structural analysis process according to claims 1, 2, 3, 4, 5 and 6 is recorded.


SEQUENCE LISTING

<110> Japan Science and Technology Corporation

<120> Novel methods of producing antibody-enzyme, the antibody-enz
ymes,
      and the use thereof

<130> A251-01/PCT

<150> JP 2002-211756
<151> 2002-7-19

<150> JP 2002-211768
<151> 2002-7-19

<150> JP 2003-051943
<151> 2003-2-27

<150> JP 2003-198270
<151> 2003-7-17

<150> JP 2003-198281
<151> 2003-7-17

<150> JP 2003-198292
<151> 2003-7-17

<160> 49

<170> PatentIn Ver. 2.1

<210> 1
<211> 117
<212> PRT
<213> Mus musculus

<400> 1
Gln Val Gln Leu Gln His Ser Gly Ala Glu Leu Val Arg Pro Gly Ser
  1               5                  10                  15

Ser Val Lys Val Ser Cys Lys Ala Leu Gly Tyr Thr Ser Thr Asp Tyr
             20                  25                  30

Glu Ile His Trp Val Lys Gln Thr Pro Val Arg Gly Leu Glu Trp Ile
         35                  40                  45

Gly Ala Ile His Pro Gly Ser Asp Val Ile Val Tyr Asn Gln Lys Phe
     50                  55                  60

Lys Gly Thr Ala Thr Leu Thr Ala Asp Lys Ser Ser Ser Thr Ala Tyr

65                  70                  75                  80

Met Glu Leu Arg Ser Leu Thr Ser Glu Asp Ser Ala Val Tyr Tyr Cys
                85                  90                  95

Thr Arg Glu Gly Gly Ser Val Asp Tyr Val Trp Gly Gln Gly Thr Leu
            100                 105                 110

Val Thr Val Ser Ala

        115

<210> 2
<211> 351
<212> DNA
<213> Mus musculus

<400> 2
caggttcaac tgcagcactc tggggctgag ctggtgaggc ctgggtcttc agtgaaggtg
 60
tcctgcaagg ctttgggcta cacatctact gactatgaaa tacactgggt gaagcagaca
 120
cctgtgcgtg gcctggaatg gattggagct attcatccag gaagtgatgt tattgtctac
 180
aatcagaagt tcaagggcac ggccacactg actgcagaca aatcctccag cacagcctac
 240
atggagctca gaagtctgac atctgaggac tctgctgtct attactgtac aagagagggg
 300
ggatctgttg actacgtttg gggccaaggg actctggtca ctgtctctgc a
 351

<210> 3
<211> 114
<212> PRT
<213> Mus musculus

<400> 3
Asp Ile Val Met Thr Gln Thr Gln Leu Thr Leu Thr Ile Asn Ile Gly
 1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Phe Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile

65                  70                  75                  80

Ser Arg Val Glu Ala Glu Asp Leu Gly Val Tyr Tyr Cys Trp Gln Gly
                85                  90                  95

Thr His Phe Pro Leu Thr Phe Gly Ala Gly Thr Lys Leu Glu Leu Arg
            100                 105                 110

Arg Ala

<210> 4
<211> 342
<212> DNA
<213> Mus musculus

<400> 4
gacattgtga tgacccagac tcaactcact ttgacgatta acattggaca accagcctcc
60
atctcttgca agtcaagtca gagcctctta gatagtgatg gaaagacata tttgaattgg
120
ttgttccaga ggccaggcca gtctccaaag cgcctaatct atctggtgtc taaactggac
180
tctggagtcc ctgacaggtt cactggcagt ggatcaggga cagatttcac actgaaaatc
240
agcagagtgg aggctgagga tttgggagtt tattattgct ggcaaggtac acattttcct
300
ctcacgttcg gtgctgggac caagctggag ctgagacggg ct
342

<210> 5
<211> 120
<212> PRT
<213> Mus musculus

<400> 5
Glu Ile Gln Leu Gln Gln Ser Gly Pro Glu Leu Val Arg Pro Gly Ala
1               5                   10                  15

Ser Val Lys Val Ser Cys Lys Ala Ser Gly Tyr Ser Phe Thr Asn Ser
            20                  25                  30

Ile Met Tyr Trp Val Arg Gln Ser His Gly Lys Ser Leu Glu Trp Ile
        35                  40                  45

Gly Tyr Ile Asp Pro Tyr Asn Gly Gly Thr Ser Tyr Asn Gln Lys Phe
    50                  55                  60

Lys Gly Lys Ala Thr Leu Thr Val Asp Lys Ser Ser Ser Thr Ala Phe
65                  70                  75                  80

Met His Leu Asn Ser Leu Thr Ser Glu Asp Ser Ala Val Tyr Phe Cys
                85                  90                  95

Ala Arg Phe Ile Val Val Val Ala Asp Val Met Asp Tyr Trp Gly Gln
            100                 105                 110

Gly Thr Ser Val Thr Val Ser Ser

        115                 120

<210> 6
<211> 360
<212> DNA
<213> Mus musculus

<400> 6
gagatccagc tgcagcagtc tggacctgag ctggtgaggc ctggggcttc agtgaaggtg
60
tcctgcaagg cttctggtta ctcattcact aactccatca tgtactgggt gaggcagagc
120
catggaaaga gccttgagtg gattggatat attgatcctt acaatggtgg tactagctac
180
aaccagaagt tcaagggcaa ggccacattg actgttgaca agtcctctag cacagccttc
240
atgcatctca acagcctgac atctgaggac tctgcagtct atttctgtgc aagatttatt
300
gtggttgttg ctgatgttat ggactactgg ggtcaaggaa cctcagtcac cgtctcctca
360

<210> 7
<211> 109
<212> PRT
<213> Mus musculus

<400> 7
Asp Ile Val Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Glu Val Pro Asp Arg Phe Thr Gly
    50                  55                  60

Ser Gly Ser Gly Thr Asp Tyr Thr Leu Thr Ile Ser Ser Val Gln Ala
65                  70                  75                  80

Glu Asp Leu Ala Leu Tyr Tyr Cys Gln Gln His Tyr Asn Thr Pro Leu
                85                  90                  95

Thr Phe Gly Ala Gly Thr Lys Leu Glu Leu Lys Arg Ala
            100                 105

<210> 8
<211> 327
<212> DNA
<213> Mus musculus

<400> 8
gacattgtga tgacccagtc tcacaaattc atgtccacat cagtaggaga cagggtcagc
60
atcacctgca aggccagtca ggatgtgagt actgctgtag cctggtatca acaaaaacca
120
gggcaatctc ctaaactact gatttactgg gcatccaccc ggcacactga agtccctgat
180
cgcttcacag gcagtggatc tgggacagat tatactctca ccatcagcag tgtgcaggct
240
gaagacctgg cactttatta ctgtcagcaa cattataaca ctccgctcac gttcggtgct
300
gggaccaagc tggagctgaa acgggct
327

<210> 9
<211> 16
<212> PRT
<213> Artificial Sequence

<220>
<223> Description of Artificial Sequence: Artificially
      Synthesized Polypeptide

<400> 9
Arg Ser Ser Lys Ser Leu Leu Tyr Ser Asn Gly Asn Thr Tyr Leu Tyr
  1               5                  10                  15

<210> 10
<211> 21
<212> PRT
<213> Artificial Sequence

<220>
<223> Description of Artificial Sequence: Artificially
      Synthesized Polypeptide

<400> 10

Thr Pro Arg Gly Pro Asp Arg Pro Glu Gly Ile Glu Glu Glu Gly Gly
1               5                   10                  15

Glu Arg Asp Arg Asp
            20

<210> 11
<211> 7
<212> PRT
<213> Artificial Sequence

<220>
<223> Description of Artificial Sequence: Artificially
      Synthesized Polypeptide

<400> 11
Gly Ile Leu Pro Gly Ser Gly
1               5

<210> 12
<211> 7
<212> PRT
<213> Artificial Sequence

<220>
<223> Description of Artificial Sequence: Artificially
      Synthesized Polypeptide

<400> 12
Ser Gly Glu Ile Lys Tyr Glu
1               5

<210> 13
<211> 115
<212> PRT
<213> Mus musculus

<400> 13
Asp Ile Val Met Thr Gln Ala Thr Pro Ser Val Ser Val Thr Pro Gly
1               5                   10                  15

Glu Ser Val Phe Ile Ser Cys Arg Ser Ser Lys Ser Leu Leu Tyr Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Leu Phe His Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Tyr Thr Phe Gly Gly Gly Thr Lys Leu Glu Ile Lys
            100                 105                 110

Arg Ala Asp
        115


<210> 14
<211> 114
<212> PRT
<213> Helicobacter pylori

<400> 14
Asp Val Leu Leu Thr Gln Thr Pro Leu Ser Leu Pro Val Ser Leu Gly
1               5                   10                  15

Asp Gln Ala Ser Ile Ser Cys Arg Ser Gly Gln Ser Ile Val His Ser
            20                  25                  30

Asp Gly Asp Thr Asp Leu Glu Trp Tyr Leu Gln Arg Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Leu Tyr Tyr Cys Phe Gln Gly
                85                  90                  95

Ser His Val Pro Pro Thr Phe Gly Ser Gly Thr Lys Leu Glu Ile Lys
            100                 105                 110

Arg Ala


<210> 15
<211> 114
<212> PRT
<213> Helicobacter pylori

<400> 15
Asp Ile Val Val Thr Gln Thr Pro Leu Ser Leu Pro Val Ser Leu Gly
1               5                   10                  15

Asp Gln Val Ser Ile Ser Cys Arg Ser Ser Gln Ser Leu Ala Asn Ser
            20                  25                  30

Tyr Gly Asp Thr Tyr Leu Ser Trp Tyr Leu His Lys Pro Gly Gln Ser
        35                  40                  45

Pro Gln Leu Leu Ile Tyr Gly Ile Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Thr Ile Lys Pro Glu Asp Leu Gly Met Tyr Tyr Cys Leu Gln His
                85                  90                  95

Thr His Gln Pro Tyr Thr Phe Gly Gly Gly Thr Lys Leu Glu Ile Lys
            100                 105                 110

Arg Ala

<210> 16
<211> 118
<212> PRT
<213> Helicobacter pylori

<400> 16
Gln Val Gln Leu Gln Gln Ser Gly Ala Glu Leu Ala Arg Pro Gly Ala
1               5                   10                  15

Ser Val Lys Leu Ser Cys Arg Ala Ser Gly Tyr Thr Phe Thr Asp Tyr
            20                  25                  30

Tyr Ile Asn Trp Val Lys Gln Arg Thr Gly Gln Gly Leu Glu Trp Ile
        35                  40                  45

Gly Glu Ile Tyr Pro Gly Ser Asp Lys Asn Tyr Tyr Asn Glu Lys Phe
    50                  55                  60

Lys Gly Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ser Ser Tyr Tyr Arg Phe Asp Trp Phe Ala Tyr Trp Gly Gln Gly Thr
            100                 105                 110

Leu Val Thr Val Ser Ala
        115

<210> 17
<211> 114
<212> PRT
<213> Helicobacter pylori

<400> 17
Asp Val Leu Met Thr Gln Thr Pro Leu Ser Leu Pro Ile Ser Leu Gly
1               5                   10                  15

Asp Gln Ala Ser Ile Ser Cys Arg Ser Ser Gln Ser Ile Val Gln Ser
            20                  25                  30

Asn Gly Asn Thr Tyr Leu Glu Trp Tyr Val Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Phe Lys Ile
65                  70                  75                  80

Ser Arg Val Glu Ala Glu Asp Leu Gly Val Tyr Tyr Cys Ile Gln Gly
                85                  90                  95

Ser His Val Pro Leu Thr Phe Gly Ala Gly Thr Lys Leu Glu Leu Lys
            100                 105                 110

Arg Ala

<210> 18
<211> 342
<212> DNA
<213> Helicobacter pylori

<400> 18
gatgttttga tgacccaaac tccactctcc ctgcctatca gtcttggaga tcaagcctcc
60
atctcttgca gatctagtca gagcattgta caaagtaatg gaaacaccta tttagaatgg
120
tacgtgcaga aaccaggcca gtctccaaag ctcctgatct acaaagtttc caaccgattt
180
tctggggtcc cagacaggtt cagtggcagt ggatcaggga cagatttcac attcaagatc
240
agcagagtgg aggctgagga tctgggagtt tattactgca ttcaaggttc acatgttccg
300
ctcacgttcg gtgctgggac caagctggag ctgaaacggg ct
342

<210> 19

<211> 121
<212> PRT
<213> Helicobacter pylori

<400> 19
Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Arg Leu Glu Trp Val
        35                  40                  45

Ala Thr Ile Ser Ser Arg Gly Thr Tyr Thr Tyr Tyr Pro Asp Ser Val
    50                  55                  60

Lys Gly Arg Phe Thr Ile Ser Arg Asp Asn Ala Lys Asn Thr Leu Phe
65                  70                  75                  80

Leu Gln Met Ser Ser Leu Arg Ser Glu Asp Thr Ala Met Tyr Tyr Cys
                85                  90                  95

Ala Arg Gln Gly Gly Tyr Asp Tyr Asp Ala Trp Phe Pro Tyr Trp Gly
            100                 105                 110

Gln Gly Thr Leu Val Thr Val Ser Ala
        115                 120


<210> 20
<211> 363
<212> DNA
<213> Helicobacter pylori

<400> 20
gaagtgcagc tggtggagtc tgggggaggc ttagtgaagc ctggaggatc cctgaaactc
60
tcctgtgcag cctctggatt cactttcagt agctatgcca tgtcttgggt tcgccagact
120
ccggagaaga ggctggagtg ggtcgcaacc attagtagtc gtggtactta cacctactat
180
ccagacagtg tgaagggtcg attcaccatc tccagagaca atgccaagaa caccctgttc
240
ctgcaaatga gcagtctgag gtctgaggac acggccatgt attactgtgc aagacagggg
300
ggttatgatt acgacgcctg gtttccttac tggggccaag ggactctggt cactgtctct
360
gca
363

<210> 21
<211> 110
<212> PRT
<213> Helicobacter pylori

<400> 21
Asp Ile Val Leu Thr Gln Ser Pro Ala Ser Leu Ala Val Ser Leu Gly
1               5                   10                  15

Gln Arg Ala Thr Ile Ser Tyr Arg Ala Ser Lys Ser Val Ser Thr Ser
            20                  25                  30

Gly Tyr Ser Tyr Met His Trp Asn Gln Gln Lys Pro Gly Gln Pro Pro
        35                  40                  45

Arg Leu Leu Ile Tyr Leu Val Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln His Ile Arg
                85                  90                  95

Glu Leu Tyr Thr Phe Gly Gly Gly Thr Lys Leu Glu Ile Lys
            100                 105                 110

<210> 22
<211> 332
<212> DNA
<213> Helicobacter pylori

<400> 22
gacattgtgc tgacacagtc tcctgcttcc ttagctgtat ctctggggca gagggccacc
60
atctcataca gggccagcaa aagtgtcagt acatctggct atagttatat gcactggaac
120
caacagaaac caggacagcc acccagactc ctcatctatc ttgtatccaa cctagaatct
180
ggggtccctg ccaggttcag tggcagtggg tctgggacag acttcaccct caacatccat
240
cctgtggagg aggaggatgc tgcaacctat tactgtcagc acattaggga gctttacacg
300
ttcggagggg ggaccaagct ggaaataaaa cg
332

<210> 23
<211> 117
<212> PRT
<213> Helicobacter pylori

<400> 23
Gln Val Gln Leu Leu Gln Ser Gly Ala Glu Leu Met Lys Pro Gly Ala
1               5                   10                  15

Ser Val Lys Ile Ser Cys Lys Ala Thr Gly Tyr Thr Phe Ser Thr Tyr
            20                  25                  30

Trp Ile Glu Trp Val Lys His Arg Pro Gly His Gly Leu Glu Trp Ile
        35                  40                  45

Gly Glu Ile Leu Pro Gly Asn Gly Arg Thr Asn Tyr Asn Glu Lys Phe
    50                  55                  60

Lys Gly Lys Ala Thr Phe Thr Ala Asp Thr Ser Ser Asn Thr Ala Tyr
65                  70                  75                  80

Met Gln Leu Ser Gly Leu Thr Ser Glu Asp Ser Ala Val Tyr Tyr Cys
                85                  90                  95

Ala Gln Gln Leu Gly Leu Arg Phe Ala Tyr Trp Gly Gln Gly Thr Leu
            100                 105                 110

Val Thr Val Ser Ser
        115

<210> 24
<211> 351
<212> DNA
<213> Helicobacter pylori

<400> 24
caggtgcagc tgctgcagtc tggagctgag ctgatgaagc ctggggcctc agtgaagata
60
tcctgcaagg ctactggcta cacattcagt acctactgga tagagtgggt aaagcatcgt
120
cccgggcatg gccttgagtg gattggagag attttgcctg gaaacggccg tactaactac
180
aatgagaagt tcaagggcaa ggccacattc actgcagata catcctccaa cacagcctac
240
atgcaactca gcggcctgac atctgaggac tctgccgtct attactgtgc acaacaactc
300
gggcttcggt ttgcttactg gggccaaggg actctggtca ccgtctcctc a
351

<210> 25
<211> 19
<212> PRT
<213> Artificial Sequence

<220>
<223> Description of Artificial Sequence: Artificially
      Synthesized Polypeptide

<400> 25
Ser Val Glu Leu Asp Ile Gly Gly Asn Arg Arg Ile Phe Gly Asn Ala
  1               5                  10                  15

Leu Val Asp

<210> 26
<211> 114
<212> PRT
<213> Homo sapiens

<400> 26
Asp Val Leu Met Thr Gln Thr Pro Leu Ser Leu Pro Val Ser Leu Gly
  1               5                  10                  15

Asp Gln Ala Ser Ile Ser Cys Arg Ser Ser Gln Ser Ile Val His Ser
             20                  25                  30

Asn Gly Asn Thr Tyr Leu Glu Trp Tyr Leu Gln Lys Pro Gly Gln Ser
         35                  40                  45

Pro Lys Leu Leu Ile Tyr Lys Val Ser Asn Arg Phe Ser Gly Val Pro
     50                  55                  60

Asp Arg Phe Ser Gly Ser Gly Ser Gly Thr Asp Phe Thr Leu Lys Ile
 65                  70                  75                  80

Asn Arg Val Ala Ala Glu Asp Leu Gly Val Tyr Tyr Cys Phe Gln Gly
                 85                  90                  95

Ser His Val Pro Trp Thr Phe Gly Gly Gly Thr Lys Leu Glu Ile Lys
            100                 105                 110

Arg Ala

<210> 27
<211> 342
<212> DNA
<213> Homo sapiens

<400> 27
gatgttttga tgacccaaac tccactctcc ctgcctgtca gtcttggaga tcaagcctcc
60
atctcttgca gatctagtca gagcattgta catagtaatg gaaacaccta tttagaatgg
120

tacctgcaga aaccaggcca gtctccaaag ctcctgatct acaaagtttc caaccgattt
180
tctggggtcc cagacaggtt cagtggcagt ggatcaggga cagatttcac actcaagatc
240
aacagagtgg cggctgaaga tctgggagtt tattactgct ttcaaggttc acatgttccg
300
tggacgttcg gtggaggcac caagctggaa atcaaacggg ct
342

<210> 28
<211> 117
<212> PRT
<213> Homo sapiens

<400> 28
Asp Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Arg Tyr
            20                  25                  30

Thr Met Ser Trp Val Arg Gln Thr Pro Glu Lys Arg Leu Glu Trp Val
        35                  40                  45

Ala Thr Ile Ser Ser Gly Gly Ser Tyr Thr Tyr Tyr Pro Asp Ser Val
    50                  55                  60

Lys Gly Arg Phe Thr Ile Ser Arg Asp Ile Ala Lys Asn Thr Leu Tyr
65                  70                  75                  80

Leu Gln Met Ser Ser Leu Lys Ser Glu Asp Thr Ala Leu Tyr Tyr Cys
                85                  90                  95

Thr Arg Glu Gly Gly Tyr Gly Ala Phe Ala Ser Trp Gly Gln Gly Thr
            100                 105                 110

Leu Val Thr Val Ser
        115

<210> 29
<211> 355
<212> DNA
<213> Homo sapiens

<400> 29
cagtgtgacg tgaagctggt ggagtctggg ggaggcttag tgaagcctgg agggtccctg
60
aaactctcct gtgcagcctc tggattcact ttcagtagat ataccatgtc ttgggttcgc
120
cagactccgg agaagaggct ggagtgggtc gcaaccatta gtagtggtgg tagttacacc

gggatcccag gcaggtttag tggcagtggg tctgggacag acttcaccct caacatccat
240
cctgtggagg aggaggatat tgcaacctat tactgtcaac aaagttatga ggatccattc
300
acgttcggct cggggacaat attggacata aaacgggct
339

<210> 32
<211> 117
<212> PRT
<213> Homo sapiens

<400> 32
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Glu Trp Met
        35                  40                  45

Gly Trp Ile Asn Thr Tyr Thr Gly Glu Pro Thr Tyr Ala Gly Asp Phe
    50                  55                  60

Met Gly Arg Ser Val Phe Ser Leu Glu Thr Ser Ala Ser Thr Ala Tyr
65                  70                  75                  80

Leu Gln Ile Asn Asn Leu Glu Asn Lys Asp Thr Ala Thr Tyr Ile Cys
                85                  90                  95

Ala Arg Ser Gly Ser Pro Tyr Ala Met Asp Tyr Trp Gly Gln Gly Thr
            100                 105                 110

Ser Val Thr Val Ser
        115

<210> 33
<211> 351
<212> DNA
<213> Homo sapiens

<400> 33
cagatccagt tggtgcagtc tggacctgag ctgaagaagc ctggagagac agtcaagatc
60
tcctgtaagg cttctgggta taccttcaca aactacggaa tgaactgggt gaagcaggct
120
ccaggaaagg gattagagtg gatgggttgg ataaatacct acactggaga gccaacatac
180
gctggcgact tcatgggacg gtcagtcttc tctttggaaa cctctgccag cactgcctat

240
ttgcagatca acaacctcga aaataaggac acggctacat atatctgtgc aagatcgggg
300
tctccctatg ctatggacta ctggggtcaa ggaacctcag tcaccgtctc c
351

<210> 34
<211> 114
<212> PRT
<213> Homo sapiens

<400> 34
Asp Val Leu Met Thr Gln Thr Pro Leu Ser Leu Pro Val Ser Leu Gly
1               5                   10                  15

Asp Gln Ala Ser Ile Ser Cys Arg Ser Ser Gln Ser Ile Val His Ser
            20                  25                  30

Asn Gly Asn Thr Tyr Leu Glu Trp Tyr Leu Gln Lys Pro Gly Gln Ser
        35                  40                  45

Pro Lys Phe Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Thr Glu Asp Leu Gly Val Tyr Tyr Cys Phe Gln Gly
                85                  90                  95

Ser His Val Pro Trp Thr Phe Gly Gly Gly Thr Lys Leu Glu Ile Lys
            100                 105                 110

Arg Ala

<210> 35
<211> 342
<212> DNA
<213> Homo sapiens

<400> 35
gatgttttga tgacccaaac tccactctcc ctgcctgtca gtcttggaga tcaagcctcc
60
atctcttgca gatctagtca gagcattgta catagtaatg gaaacaccta tttagaatgg
120
tacctgcaga aaccaggcca gtctccaaaa ttcctgatct acaaagtttc caaccgattt
180
tctggggtcc cagacaggtt cagtggcagt ggatcaggga cagatttcac actcaagatc
240

agcagagtgg agactgagga tctgggagtt tattactgct ttcaaggttc acatgttccg
300
tggacgttcg gtggaggcac caagctggaa atcaaacggg ct
342

<210> 36
<211> 117
<212> PRT
<213> Homo sapiens

<400> 36
Gln Cys Asp Val Lys Leu Val Glu Ser Gly Gly Gly Leu Val Lys Pro
1               5                   10                  15

Gly Gly Ser Leu Lys Val Ser Cys Ala Ala Ser Gly Phe Ser Phe Ser
            20                  25                  30

Ser Tyr Ser Met Ser Trp Val Arg Gln Ser Pro Glu Lys Arg Leu Glu
        35                  40                  45

Trp Val Ala Thr Ile Ser Ser Gly Gly Ser Tyr Thr Tyr Tyr Pro Asp
    50                  55                  60

Ser Val Lys Gly Arg Phe Thr Ile Ser Arg Asp Ile Ala Lys Asn Thr
65                  70                  75                  80

Leu Tyr Leu Gln Met Asn Ser Leu Lys Ser Glu Asp Thr Ala Met Tyr
                85                  90                  95

Tyr Cys Thr Arg Glu Gly Gly Tyr Gly Ala Phe Ala Tyr Trp Gly Gln
            100                 105                 110

Gly Thr Leu Val Thr
        115

<210> 37
<211> 351
<212> DNA
<213> Homo sapiens

<400> 37
cagtgtgacg tgaagctggt ggagtctggg ggaggcttag tgaagcctgg agggtccctg
60
aaagtctcct gtgcagcctc tggattcagt tttagtagct atagcatgtc ttgggttcgc
120
cagagtccgg agaagaggct ggagtgggtc gcaaccatta gtagtggtgg tagttacacc
180
tactatccag acagtgtgaa gggccgattc accatctcca gagacattgc caagaacacc
240
ctatacctgc aaatgaacag tctgaagtct gaggacacag ccatgtatta ctgtacaaga

300
gaggggggtt acggagcctt tgcttactgg ggccaaggga ctctggtcac t
351

<210> 38
<211> 20
<212> PRT
<213> Artificial Sequence

<220>
<223> Description of Artificial Sequence: Artificially
      Synthesized Polypeptide

<400> 38
Arg Ser Ser His Phe Pro Tyr Ser Gln Tyr Gln Phe Trp Lys Asn Phe
  1               5                  10                  15

Gln Thr Leu Lys
             20

<210> 39
<211> 12
<212> PRT
<213> Artificial Sequence

<220>
<223> Description of Artificial Sequence: Artificially
      Synthesized Polypeptide

<400> 39
Arg Ser Gln Lys Glu Gly Leu His Tyr Thr Cys Ser
  1               5                  10

<210> 40
<211> 21
<212> PRT
<213> Artificial Sequence

<220>
<223> Description of Artificial Sequence: Artificially
      Synthesized Polypeptide

<400> 40
Arg Ser Ser His Phe Pro Tyr Ser Gln Tyr Gln Phe Trp Lys Asn Phe
  1               5                  10                  15

Gln Thr Leu Lys Gly
             20

<210> 41
<211> 35
<212> PRT
<213> Artificial Sequence

<220>
<223> Description of Artificial Sequence: Artificially
      Synthesized Polypeptide

<400> 41
Cys Thr Arg Pro Asn Asn Asn Thr Arg Lys Ser Ile His Ile Gly Pro
  1               5                  10                  15

Gly Arg Ala Phe Tyr Thr Thr Gly Glu Ile Ile Gly Asp Ile Arg Gln
             20                  25                  30

Ala His Cys
         35


<210> 42
<211> 21
<212> PRT
<213> Artificial Sequence

<220>
<223> Description of Artificial Sequence: Artificially
      Synthesized Polypeptide

<400> 42
Thr Pro Arg Gly Pro Asp Arg Pro Glu Gly Ile Glu Glu Glu Gly Gly
  1               5                  10                  15

Glu Arg Asp Arg Asp
             20


<210> 43
<211> 20
<212> PRT
<213> Artificial Sequence

<220>
<223> Description of Artificial Sequence: Artificially
      Synthesized Polypeptide

<400> 43
Lys Leu Leu Arg Gly Thr Lys Ala Leu Thr Phe Val Ile Pro Leu Thr
  1               5                  10                  15

Glu Glu Ala Glu

            20

<210> 44
<211> 16
<212> PRT
<213> Artificial Sequence

<220>
<223> Description of Artificial Sequence: Artificially
      Synthesized Polypeptide

<400> 44
Arg Ser Ser Lys Ser Leu Leu Tyr Ser Asn Gly Asn Thr Tyr Leu Tyr
  1               5                  10                  15

<210> 45
<211> 17
<212> PRT
<213> Artificial Sequence

<220>
<223> Description of Artificial Sequence:Artificially
      Synthesized Polypeptide

<400> 45
Asp Lys Phe Asn Gln Asn Tyr Ser Thr Ala Gly Asn Tyr Pro Asn Ile
  1               5                  10                  15

Arg

<210> 46
<211> 35
<212> PRT
<213> Artificial Sequence

<220>
<223> Description of Artificial Sequence: Artificially
      Synthesized Polypeptide

<400> 46
Arg Ser Ser Lys Ser Leu Leu Tyr Ser Asn Gly Asn Thr Tyr Leu Tyr
  1               5                  10                  15

Gly Pro Asp Lys Phe Asn Gln Asn Tyr Ser Thr Ala Gly Asn Tyr Pro
             20                  25                  30

Asn Ile Arg
         35

<210> 47
<211> 342
<212> DNA
<213> Helicobacter pylori

<400> 47
gatgttttgc tgacccaaac tccactctcc ctgcctgtca gtcttggaga tcaagcctcc
60
atctcttgca gatctggtca gagcattgta catagtgatg gagacaccga tttagaatgg
120
tacctgcaga gaccaggcca gtctccaaag ctcctgatct acaaagtttc caaccgattt
180
tctggggtcc cagacaggtt cagtggcagt ggatcaggga cagatttcac actcaagatc
240
agcagagtgg aggctgaaga tctgggactt tattactgct ttcaaggttc acatgttcct
300
cccacgttcg gctcggggac aaagttggaa ataaaacggg ct
342

<210> 48
<211> 342
<212> DNA
<213> Helicobacter pylori

<400> 48
gatattgtgg tgactcaaac tccactctcc ctgcctgtca gtcttggaga tcaagtttct
60
atctcttgca ggtctagtca gagtcttgca aacagttatg gggacaccta tttgtcttgg
120
tacctgcaca agcctggcca gtctccacag ctcctcatct atgggatttc caacagattt
180
tctggggtgc cagacaggtt cagtggcagt ggttcaggga cagatttcac actcaagatc
240
agcacaataa agcctgagga cttgggaatg tattactgct tacaacatac acatcagccg
300
tacacgttcg gaggggggac caagctggaa ataaaacggg ct
342

<210> 49
<211> 354
<212> DNA
<213> Helicobacter pylori

<400> 49
caggttcagc tgcagcagtc tggagctgag ctggcgaggc ccggggcttc agtgaagctg
60
tcctgcaggg cttctggcta caccttcact gactactata taaactgggt gaaacagagg

120
actggacagg gccttgagtg gattggagag atttatcctg gaagtgataa aaattattat
180
aatgagaagt tcaagggcaa ggccacactg actacagaca aatcctccag cacagcctac
240
atgcagctca gcagcctgac atctgaggac tctgcagtct atttctgttc aagctactat
300
aggttcgact ggtttgctta ctggggccaa gggactctgg tcactgtctc tgca
354